# EUROPÄISCHE PATENTANMELDUNG

(11) **EP 0 798 004 A1**
(43) Veröffentlichungstag der Anmeldung: **01.10.1997**
(21) Anmeldenummer: 97250104.3
(22) Anmeldetag: 29.03.1997
(51) Int. Cl.: A61M 5/42, A61B 5/00

(54) **Einrichtung und einrichtungsbezogenes Verfahren für die Stoffwechselkontrolle bei einem Lebewesen**

(30) Priorität: 29.03.1996 DE 19613722; 01.02.1997 DE 19705091
(71) Anmelder: Wagner, Wolfgang, Dr.med., 13503 Berlin (DE)
(72) Erfinder: Wagner, Wolfgang, Dr.med., 13503 Berlin (DE)

(57) **Zusammenfassung**

Einrichtung für die Stoffwechselkontrolle bei einem Lebewesen oder im engeren Sinne Punktionsgerät, das mit Vorrichtungen ausgestattet ist, die nach Eindringen einer Schneidspitze in die Haut dieselbe parallel zu deren Oberfläche ruckartig um eine einstellbare, kurze Strecke bewegt und das durch Gefäßzerstörung austretende Blut vorzugsweise innerhalb einer Saugglocke direkt auf das Testfeld eines möglichst direkt angeschlossenen Meßgerätes, vcrzugsweise für Glukose, leitet. Es werden verschiedene Punktionsgriffel und -gerätetypen sowie Anwendungsvarianten angegeben.

## Beschreibung

### Aufgabenstellung

Die Erfindung bezieht sich auf das Gebiet der Medizintechnik, spezieller auf die Diagnostik von Stoffwechselzuständen. Letzteres inbesordere bei Diabetikern und hinsichtlich der Blutzuckerkontrolle.
Die Punktion des Muskelfleisches an Fingern und Fußballen (letzteres besonders bei Kindern) oder auch am Ohrläppchen zur Blutgewinnung ist besonders in ständiger Wiederholung wegen der Narbenbildungen schmerzhaft und teilweise umständlich. Im Hand- und Fußbereich besteht außerdem erhöhte Infektionsgefahr. Es ist die Aufgabe dieser Erfindung, die Blutabnahme an einer nahezu beliebigen Hautstelle und in der Regel schmerzfrei vornehmen zu lassen, wozu die apparativen Voraussetzungen wesentlich zu verbessern waren.

### Stand der Technik

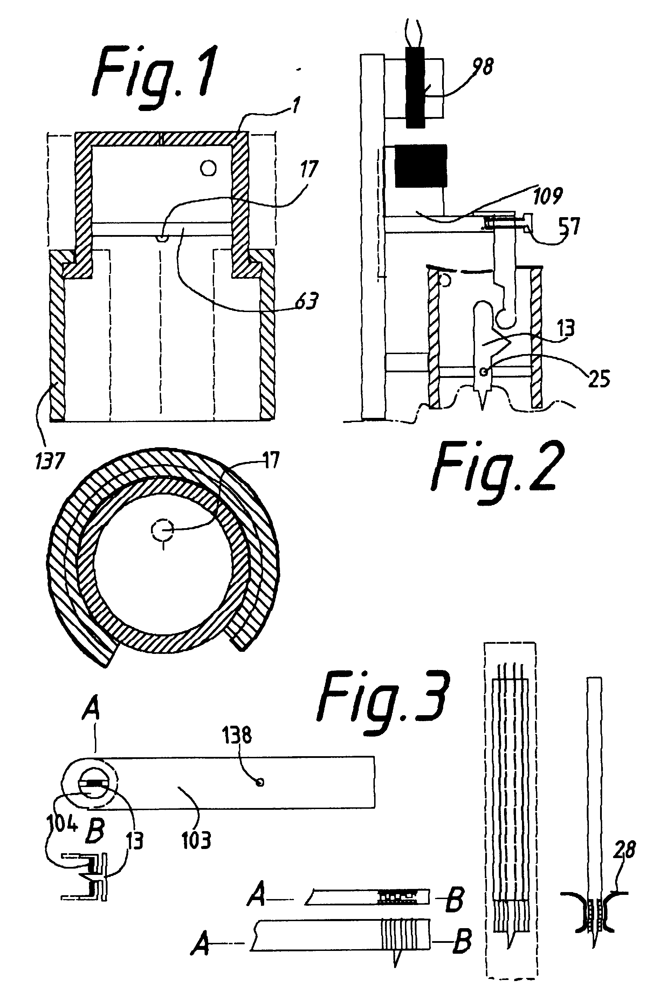

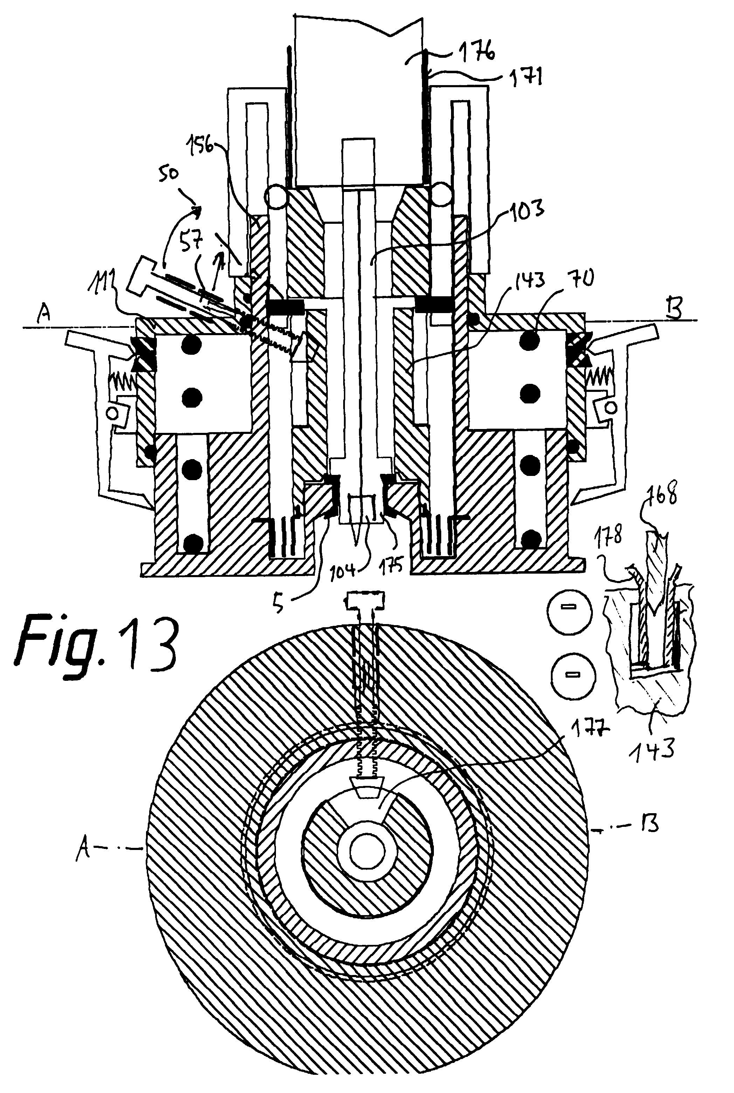

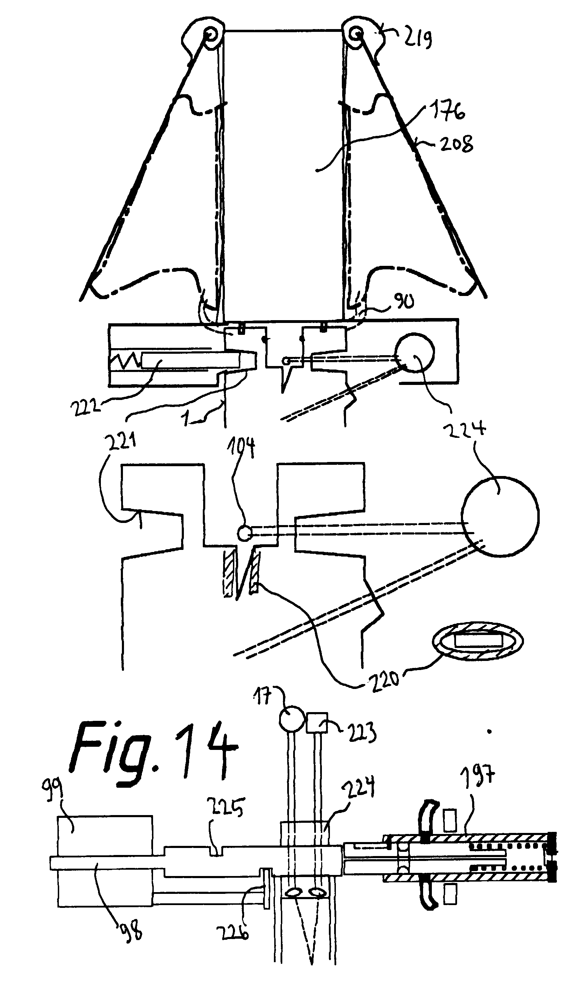

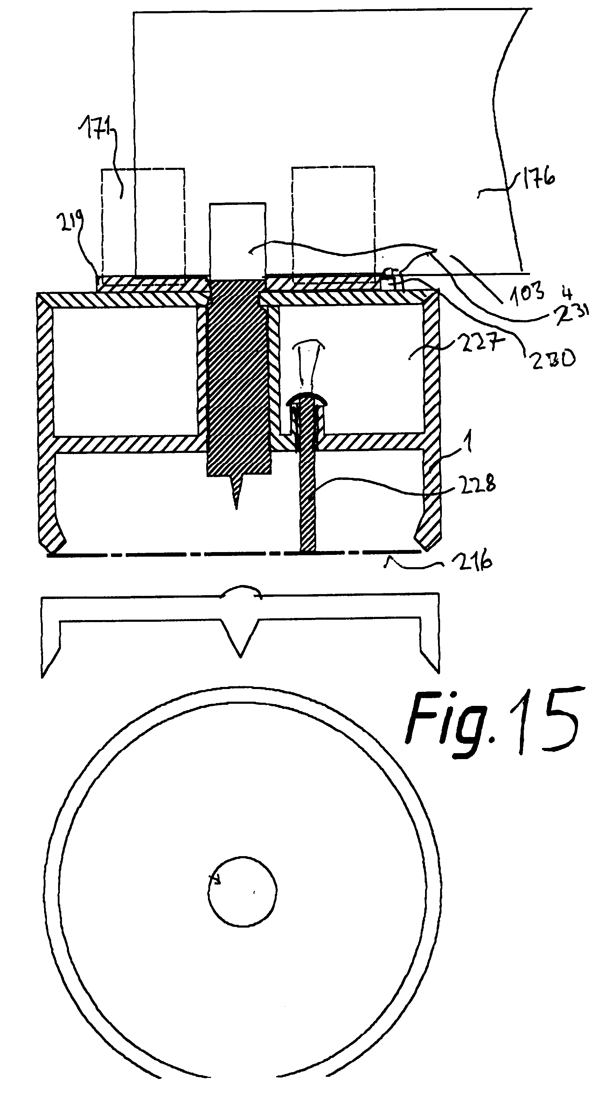

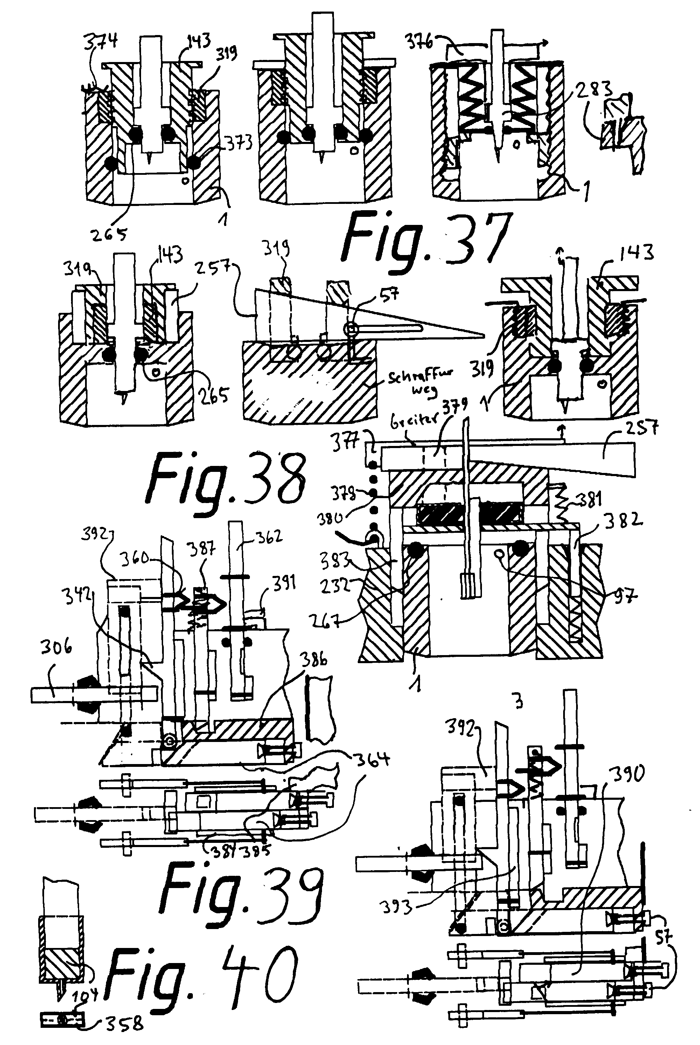

Wesentlich stützt sich die Erfindung auf Ausführungen in DE 3806574 A1 (Fig.3,5-13), worin vorgeschlagen wurde, die Sicherheit der Eröffnung eines genügend großen Blutgefäßes dadurch zu erhöhen, daß man die Spitze einer Kanüle durch deren Rotation nach dem Einstich eine Kreisbahn im Bereich der tiefer liegenden größeren Blutkapillaren beschreiben läßt. Auch die weit preiswertere Lanzette wurde als Punktionsmittel vorgeschlagen, die längs ihrer Schnittkante in eine schwingende Bewegung versetzt wird. Wo diese Bewegung keine Kippbewegung um eine Achse parallel zur Haut ist, war die vorgeschlagene Anordnung allerdings wenig tauglich. Dies geht schon aus der Angabe über die "mitschwingende Haut"(Seite 14, Zeile 24) hervor. Ein Lanzettenrückzug zur Freigabe des Stichkanals wird unter "Fig.55" im Text angegeben. Ein entsprechendes Vorgehen schlagen bereits Garcia et.al. (US-Pat.No. 4,637,403) in den Fig.14,15 vor, wobei letztere sich auch näher mit der meßtechnischen Auswertung befassen, aber in der Anwendung ihrer Erfindung auf die Punktion oberflächlich liegender Muskeln angewiesen bleiben. Die Euro-Patentanmeldung 0301165 A2 wiederholt die eben beschriebenen Beispiele im Wesentlichen (mit den Figuren 39,41-46,49,53,55-58) und wendet Drehung und Vibration,d.h. Mehrfachschwingung von Kanüle und Lanzette an. Anspruch 12 bezieht sich auf einen Punktionsgriffel der quer zur Haut bewegt wird. DE 37 08 031.8 A1 ist inhaltlich in der genannten Euro-Patentanmeldung verwertet. DE 3925940 A1 erweitert mit den Figuren 13 und 20-22 die Erfindung. Hier wird auch eine Drehachse für die Lanzette angegeben, die sich in einer Hülse befindet. Nach Anspruch 48 weist der Lanzettenschaft eine seitliche Einkerbung (für die Drehachse) auf, nach Anspruch 53 ist der Lanzettenschaft dadurch sowohl höhenverschieblich als auch seitlich abkippbar. Nach Arspruch 54 ist die Lanzette über eine Vorrichtung federnd seitlich mit der Umrahmung der Hautkuppe verbunden. Nach Anspruch 55 (Fig.13) wird eine Kapillare in einer Furche (durch Schaftverformung) zur Blutabnahme bis nahe an die Lanzettenspitze herangeführt. Auf der Figur ist auch ein zentraler Schlitz im Schaft zu erkennen.
Alle geschilderten Beispiele sind auf den Gebrauch in einem Injektor hin ausgelegt und dadurch verhältnismäßig aufwendig und störanfällig, so daß es offenbar zu keiner industriellen Nutzung kam.

### Lösung der gestellten Aufgabe

Die bisher vorgeschlagenen Lösungen entsprachen infolge längerer Verletzungseinwirkung auf das Unterhautzellgewebe - etwa durch Rotation einer Kanüle oder Mehrfachschwingung einer Lanzette - nicht dem Ziel eines minimal-invasiven Vorgehens. Zwar ist das Verletzen einer größeren Kapillare unter der Haut durch eine Kanülen- oder Lanzettenspitze zufällig, aber es genügt auch schon eine Schnitterweiterung um meist weniger als einen Millimeter, wenn diese genügend ruckartig erfolgt oder die Blutkapillaren sonst am Ausweichen verhindert werden. In der Berücksichtigung dieser Faktoren, nämlich in der möglichst inviduell einstellbaren, jedenfalls aber auf das notwendige Maß begrenzten Schnittlängeneinstellung und in der ruckartigen Gestaltung dieser Schnittbewegung liegt ein wesentliches Lösungselement dieser Erfindung. Da inzwischen eine Reihe von Glukometern sich in der Hand der Patienten befinden, war es ein weiteres für die Anwendbarkeit (und Wirtschaftlichkeit) dieser Einrichtung, sie so zu gestalten, daß ein handelsübliches Glukometer direkt mit ihr für die Meßwertgewinnung verbunden werden kann.
Die jetzige Erfindung zielt auf wesentliche Vereinfachungen ab, indem die Anwendung auf den diagnostischen Bereich schon von der Aufgabe her eingeschränkt wurde. Zwar soll eine Stangenführung innerhalb einer Dichtung im Saugglockendach noch vom Erfindungsschutz abgedeckt werden; tatsächlich aber wurden Bedienungselemente über Dichtungen in der Saugglocke und insbesondere im Saugglockendach eingespart oder tunlich ganz vermieden. Zunächst geschah dies dadurch, daß -wo nicht die Saugglocke selbst Wegwerfteil wurde- die Lanzette von Seiten der Saugglockenöffnung her gewechselt wird.

Die besonders hohe Hautelastizität hat zur Folge, daß die Haut als Membran unter Sogwirkung schneller in die Saugglocke gehoben wird als eine robustere technische Membran, wie sie vom Saugglockendach bei Unterdruck in den Saugglockenraum abgesenkt werden kann. Eine (Teleskop-)Stange, die unter einem rückfedernden Saugglockendach befestigt ist, läßt sich vortrefflich zur Erzielung der seitlichen Kippbewegung einer Lanzette nutzen. Das Stangenende -auch über eine Kurvenscheibe am Ende im Breitendurchmesser variierbar- kann etwa hierfür an einer keilförmigen Lanzettenschaftausladung vorbeibewegt werden (und zwar sowohl von oben mit der Annährung des Saugglockendaches als von unten, wenn zur Sogerzeugung das Dach -etwa durch Federkraft- angehoben wird). Die Ausladung am Schaft kann der Spitze zu auch waagerecht und abrupt sein, so daß eine sich hebende Stange mit ihrer Endausladung sich dort verhakt und die Lanzette bei ihrer Anhebung aus dem Stichkanal zieht(Fig.2). Um das Blut aus dem durch die hochelastische Haut sich schließenden Stichkanal abzuleiten, bedarf keiner besonderen zusätzlichen Kapilaren, wenn der Schaft auf seiner Oberfläche bis in Spitzennähe passende Schaftverbreiterungen etwa in Gestalt von Lamellen mit kapillären Zwischenräumen oder Kantenumbiegungen -auch von einem ausgestanzten Mittelschlitz her- aufweist. In einer speziellen Variation bilden aus der Schaftebene umgebogene Randkante(n) und Schlitzkante(n) nahezu eine (oder zwei) Röhre(n). Das Abgeleitete Blut kann mit dem Testfeld eines handelsüblichen Sensors (etwa für die elektrische Glukosemessung) dadurch in Kontakt gebracht werden, daß der Schaft mit seiner Breitseite im Bereich der beschriebenen Lamellen mit dem Testfeld in Berührung gebracht wird. Das Testfeld kann aber auch in üblicherweise mit seinem streifenförmigen Träger einem Blutstropfen auf der Haut nach Wiederbelüftung und Entfernung der Saugglocke genähert werden. Ein seitliches Herablaufen des Blutstropfens läßt sich außer durch die adhesiven Eigenschaften des noch in der Haut steckenden Lanzettenschaftes dadurch vermeiden, daß die Saugglocke durch eine tiefstehende Lanzette in ihrer Kuppe napfförmit bis zur Wiederbelüftung der Saugglocke eingesenkt wird. Eine solche Tiefstellung der Lanzette erlaubt auch bei Verwendung eines sogenannten "Sogschalters" (DE 2551993 aus 1974) gegen eine Rückstellfeder eine ruckartige Stiftbewegung seitlich gegen den Lanzettenschaft, deren Winkelgröße durch eine Anschlagschraube dem Gefäßreichtum der jeweiligen Haut angepaßt werden kann Eine die Endkuppe des Schaftes überholende Blattfeder erlaubt die Schaftrückkehr in die Senkrechte unter der Einwirkung einer Rückstellfeder(eventuell gegen einen Anschlag) aber auch schon durch die rückfedernd wirkende Hautelastizität. Es ist eines der Hauptmerkmale der Erfindung, daß die Kippachse für die Lanzette entweder selbst ein Bestandteil der Einrichtung selbst ist oder, wo die Kippachse etwa in Gestalt einer Achsnoppe ein Bestandteil des Lanzettenschaftes ist, daß in einem Gerätebestandteil ein Achslager für diese Noppe (und eventuell der ihr entsprechenden Aushöhlung auf der Gegenschaftseite) vorhanden ist. Ein solches Achslager kann auf einer Querstrebe angeordnet sein, welche vom Saugglockenzylinder ausgeht; es kann das Achslager aber auch in einem Schlitz einer Zwischenwand montiert sein, welche Bestandteil einer (vorzugsweise segmentierten) Hülse ist, welche innerhalb der Saugglocke höhenverschieblich ist.
Günstig für die Ausführung der seitlichen Stoßbewegung ist deren ruckartige Ausführung durch einen elektrischen Magneten. Dieser wirkt entweder direkt auf die dafür besonders eisenhaltige Aufnahme der Lanzette ein oder bewegt vorzugsweise einen Stößel mit Rückfederung mit dem Magnetanker. Die elektrischen Zuleitungen durch eine frei wählbare Stelle im Saugglockenbereich bietet keine Abdichtungsprobleme. Als Alternative zur Kipp-achse, in welche die Lanzette bereits vor Einwirkung des Soges eingeführt wird (oder die an der Aufnahme des Schaftes kippend wirkt) können Lanzette, beziehungsweise deren Aufnahme, auch gegenüber einer relativ dazu feststehenden Haut quer zu dieser ruckartig bewegt werden.
Dies kann über einen Stößel, dessen Bewegung über einen Sogschalter bewirkt wird, oder elektromagnetisch erfolgen oder auch über die Bewegung der Querstrebe für die Lanzettenaufnahme. Da der spitzenabgewandte noch in die Haut eindringende Lanzettenteil abgestumpft ist, werden bei der Querbewegung des Lanzettenschaftes die Oberhaut mit ihren Gefäßen mitgenommen und nicht verletzt. Der Stichkanal kann also kleingehalten werden, ein Vorteil, der noch durch die Herabsetzung der Lanzettenbreite (gegenüber den heute handelsüblichen) und den Einstich in eine durch Sog gedehnte Haut (die sich später wieder zusammenzieht) wesentlich erhöht wird.
Es kann jetzt das Testfeld eines Sensors (etwa ein solcher des Produktes von Medidense oder Glucometer "Elite" von BAYER) direkt mit der Blutaustrittsstelle schon innerhalb der Saugglocke und während der Hautanhebung in Kontakt gebracht werden. Die Lanzette wird hierfür in den Schlitz eines Schiebers gesteckt, der auf einer Querstrebe oder selbst als Querstrebe dienend mittels einer Keilführung im Zusammenhang mit einer vertikal bewegten Stange oder einem parallel bewegten Stössel, etwa einem Anker eines Elektromagneten, seitwärts in Richtung der Lanzettenschneide gestoßen wird. Letztere, im Spitzenbereich wie hinter der Spitze, ist quer zur Schaftlänge über dem Testfeld des Sensorstreifens etwa rechtwinkelig abgebogen. Der Lanzettenschaft weist zum Testfeld hin ein Fenster auf oder feinere Durchlässe für das aus der Schnittwunde austretende Blut. Der Lanzettenschaft kann schließlich gänzlich fehlen und der schneidende Lanzettenteil direkt auf dem Testfeld fest plaziert sein (mit oder ohne den Teststreifen umklammernde Lasche. Im letzteren Falle wird wenigstens der Endabschnitt des Streifens mit dem Testfeld selbst stoßartig zur Blutgewinnung seitwärts zur Saugglockenachse bewegt. Die relative Hautfixierung ist besser, wenn der Durchmesser der Saugglocke kleiner ist, wie etwa bei einem Pen-artig, also schlanken Gerät. Die Möglichkeit der Hautausreckung wird aber besser über eine Saugglokke größeren Durchmessers genutzt. Es kann dann der Lanzettenspitze zur Hautfixierung eine Lochplatte vorgelagert werden. Letztere kann vorteilhaft auch der diaphanischen Hautkontrolle auf Eignung zur Punktion dienen. Der Lichtstrahl führt dabei, vorzugsweise innerhalb der Lochwandung (oder des Fensters) tangential durch die kleine zentrale Hautkuppe. Ein Vergleich der Strahlenabschwächung nach verschiedenen Benutzungen, aber auch während der Hautbewegung in das Fenster hinein kann rechnerisch zur Kontrolle herangezogen werden. Knotige Hautveränderungen verraten sich dadurch, daß überhaupt keine Haut in das Fenster der Lochplatte eintritt.
Zur Hautkontrolle auf Eignung zur Punktion muß entweder die der Lanzettenspitze vorgelagerte Lochplatte oder die höher zurückliegende Trägerplatte für die Lanzette zunächst festgestellt und bei Eignung zur Punktionsbewegung freigegeben werden. Auch eine kombinierte Annäherung beider Platten kommt in Frage. Sie kann durch seitliche Auslösestifte von Hand (auf ein akustisches oder sonst wahrnehmbares Zeichen hin) durch das Zusammendrücken zweier Tasten ausgelöst werden. Zweckmäßiger ist der Rückzug mindestens eines Arretiergliedes durch den Elektromagneten, der zugleich den Stoß zur Bewegung der Lanzette bewirkt. (Die Mitbewegung der Lanzette bei der Auslösung der Distanzierungsvorrichtung für die Haut stört dabei nicht) (Fig.7).

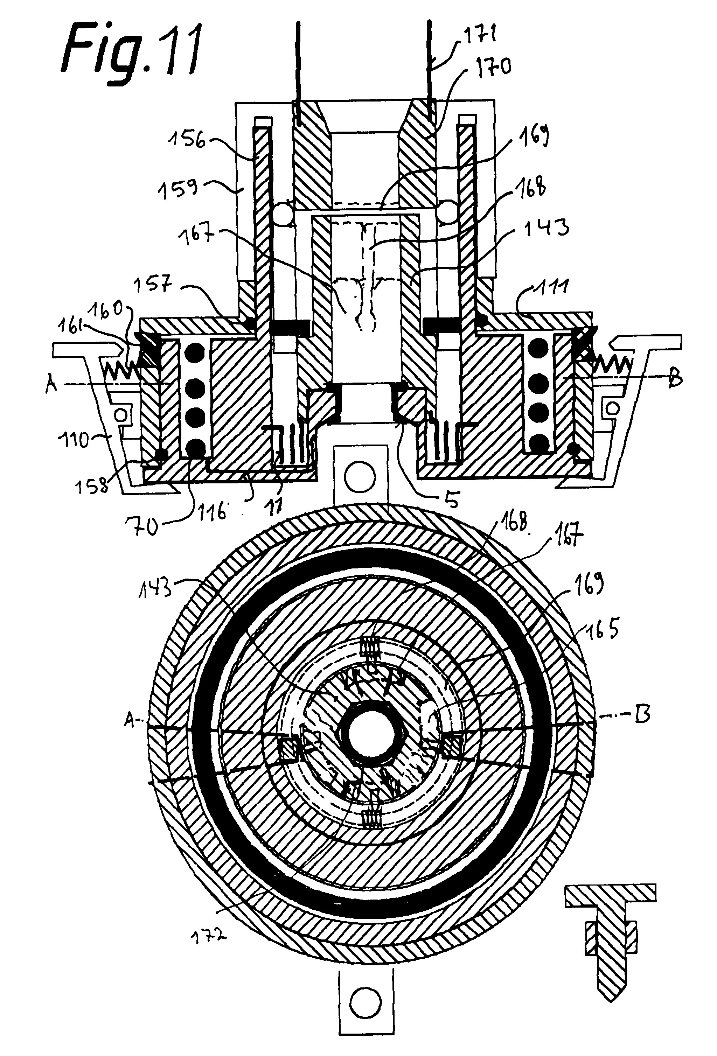

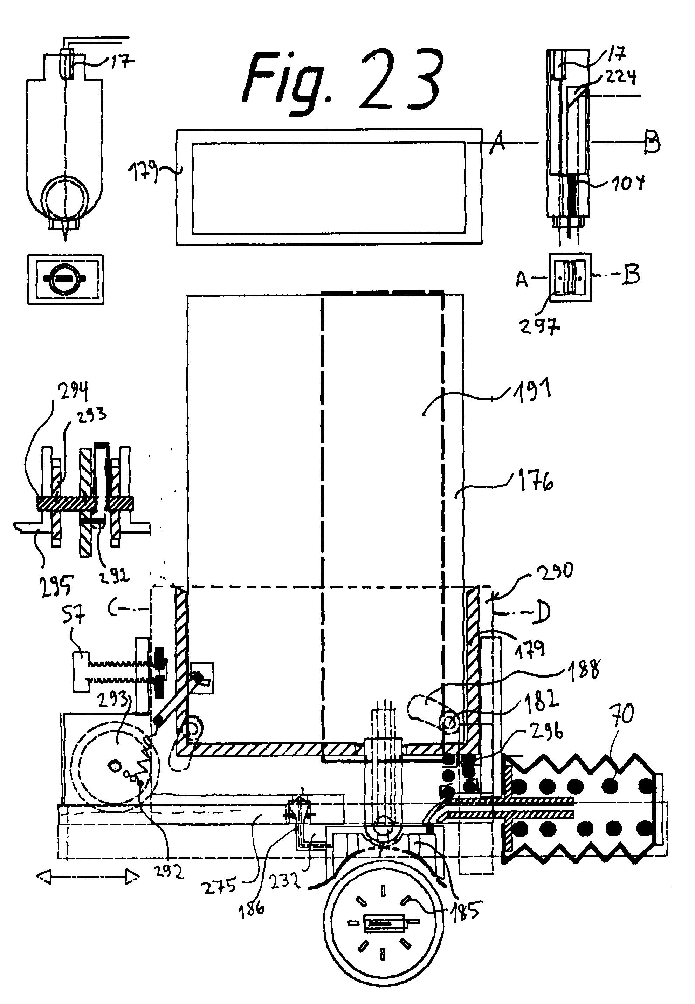

Gegenstand der Erfindung waren aber auch Mittel zur visuellen Hautkontrolle, um den Aufwand zu senken. Am Einfachsten ist es, eine Saugglocke oder einen ihr an Größe entsprechenden Ring gegen die Haut zu pressen. Deren mechanisch beanspruchten Stellen sind anschließend noch eine Zeitlang markiert, sei es ales Dellenbildung oder Rötung. Ein der Saugglokke zweckmäßigerweise (zur Aufbewahrung) aufschiebbarer Teller mit vorspringendem Rand und zentraler Spitze, kann hierzu nützlich sein, weil so die Punktionsstelle exakt vormerkbar ist (Fig.15 unten). Ist die Hautstelle geeignet und der Punkttionsgriffel eingeführt, so wird die Saugglocke wieder in den Abdruckring hineingestellt, der durch den Schablonenring oder den Saugglockenrand selbst erzeugt worden war. Vorteilhaft sind auch zwei gegenüberliegende Markierungspunkte durch Absenkung von angespitzten Bolzen, welche in Bohrungen von Laschen am Saugglockenaußenrand geführt werden (Fig.11). Eine aufwendigere Lösung ist ein durchsichtiger Gußzylinder (für Meßfeld und Lanzettenspitze), durch welchen von oben aus der Richtung des Glukometers Licht auf die Punktionsstelle geworfen wird. Das Licht wird von der Haut reflektiert und über ein Prisma oder einen Spiegel seitlich am Griffelansatz das Abbild der Punktionsstelle einsehbar (Fig. 23 oben). Besonders vorteilhaft ist die Einsichtmöglichkeit vor der Einführung eines Punktionsgriffels mit Teststreifen von oben. Es kann dann ein besonderes Instrument mit Lichtführung nach unten durch die Ringdichtung des Saugglockendaches wirksam werden mit entprechender Okularlinse. Weit praktischer ist die Möglichkeit bei Anwendung einer Schwinglanzette die Einsicht von oben in die Saugglocke weit offen zu lassen. Ein Lämpchen mit Strahlenkonzentration besonders auf die Punktionsstelle kann am Basissockel oben montiert sein, aber auch schräg im Bereich der Saugglockenwand.
Die Abdeckelung der Saugglocke erfolgt dann gegen einen weiten Dichtungsring über einen Deckelring der am Glukometer mit Schlitzöffnung für den Teststreifenschacht dicht befestigt ist unter Druckwirkung zum Saugglockenrand hin. Liegt im Glukometer keine Abdichtung um den Schacht herum vor und ist der Luftraum im Glukometer erheblich, so muß eine Abdichtung des Teststeifens oder Punktionsgriffels im Deckelbereich vorgesehen werden. Auch bei Rotationslanzetten kann dies dadurch geschehen, daß oben,also weit ab vom Gußstück um das Meßfeld, eine Zylinderrundung um den Teststreifen (und zu diesem gedichtet) in eine Dichtung im Deckelbereich eingeführt wird, wobei eine zweite Dichtung unten auch beim Rotationsgriffel entfallen kann. Es kann aber auch der flache Teststreifen zwischen zwei einander genäherten Gummiplatten eingefaßt werden. Durch deren Andruck gegeneinander erfolgt die Abdichtung um den Teststreifen in der Deckelmitte, während die seitliche Abdichtung wiederum durch eine gesonderte Ringdichtung vorzugsweise bei Druck von oben erfolgt. Die Annäherung der Gummiplatten kann durch eine Klammer in Nähe der Innenränder geschehen, welche gegen eine Keilführung außen verschoben wird. Der kreuzweise Verschluß kann aber auch von den Außenrändern der Gummiplatten her erfolgen, wenn diese bei Einschub von Keilen in eine seitliche Schienenführung zusammengedrängt werden. Die Schienenführung kann auch dem Andruck des Deckelringes von oben bei entsprechender Keilführung dienen. Letztere wird dann nach der Keilführung für die Gummiplatten wirksam, ist also geräteentfernter auf der Schiene montiert. Zweckmäßigerweise sind die Gummiplatten Bestandteile des mit dem Glukometer abhebbaren Deckelringes.

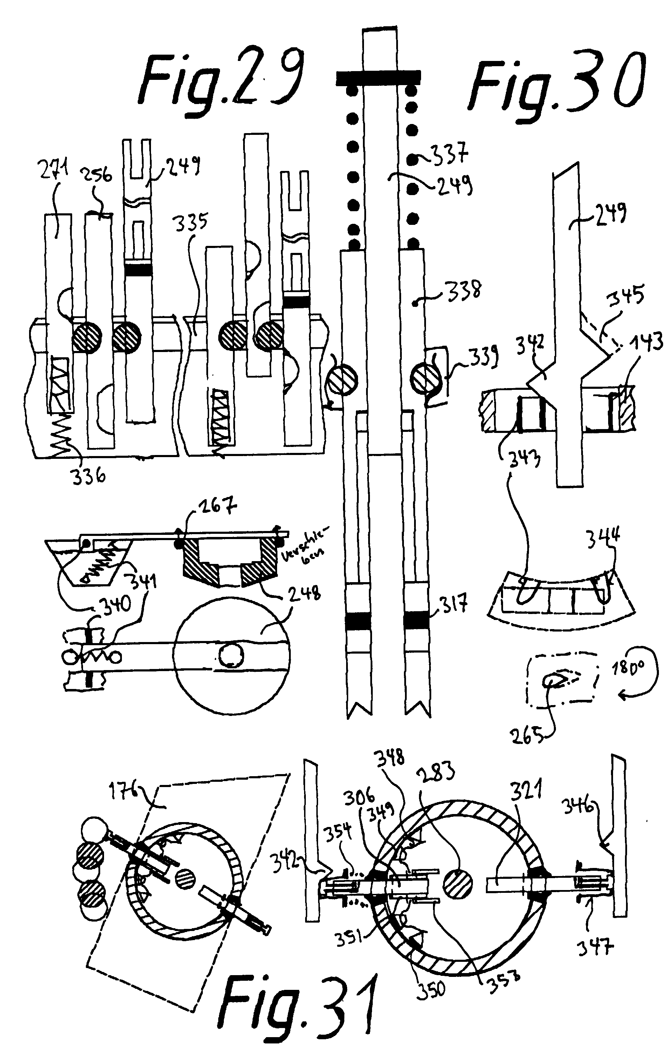

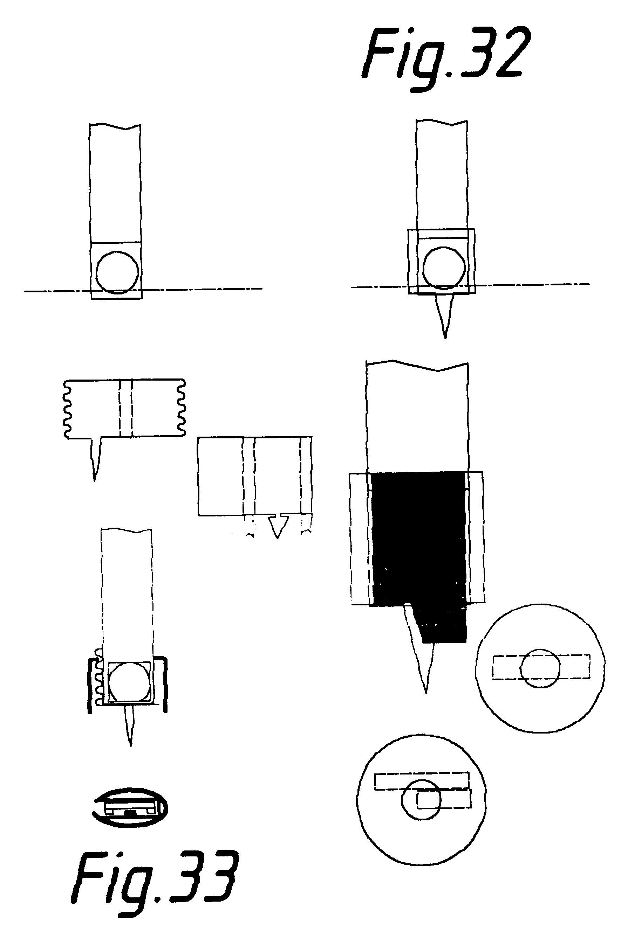

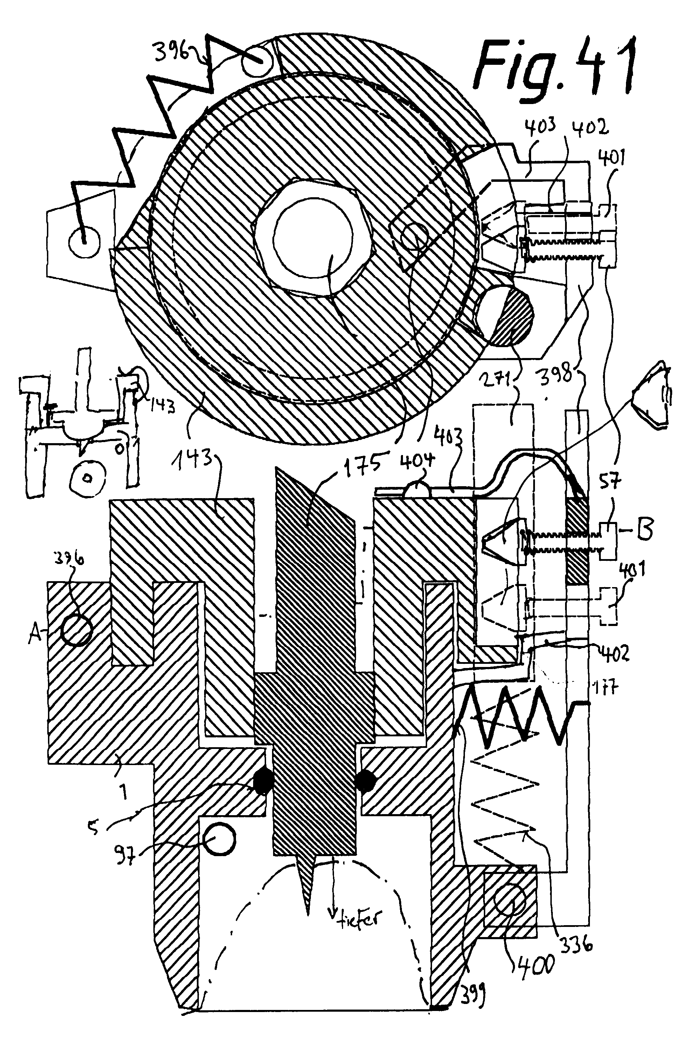

Eine Abdichtung um den Teststreifen kann entfallen, wenn das Glukometer eine Abdichtung um den Schacht für die Teststreifeneinführung hat oder nur geringen inneren Totraum bei dichter Hülle aufweist. Es kann dann nämlich ein Deckel ring um den Schacht des Glukometers dicht montiert oder gekittet werden, der seinerseits nur noch der Abdichtung gegenüber dem Saugglockenrand beispielsweise durch einen O-Ring bedarf. Bei Anwendung eines Rotationsgriffels kann die Abdichtung zum Saugglockendach unmittelbar oberhalb der Hautkuppe erfolgen. Der Teststreifen oder Rotationsgriffel wird dabei zweckmäßig von oben in Verbindung mit dem Glukometer eingeführt. Die Rotation selbst einer exzentrisch angeordneten Lanzettenspitze in der Haut ist jetzt sinnvoll, da die Rotationsbewegung ja, wie die Schwingbewegung, auf eine kurze Distanz erfindungsgemäß eingeengt wird. Die Oberschicht der Haut wird dabei wegen deren Elastizität mitgenommen, ohne den Schnitt zu erweitern, weil nur der Spitzenbereich der Lanzette scharf gehalten wird. Der Teststreifen für ein Glukometer braucht dabei nur in der Zone des Blutauftrages etwa in einen Gußzylinder eingebettet werden, um innerhalb einer Ringdichtung zum Saugglockenraum gedreht werden zu können. Das Glukometer selbst braucht dabei nicht mitzudrehen, sondern kann fest in einer Klammer über der Saugglocke eingeschoben sein. (Der freie Teil des Teststreifens oberhalb des Grußstückes kann die Torsionsbewegung mitmachen). Der Gußzylinder weist dabei zweckmäßig oberhalb dem für die Rotation in der Ringdichtung bestimmten Teil einen den Durchmesser erweiternden Kaliberstprung mit seitliche Abplattung oder ovalärer oder noch besser polygonaler Formgebung auf. In dieses Polygon greift wie ein Schlüssel von oben ein rotierende Teil ein, der sich vorzugsweise in einen die Saugglocke überwölbenden Ring fortsetzt (Fig. 41, Fig.37 rechts oben). Wenn die Ableitungsdrähte innerhalb der Teststreifen (wobei hier in der Regel Glukometer auf der Basis von Stromerzeugung oder Stromfluß in Frage kommen) relativ starr gehalten werden, kann auch nahezu der gesammte Teststreifen durch die Plastikgußmasse verstärkt werden und das Glukometer auf einer Art Platten-"Teller" mitgedreht werden. (F.19) Die Begrenzungen der Drehbewegungen sind mannigfaltig: durch Stifte am Drehteller, die gegen einen Anschlag stoßen, durch eine Stellschraube zwischen den anschlagend bewegten Teilen; in der Regel ist sie für den jeweiligen Hauttyp individuell einstellbar. Wegen der kurzen Streckenlänge des Schnittes kommen Feingewinde bei den Einstellungsschrauben in Frage oder deren Endigung in einem Kegel, welcher stufenlos seinen Berpührungsdurchmesser mit dem Anschlag zum drehenden Teil verstellen läßt.
Die Drehung selbst kann innerhalb eines schalenförmig angeordneten Zylinderassembles erfolgen, wobei die rotierende Spiralfeder beim Niederdrücken eines überstehenden Teiles über eine Schrägschlitzführung gespannt wird. Die Auslösung der Sektordrehung erfolgt durch Freigabe des Drehzylinders im Eingriff mit dem Punktionsgriffel über Anhebung eines Sperrgliedes nach einer Vorlaufstrecke für einen die Saugglocke umgebenden (oder neben ihre stehenden) Saugzylinders nach Vakuumwirkung in der Saugglocke auf die Haut. Das gleiche Prinzip der Folgesteuerung im Zusammenhang mit der Bewegung einer vorzugsweise federbetriebenen Vakuumpumpe wird auch bei der queren oder parallelen Anordnung des Saugzylinders oder Faltenbalges zur Saugglockenachse angewandt. (Eine entsprechende Auslöseraustattung für die Auslösung auch vom Rand der Saugglocke aus wurde bereits in P 25 51 992 1974 im Zuge der kontinuierlichen Fortentwicklung der Gesamterfindung beschrieben). Außerhalb des sogerzeugenden Faltenbalgs mit Innendruckfeder können vorzugsweise zwei Stäbe an der Saugglocke vorbei oder über ihr mit entsprechenden Stäben unter Druckfederwirkung in Gegenrichtung streckenweise in Kontakt treten. Dieser Kontakt kann zur Auslösung der Schnittbewegung genutzt werden. Dabei erteilt wenigstens ein Stab unter Druckfederwirkung - in der Regel einer einseitig, aber auch der zweite über Hebelzwischenschaltung möglicherweise- dem Drehteller* das Bewegungsmoment für die Lanzettenrotation. Diese Rotatation kann aber auch über wenigstens einen von oben nach unten bewegten Stab manuell erzeugt werden, wenn von diesem ein seitlicher Vorsprung oder sein Ende innerhalb einer Lücke oder am Rande auf eine entsprechende Keilführung trifft und diese überholt. Das Keilprofil kann sich auch am Stab befinden. Zur Eröffnung der Kapillaren bedarf es mehr der Plötzlichkeit der Lanzetten-Seitverschiebung als der Streckenlänge, weil die Kapillaren einer plötzlichen Bewegung nicht ausweichen können. Hierfür können kugelschnäpperartige Raster die Stangenbewegung unter Kraftaufstau zunächst aufhalten, um eine größere Losbrechgeschwindigkeit zu erzielen. Es können aber auch - und dies kann oft zusätzlich der Fall sein- die Kräfte einer Auslösebewegung zunächst gemäß Kräfteparallelogramm so gegen eine Drehachse richten, daß zunächst ein Gegenhalt in einer Art labilem Gleichgewicht statthat. Die Überholklinke einer Stange kann auch eine Feder aufweisen, welche auf die abfallende Schulter des Keilprofils treffend, den Drehteller wieder zurückfedern läßt. Bei der Anwendung einer Kippbewegung für den Lanzettenschnitt stellt die stabilisierende Vakuumwirkung auf den Dichtungsbereich im Saugglockendach ein Problem dar. Es kann dadurch gelöst werden, daß der Lanzettenträger hinter der Schneide eine gleichmäßige Halbrundung aufweist, welche in eine ebensolche gegengewölbe Rinne im Saugglockendach eingepaßt ist. Diese Rinne weist einen Schlitz für den Durchtritt der Lanzettenschneide auf. Die Dichtung erfolgt auf der Abrollfläche, etwa bei Federandruck des mit dem Lanzettenträger bestückten Glukometers in Richtung Saugglocke. Die Kippbewegung des Glukometers auf einem schachtel artigen Basissockel kann gegen eine Feder über den Zug an einer Exzenterstange erfolgen, welcher an einem Zahnrad mit Zahnstangeneingriff erzeugt wird.(Fig.23)
Statt der Rotation des Punktionsgriffels kann auch die Haut durch eine rotierende Saugglocke mitgenommen werden. Für die Auslösung einer Schwingbewegung kann ein Stößel durch eine seitliche Dichtung in der Saugglocke hindurch bewegt werden. Ein Gegenstößel innerhalb einer gegenüberliegenden Wanddichtung kann die Schnittlänge begrenzen oder - etwa durch später wirksamen Keil betätigt - die Rückführung des Teststreifens oder Griffels in die Ausgangslage bewirken. Der Teststreifen wird bei dieser Lösung von oben durch das Saugglockendach eingeführt. Dies geschieht in Verbindung mit dem Glukometer der in eine Art Klammer im Basissockel eingeschoben ist. Über ein Scharniergelenk seitlich wird die Platte des Basissockels auf das Saugglockendach abgeklappt (vorzugsweise unter Überwindung einer Überholfeder, welche einen leichten Andruck gegen die Saugglocke garantiert). Dabei schließt eine Dichtung am Plattenende des Basissockels mit einer Gummiplatte so an eine Gegenplatte im Saugglockendach an, daß letzteres auch um den eingeführten Teststreifen abgedichtet wird. Letzterer endet in der Saugglocke in einem Gußstück mit Lanzettenspitze und Meßzone. Die Schnittbewegung erfolgt -wie vorhin geschildertüber einen Stößel, der durch eine Dichtung in der Saugglockenwand quer geführt wird. Er stößt über eine Art Keilführung von außen betätigt ruckartig gegen das Gußstück oder den Gußzylinder bzw. gegen den Teststreifen und bewegt diesen gegen einen Gegenstift. Letzterer ist durch eine Dichtung in der Seitenwand der Saugglocke hindurch von außen längenverstellbar. Die Erzeugung des Unterdrucks erfolgt nur beispielsweise und zur Variation über die manuelle Ausdehnung eines Faltenbalges. Ebenfalls nur beispielsweise wird mit dieser Bewegung über einen Sperraster die Betätigung des Stößels für die Lanzettenschwingung bewirkt. Bei dieser räumlichen Anordnung läßt sich besonders leicht ein blutfreier Vakuumkanal quer hinter den Schlitz neben dem Kissen mit der Meßzone einleiten, was den Bluteinstrom begünstigt. Im Gußzylinder ist ein durchgehender Schlitz für das Einführen des Endes eines Teststreifens vorgesehen, der eingekittet wird, falls er nicht dicht eingegossen ist (Fig.32, rechts unten im Querschnitt). Die Länge des Gußzylinders, der im oberen Abschnitt auch abgeplattet sein kann, ist sehr variabel. Der Sogkanal hinter die Meßzone kann auch von unten neben einer kleinen die Lanzette teilweise umgebenden Tülle enden. Wenn das die Reaktions- oder Meßfläche umgebende Gußstück sich in den flachen Teststreifen fortsetzt, genügt eine Ausweitung des Schlitzes, in dem dann einseitig das Ende des Teststreifens befestigt ist (auch durch nachträgliches Einkleben oder Einschieben bei Passitz unter Keilprofil). Der Unterdruck wird dann auch abseitig vom Blut auf die Testfläche hin wirksam, während auch eine Kanüle oder Rinne in der Haut nach unten verschlossen liegt. Zur Herstellung einer solchen Rinne kann bis hinter den Spitzenbereich der Lanzette gußtechnisch ein Einschub vorgesehen werden, der zweckmäßig mit dem leistenförmigen Einschub für den Teststreifenschlitz verbunden ist (Fig.32 Mitte). Die Lanzette kann auch kegelförmig sein, da die Schneidwirkung Kegelspitze zur Kapillareröffnung ausreicht.
Um den Blutaustrittskanal freizuhalten kann um das Gußstück oder Blech mit Lanzettenspitze eine Hülse vorgesehen sein, welche über Dreh- oder Schubwirkung (namentlich von außerhalb der Saugglocke oder durch den Innenmagneten) nach der Schneidbewegung über die Lanzettensspitze abwärts geschoben wird. Die Haut wird dabei aus der Lanzette herausgeschoben und das Blut sammelt sich unmittelbar innerhalb der Hülse. Eine Art solcher Hülse kann auch der Regulierung der Einstichtiefe dienen, welche bereits vor dem Einstich vom Benutzer je nach dessen Hauttyp vorgenommen wird. Um den Blutaustrittskanal frei zu bekommen kann die Schwing- oder Drehbewegung der Lanzette bei geeigneter Schneidenwahl auch so festgehalten werden, daß der Dehnungseffekt entgegen der Bewegungsrichtung einen Hautspalt erzeugt.
Da die Schnittbewegung dafür in der Regel zu kurz ist, erfolgt in diesem Falle zweckmäßigerweise eine langsame Nach-oder Ergänzungsbewegung in gleicher Richtung ohne Schneidewirkung. Die Rückbewegung der Schneide kann dabei gegen eine schwache Feder ermöglicht werden, damit - etwa bei unabsichtlich vorzeitiger Saugglockenbelüftung - keine Schnitterweiterung in der sich zurückziehenden Haut erfolgt (Fig.31).
* bzw. Dachhülse

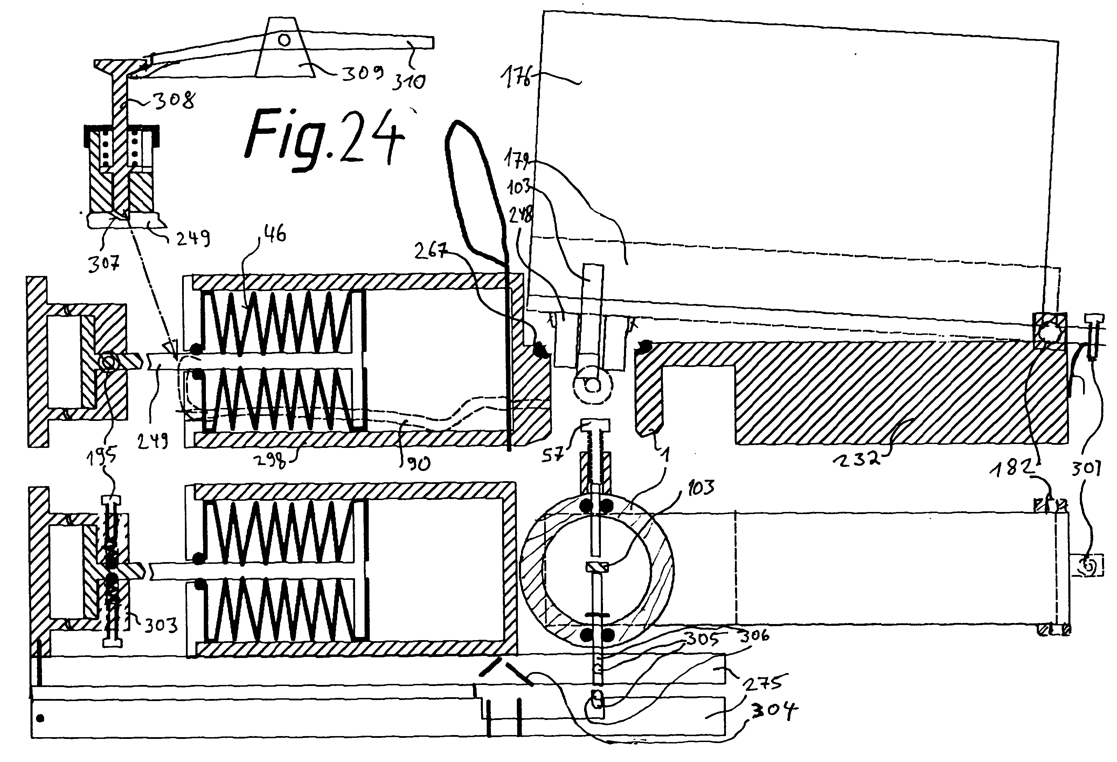

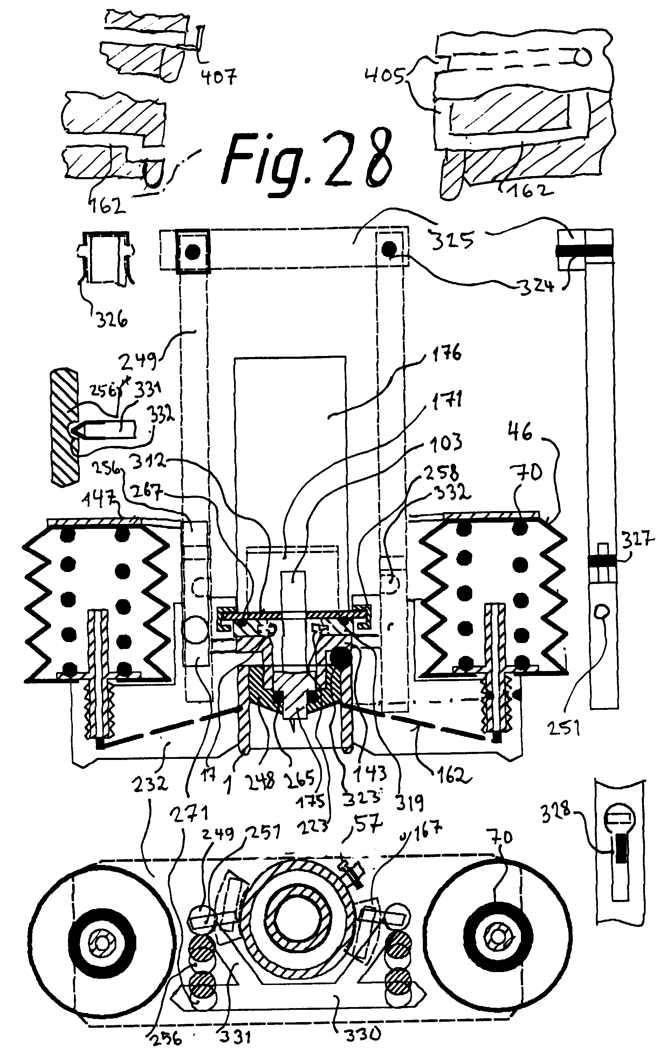

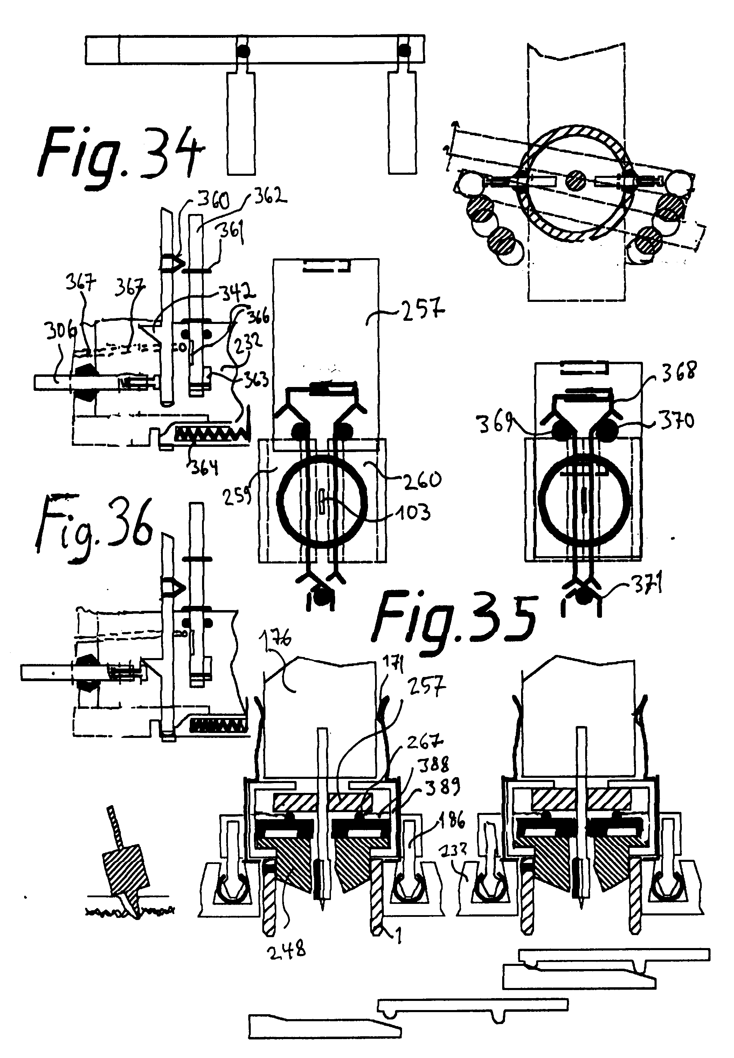

Erfolgt die Schnittbewegung über die Absenkung eines Stabes, so kann nicht nur die Freigabe der Lanzettenrückbewegung in die Ausgangslage, sondern auch die Eröffnung eines Belüftungsventiles für die Saugglocke an die Zugbewegung dieses Stabes gekoppelt werden (Fig. 39 links). Die Wiederbelüftung kann bei manueller Vakuumerzeugung auch durch Lösen des Pumpenstößelrasters bewirkt werden (Fig. 24 links oben). Bei federbetriebenen Vakuumpumpen wird der Unterdruck selbstverständlich bei manuellem Druck gegen die Feder wieder aufgehoben; in der Regel genügt aber die seitliche Achsenkippung des Gerätes, wobei sich der Saugglockenrand abhebt. Damit dies nicht vorzeitig erfolgt, wird die Brücke zwischen Saugglocke und Pumpenzylinder bis zum Saugglockenrand oder noch etwas tiefer herabgezogen. Die Körperoberfläche, die zur Punktion geeignet ist, weist ja immer eine Wölbung auf. An dieser kann dann die verbreiterte Geräteunterfläche abgestützt werden (Fig.28). Die Wiederbelüftung würde hier durch eine Kippbewegung des Gerätes um dessen Breitseite als Achse oder quer zur Bildebene erfolgen.
Um ein vorzeitiges Abheben der Saugglocke durch den Federrückschlag der Pumpe nicht zu begünstigen, könzwei Pumpen mit vertikaler Stößelachse achsenparallell zur Saugglocke diese einschließend montiert sein (Fig. 28). Zweckmäßigerweise werden die Stangen zur Auslösung der Lanzettenbewegung um eine Achse umschwenkbar gestaltet, um sie außer Benutzung der Gerätelängsseite anlegen zu können. Die Absenkbewegung der Stangen kann auch in erster Bewegungsphase die Auslöser für die Pumpen betätigen. Letzterer sind miteinander über einen Steg oder eine Leiste verbunden(Fig. 34 rechts oben).

Die Einstichtiefe kann den Hautverhältnissen dadurch angepaßt werden, daß eine Hülse mit zusätzlicher Dichtung zur Saugglocke deren Randabschluß bildet.
Es kann aber auch das Saugglockendach unter zusätzlicher Dichtung zur Grundplatte (oder zum Basisblock) gehoben oder gesenkt werden insbesondere mit Gewindehilfe. Schließlich kann auch ein Griffel oder ein Teststreifen verschieden weit in die Saugglocke eintauchen. Hierzu kann das Glukometer über einen Keil (oder eine Schraube) gegenüber dem Saugglockendach angehoben werden. Es kann auch -besonders bei Rotationsgriffeln- die Höhe des Saugglockendaches mit der Dichtung oder Eintauchtiefe des Griffels in die Dichtung (etwa durch Anhebung des in diesem Falle den Randhut des Griffels untergreifenden "Schlüssels" der Dachhülse) verändert werden (Fig.37,38) Zweckmäßigerweise sind zwei verschiedene Schnittlängen für einen Ansaugvorgang einstellbar, um bei Ausbleiben von Blutzufluß auf dem Testfeld eine Zusatzpunktion vermeiden zu können. Hierfür ist beispielsweise ein Schiebekeil vorgesehen, gegen welche eine die Schnittbewegung auslösende Stange stößt. Wird die Keilbewegungshemmung für diesen Anstoß aber (beispielsweise im Zuge der Anhebung einer Handstange) aufgehoben, so wird der Keil während des zweiten längeren Schnittes passiv zurückgeschoben (Fig.39).
Ein Punktionsgriffel mit kuppenförmigem Ende kann auch als Einmalgerät ausgebildet sein. Ein Pflasterzug mit Schlitz für die Lanzettenspitze kann in einer Eindruckmulde die Haut gegenüber der Lanzettenbewegung fixieren; die Haut kann vor der Abkippbewegung zum Schnitt aber auch zeltartig mittels des Punktionsgriffels hochgezogen werden
Zur optischen Hautkontrolle mittels Helligkeitsmeßvergleichen in einem Photometer betätigt in einem Beispiel ein Elektromagnet einen Stift mit Schneckenführung, der Licht über Leitfasern und/oder Linsen zuerst in Richtung Haut lenkt. Ist diese frei von unsicheren Schattengebungen (also innerhalb vorprogrammierten Helligkeitsdifferenzen) im Einstichbereich, so bewirkt die Magnetbewegung den Richtungswechsel des Lichtes auf eine optische Glukosemeßzone. (Fig.14)
Gegenstand der Erfindung waren aber hauptsächlich nicht nur die dargestellten Einzeleinrichtungen, die sich fast beliebig vermehren ließen, sondern die funktionellen Besonderheiten, welche samt Baumerkmalen zu ihrer Lösung auch in beliebiger Kombination geschützt sein sollen, in erster Linie, um die Schnittwegung in Schnittlänge und Zeitdauer auf ein den individuellen Verhältnissen angemessenes Minimum zu begrenzen.
Außer dem ständigen Ziel eines minimal-invasiven Vorgehens - auch schon zur Vermeidung größerer Hautnarben - wurde auch der Vorgabe der Einfachheit der Handhabung dadurch Rechnung getragen, daß oft mehrere Funktionen mit einem Hebelzug -bzw.-druck verknüpft wurden. Am nächsten kommt diesem Ziel eine Einrichtung, in welcher eine Vielzahl von Testfeldern auf einem Band aufgereiht sind, welches den manuellen Punktionsgriffelwechsel - aber auch den direkten Blutkontakt unter Einsparung von Leitungsdrähten in den Teststreifen selbst- erübrigt.

### Kurze Beschreibung der Ausführungsbeispiele

Die Fig.1 gibt oben in einem Längs- und unten in einem Querschnitt in natürlicher Größe eine Vorrichtung zur visuellen Kontrolle der Punktionsstelle wieder.
Die Fig.2 ist ein schematisierter Längsschnitt durch eine elektromagnetisch betriebene Einrichtung bei Auslösung der Schnittbewegung vom Saugglockendach her.
Die Fig.3 zeigt links einen Teststreifen in Aufsicht, darunter einen Querschnitt durch das Testfeld des Teststreifens, wie er etwa bei einer Einrichtung nach Fig.8 angewandt werden könnte. Die Schneidspitze liegt zentral.
Die Variation in der Mitte unten zeigt eine Variante - unten in Aufsicht - mit Schneidspitze seitlich des Testfeldes. Oben ein Querschnitt längs der Schnittlinie A - B des Längsschnittes. Rechts die Variante eines Teststreifens mit nach unten die Längsachse verlängerter Schneidspitze in ihrer Verpackung. Rechts davon ein solcher Teststreifen im Querschnitt, im Testfeldbereich in eine Halteklammer eingeschoben.

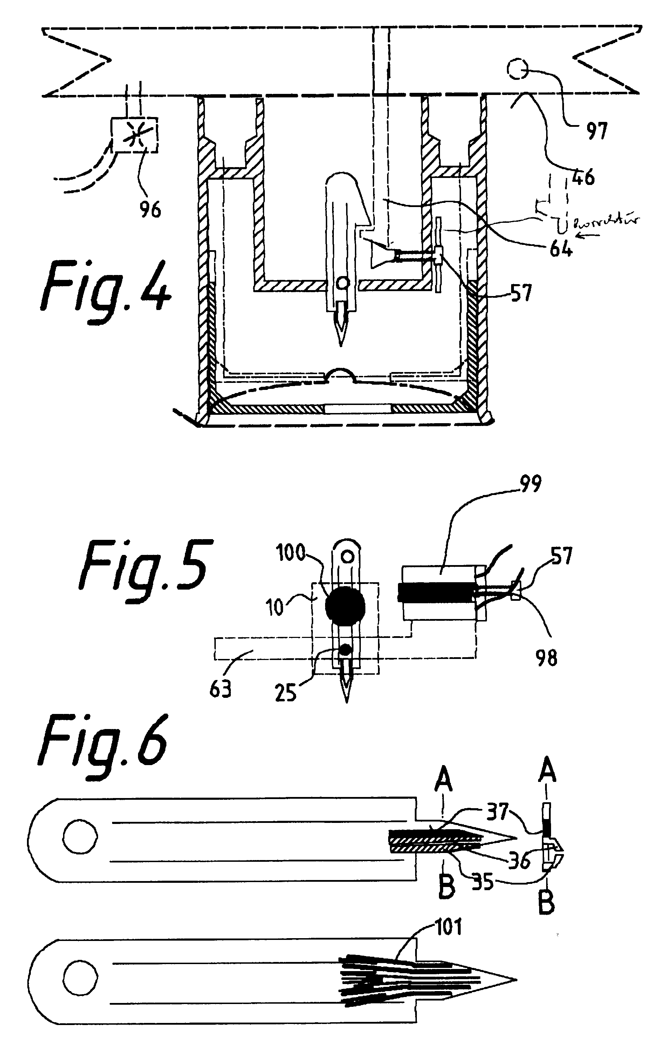

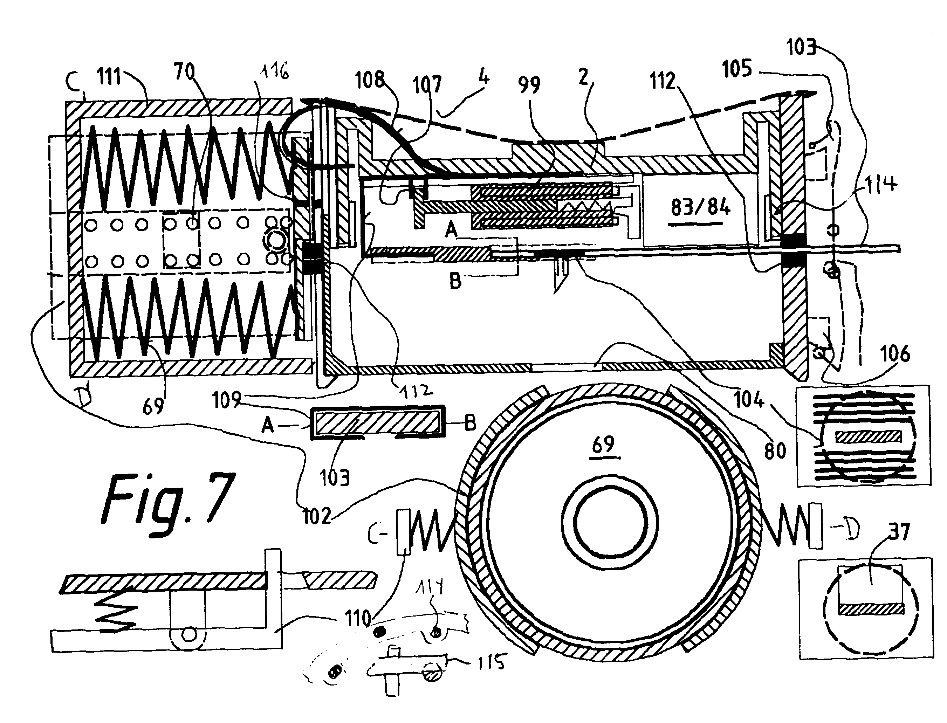

Die Fig.4 zeigt im Längsschnitt eine Einrichtung ähnlich derjenigen in Fig.2; es ist noch ein Sperrmechanismus für die Verhinderung der Punktion nach optischer Kontrolle der Punktionsstelle behandelt.
Die Fig.5 zeigt in natürlicher Größe im Längsschnitt das DEtail des Antriebes einer Kipplanzette mittels eines Elektromagneten.

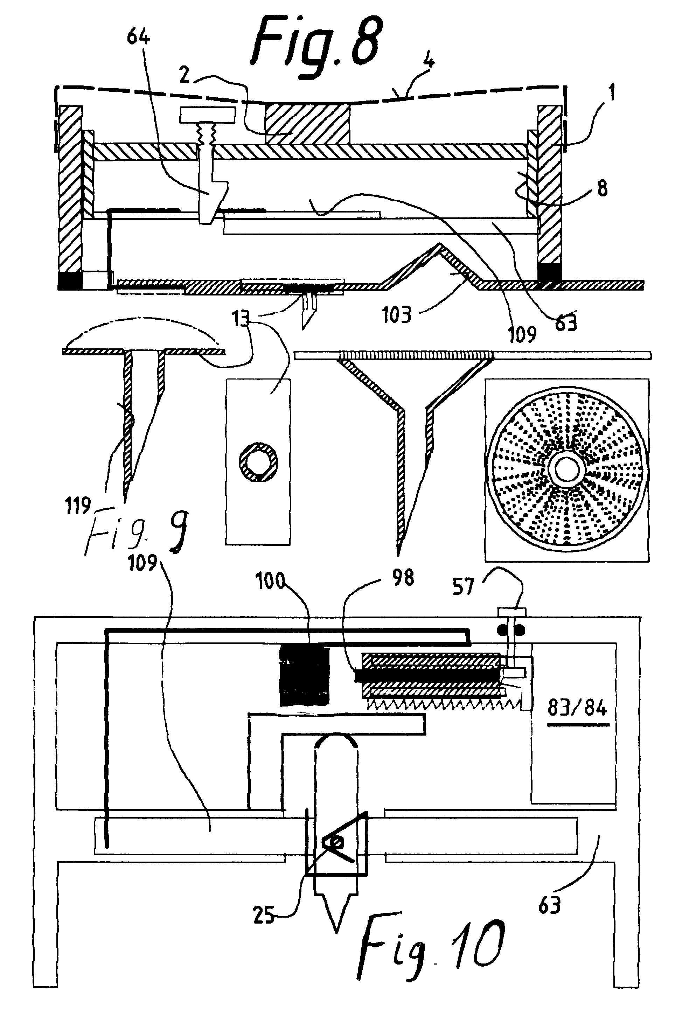

Die Fig.6 zeigt im Maßstab 4 : 1 zwei Varianten einer Punktionslanzette für die Blutableitung. Rechts oben das Detail eines Querschnittes längs der Schnittlinie A - B des Längsschnittes.
Die Fig.7 gibt eine Einrichtung mit horizontal-haut-paralleler Schnittausführung und Direktanschluß an ein Meßgerät (Glukometer) im Längschnitt natürlicher Größe.

In der unteren Hälfte erläuternde Details: so in der Mitte unten ein Querschnitt durch die Vakuumpumpe längs der Schnittlinie C - D des Längsschnittes oben. Ganz links ein Längs- oder Horizontalschnitt durch einen der Auslöser für die Pumpe. Darüber ein Vertikalschnitt-Detail längs der Schnittlinie A - B des Längsschnittes über die Führung des Teststreifens. Rechts im Maßstab 2 : 1 die Aufsicht auf das Testfeld in zwei Varianten.
Die Fig.8 zeigt im Längsschnitt eine Variante einer Einrichtung ähnlich derjenigen der Fig.7, die aber auch ohne Saugglocke betrieben werden kann. Die Saugpumpe als Antrieb für die Schnittbewegung wurde weggelassen.
Die Fig.9 zeigt zwei Varianten von Schneidspitzen für einen Teststreifen einer Einrichtung nach Fig.8 im Maßstab 4 : 1, die rechte Variante auf dem Testfeld montiert. Zur linken Variante ist rechts davon in natürlicher Größe der Querschnitt unterhalb des Teststreifens eingezeichnet.
Die Fig.10 im Längschnitt im Maßstab 2 : 1 schematisiert eine Einrichtung für die Schnittdurchführung mittels Anregung einer seitlichen Lanzettenkippung durch einen Elektromagneten ähnlich wie in Fig.5.
Die Fig.11 zeigt oben im Längsschnitt, unten im Querschnitt längs der Schnittlinie A - B des Längsschnittes, in natürlicher Größe einer Punktionseinrichtung mit Schnitterweiterung durch Rotation in konzentrischer Konstruktion. Rechts unten ist im Detail im Längschnitt ein Markierungsdorn für die visuelle Hautkontrolle dargestellt.

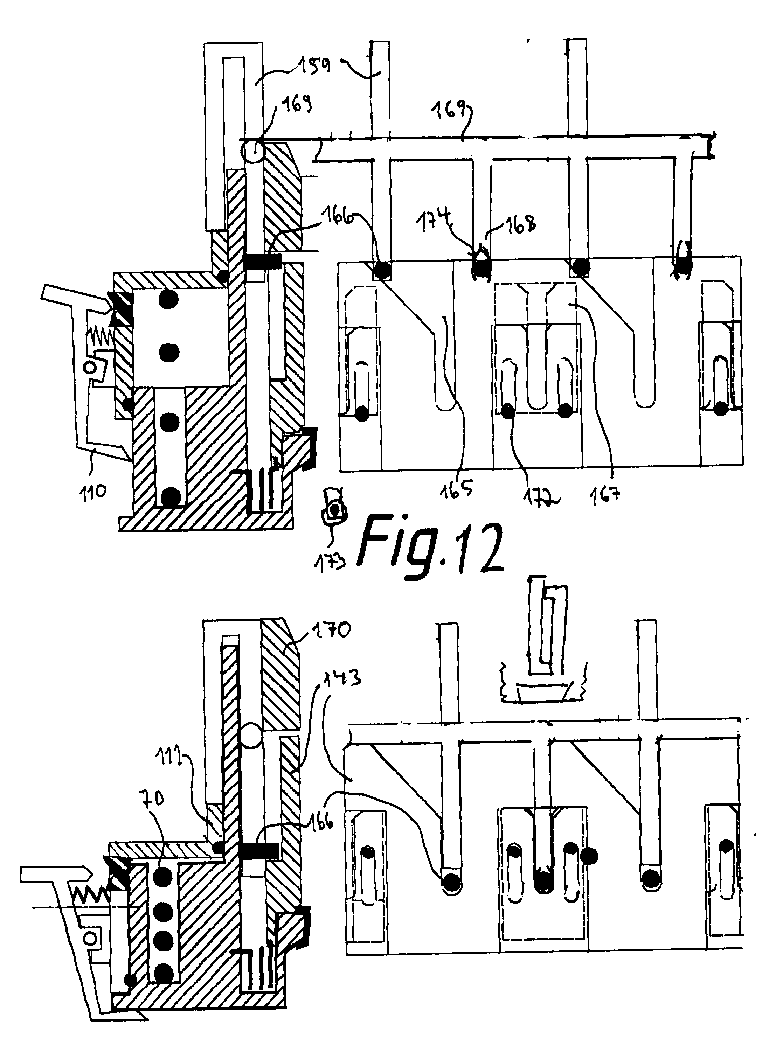

Die Fig.12 soll die Funktion einer Einrichtung nach
Fig.11 näher erläutern unten zeigt linksseitig Längsschnitte durch die linke Hälfte der Einrichtung. Oben ist eine Phase des erweiterten, unte eine Phase vor der Sogerzeugung wiedergegeben. Links davon jeweils Aufrollungen der seitlich und über dem Saugglockendach befindlichen Funktionsglieder für die plötzliche Auslösung der Schnittbewegung. Am unteren linken Rand der oberen Aufrollung die Variante einer durch eine Rolle am Stiftende erleichterten Bewegung. Oberhalb der unteren Abwicklung ein Längsschnitt-Detail und darunter ein Querschnitt-Detail mit Schwalbenschwanzführung als Varianten zur Bewegungsbegrenzung der gegenseitig bewegten bzw. gegeneinander bewegten Teile.
Die Fig.13 zeigt oben im Längsschnitt unten im Querschnitt längs der Schnittlinie A - B des Längsschnittes eine Einrichtung nach den Fig.11,12 unter Darstellung auch des Punktionsgriffels in Verbindung mit dem Meßgerät hauptsächlich zur Demonstration der Begrenzung der Schnittbewegung mittels einer Stellschraube.
Rechts zwei kleine Querschnitte durch den Punktionsgriffel zur Darstellung zweier Varianten der Schneidspitzenlage in Bezug auf den Griffelmittelpunkt.
Rechts davon das Längsschnittsdetail einer Lösung der Rotationsverzögerung durch die Stiftführung in einer Hülse in Ersatz des Schiebermechanismus in den Aufrollungen der Fig.12.
Die Fig.14 zeigt schematisiert in etwa natürlicher Größe oben im Längsschnitt eine Punktionseinrichtung mit zwei Blasebalg-Pumpen seitlich des Glukometers in Aktionsstellung, darunter eine Saugglocke als Wegwerfteil in Verbindung mit einer Vorrichtung zur optischen Hautkontrolle sowohl als auch zur anschließenden Gewinnung optischer Meßsignale aus dem Testfeld für das Glukometer. In der Mitte ein Detail der optischen Meßwertabnahme-Anordnung in Verbindung mit der Saugglocke (als Wegwerftöpfchen) im Maßstab 2 : 1. Rechts ein Querschnittsdetail durch die von einem Gummeschlauchende umgebenen Schneidspitze (um 90 Grad gedreht gegenüber dem Längsschnitt) Unten ist in einem Horizontal- oder Querschnitt der Umschaltmechanismus für die optischen Meßvorgänge durch die Funktionsglieder erläutert.
Die Fig.15 zeigt oben in einem Längschnitt im Maßstab 2 : 1 eine Saugglocke als Wegwerftöpfchen mit Vakuumspeicher in Verbindung mit einem Meßgerät.
Darunter im Längsschnitt und zugehöriger Aufsicht (ganz unten) ebenfalls Maßstab 2 : 1 eine Hilfsvorrichtung zur visuellen Hautkontrolle.

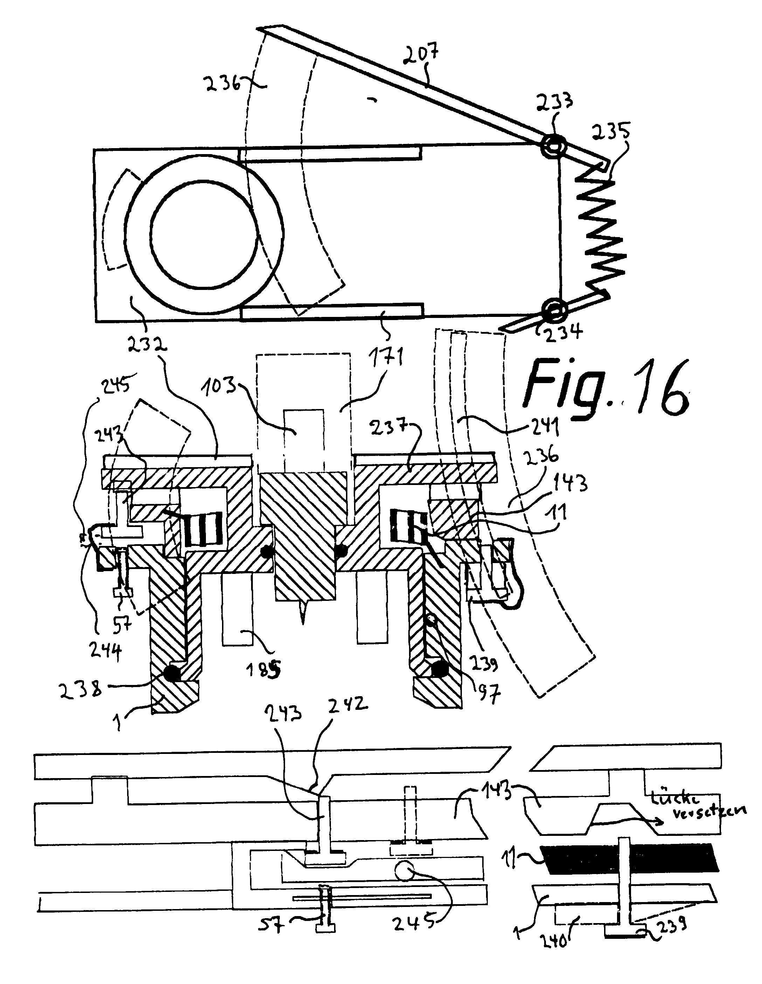

Die Fig.16 zeigt oben die Aufsicht auf eine Punktionseinrichtung nach dem Rotationsprinzip. Die beiden Deckelklappen für den Betrieb der Blasebalg-Vakuum-Pumpen sind nur teilweise eingezeichnet. Die Saugglocke links in der Gerätegrundplatte - das Glukometer zwischen der Klammer wurde weggelassen - ist in der Mitte im Maßstab 2 : 1 im Längsschnitt wiedergegeben. Unten in Aufrollung dazu die Funktionsglieder des Überholmechanismus zur sektoriellen Bewegung der Schneidspitze.

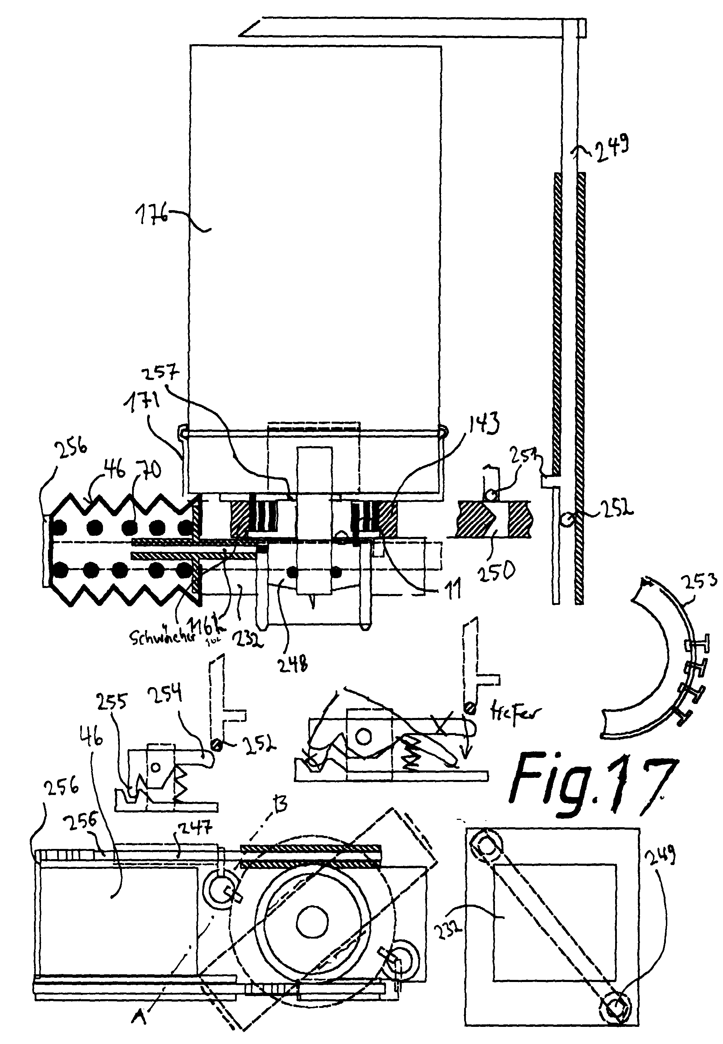

Die Fig.17 zeigt oben im Längsschnitt eine Punktionseinrichtung mit aufgesetztem Glukometer. Rechts davon das kleine Längsschnitt-Detail zur Aufzeichnung der Kulissenführung für die Schnittbewegung. Ganz rechts ein Längsschnitt durch eine Hülse mit der zugehörigen Betätigungstange für die Ausführung der Schnittbewegung.
Darunter ganz rechts das Querschnittsdetail für die Darstellung der Begrenzung der Schnittbewegung durch einen Einsteckstift. In der Mitte das Detail einer Auslöserwippe für die Sogpumpe, links vor und rechts nach Auslösung. Unten links ein Quer- oder Horizontalschnitt durch die Gundplatte mit der Stangenführung für die Auslösesteuerung mit schräger Anordnung des Glukometers darüber. Rechts unten schematisierte Vertikalansicht eines Etuis mit Grundplatte und zur Aufbewahrung aus der Grundplatte herausgezogenen Betätigungsstangen, zur Aufbewahrung diagonal im Etui angeordnet.

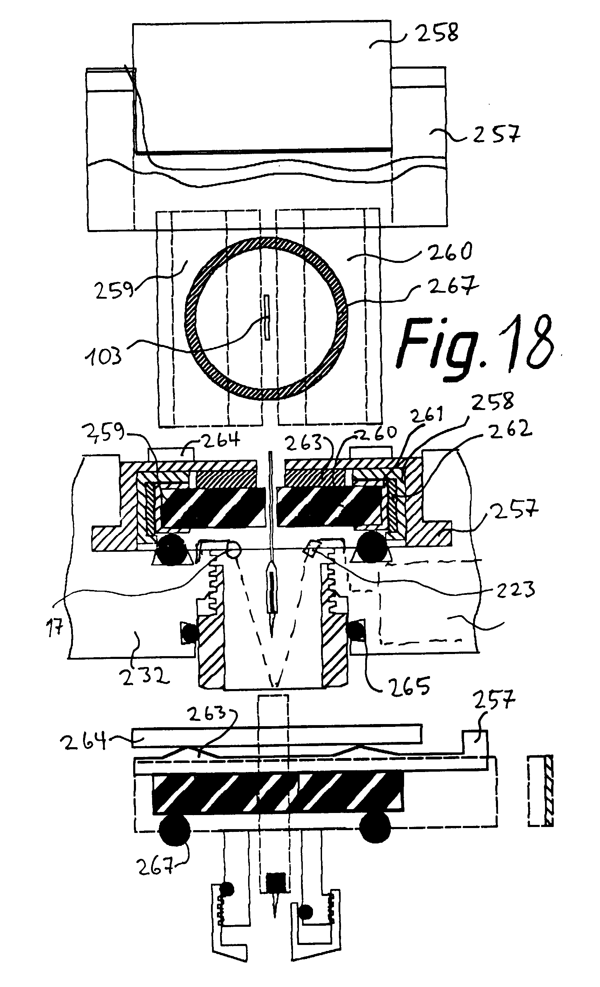

Die Fig.18 zeigt oben in verkürzter Aufsicht die Schieber
für den dichtenden Deckelabschluß einer Saugglocke.
In der Mitte der zugehörige Längsschnitt durch die Saugglocke kurz vor Abdichtung eines eingeführten Teststreifens im Deckelbereich. Es wird auch die Möglichkeit des Einbaues einer optischen Hautkontroll-Vorrichtung demonstriert. Unten (um 90 Grad gedreht) wieder ein Längsschnitt durch den Deckelbereich. Für die Saugglocke darunter wurden je zur Hälfte zwei Varianten dargestellt, um den Abstand des Saugglockenrandes von der Schneidspitze regulieren zu können.

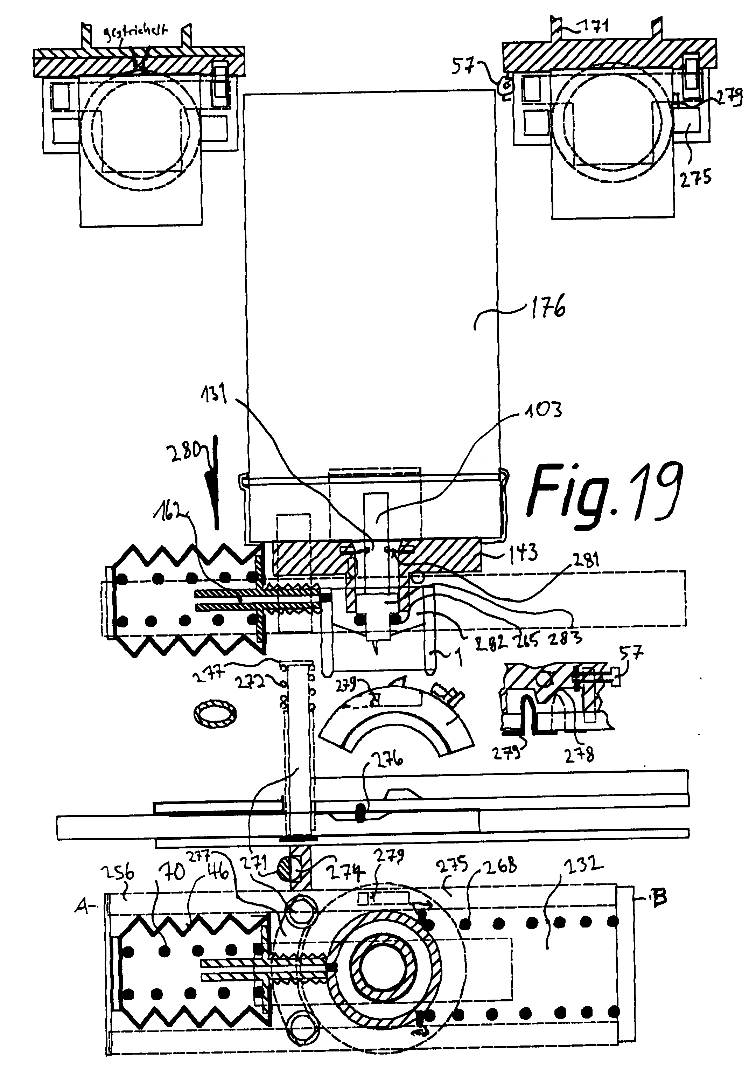

Die Fig.19 zeigt in der Mitte im Längsschnitt ein Punktionsgerät in natürlicher Größe nach dem Rotationsprinzip. Oben links zwei Varianten der Verbindung zum Glukometer, links dieser bei der Schnittbewegung feststehend, in rechter Variante mitdrehend.
Unter dem Faltenbalg der Pumpe im Längschnitt der kleine ovale Vertikalschnitt zur Montageerleichterung der Tülle für die Befestigung des Faltenbalges. In der Mitte ein Querschnitt-Detail zur Demonstration der Begrenzung der sektoralen Schnittbewegung. Ganz rechts eine Aufrollung in einem Längsschnitt Detail zur Demonstration des Überholmechanismus bei der Schnittbewegung.
Darunter im Längsschnitt längs der Schnittlinie A - B des Querschnittes ganz unten die Demonstration des Auslösers zwischen den gefederten Stangen für die Pumpe und für die Schnittbewegung. Zwischen Querschnitt (ganz unten) und beschriebenem Längsschnittdetail das kleine Querschnittsdetail der Auslösetaste für die Pumpe in der Phase bereits erfolgter Auslösung.

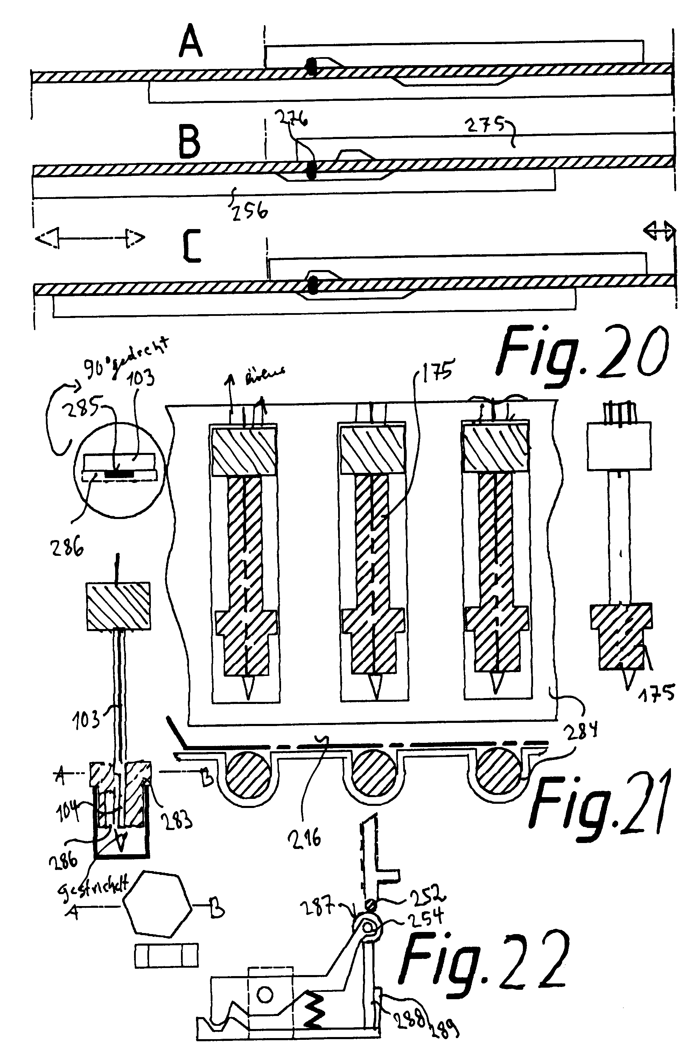

Die Fig.20 gibt schematisch in Längsschnitten die Funktionsphasen der Auslösung zwischen den Stangen der Fig.19 wieder.
Die Fig.21 zeigt in natürlicher Größe Punktionsgriffel nach dem Rotationsprinzip innerhalb einer Verpackungsfolie. Links ist eine Griffelvariante mit zwei zylindrischen Auftreibungen dargestellt. Oben ein Querschnitt dazu in Höhe des Testfeldes, unten ein weiterer längs der Schnittlinie A - B des Längsschnittes mit dem Sechskant (ganz unten von der Seite). De Griffel ganz rechts im Längsschnitt entspricht dem links dargestellten, ist aber um 90 Grad gedreht.
Die Fig.22 zeigt eine Variante der Auslöserwippe in Fig.17 in Seitenansicht in natürlicher Größe.
Die Fig.23 wird in der Mitte von der Darstellung einer Punktionseinrichtung mit Glukometer im Längsschnitt beherrscht. Hier wird eine Schwinglanzette an der halbkreisförmigen Griffelfrontseite gegen das Saugglockendach abgedichtet. Oben Mitte ist ein Querschnitt durch die Aufnahmewanndes des Glukometers gezeichnet.
Links davon oben im Längs-und unten im Querschnitt schneidspitzennahe ein Punktionsgriffel mit Ausstattung für eine visuelle Hautkontrolle. Ganz rechts derselbe Griffel im Vertikalschnitt (also 90 Grad gedreht) und unten im Querschnitt längs der Schnittlinie A - B des Vertikalschnittes.
Die Fig 24 zeigt oben im Längsschnitt und unten im Querschnitt eine Punktionseinrichtung mit Glukometer unter Vermeidung von Antriebsfedern für den direkten manuellen Antrieb sowohl der Pumpe als auch der Schnittbewegung.
Oben links ein Detail eines Wippschalters an der Pfeilstelle snkrecht zum Längssschnitt zu denken(also einem Querschnitt entsprechend; auf dem unteren Querschnitt weggelassen). Unten das Detail eines Längsschnitte durch die Stange für die schwingend ausgeführte und durch die Saugglocke seitlich hindurch durchgeführte und längenkontrollierte Schneidebewegung.

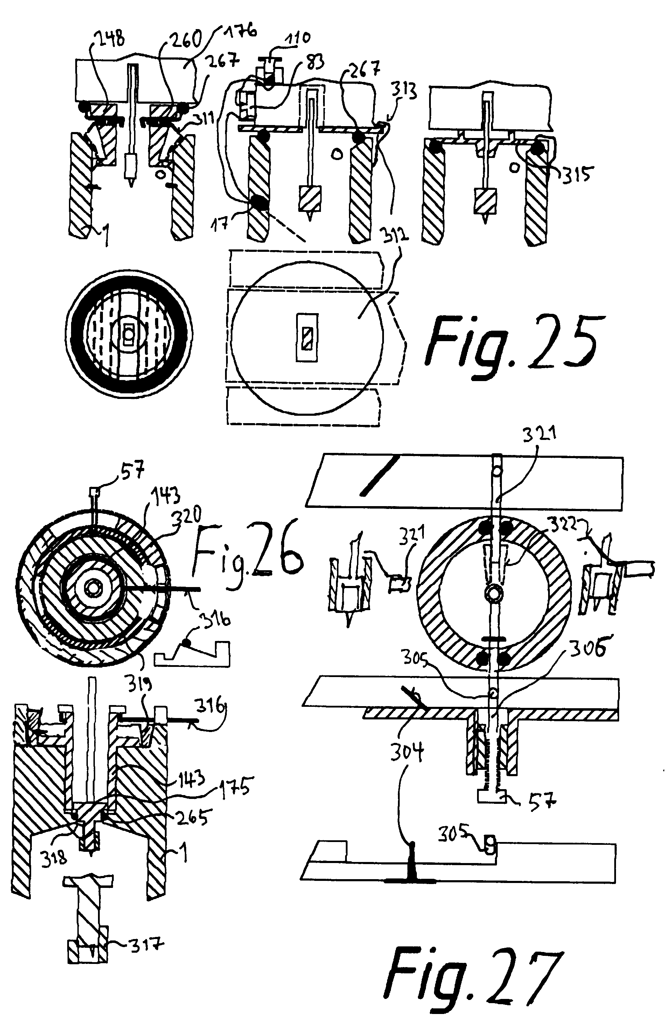

Die Fig.25 zeigt in Längsschnitt-Details in natürlicher Größe drei Varianten eines Deckelabschlusses der Saugglocke zum Meßgerät. Für die ersten beiden sind unten Querschnitte mit Schnittführung dicht unterhalb dem Glukometer eingezeichnet.
Links auf Fig.26 wird oben im Quer- und unten im Längsschnitt eine Punktionseinrichtung nach dem Rotationsprinzip gezeigt. Die kleine Aufrollung im Vertikalschnitt- Detail in Höhe der Dachpartie der Grundplatte, zeigt den Mitnahmemechanismus für den Griffel und (auf dem Querschnitt) zugleich dessen Begrenzung durch eine Stellschraube. Das kleine Querschnittsdetail unten zeigt, wie eine kleine Tülle um die Schneidspitze nach deren Rotation gegen die Haut geschoben wird,
die aus dem Wundspalt geddrängt wird. (Die Haut wurde nicht eingezeichnet).

Die Fig.27 rechts zeigt oben ein Querschnitt-Detail durch die Saugglocke eine einstellbaren Schwingmechanismus für einen unten vorzugsweise abgerundeten Griffelteil mit dem Testfeld und der benachbarten Schneidspitze.
Zu beiden Seiten das kleine Längsschnitt-Detail zu Seiten der Saugglocke zeigt links die Phase vor der Abdeckung der Hülse um den Punktionsgriffel durch eine kleine Feder am Stößel. Rechts wird die Phase nach Abdrängung der Schneidspitze von der Haut gezeigt.
Unten ein Stangen-Längsschnitt zur Demonstration des Stößelmechanismus für die Schnittbewegung.
Die Figur 28 zeigt in der Mitte im Längsschnitt eine Punktionsvorrichtung mit Glukometer für einen Rotationsgriffel und zwei symetrisch neben der Saugglocke angeordnete Faltenbälge, deren Achsen parallel zu Saugglockenache verlaufen. Das Glukometer wird von zwei Betätigungsstangen mit Querholm eingerahmt, von denen eine um 90 Grad achsengedreht links als Detail herausgezeichnet ist.
Links vom Querholm ein Vertikal-Detail zur Demonstration der Koppelung des Querholms mit der Betätigungsstange. Darunter das Detail der Betätigungsstange in Gestalt einer von dieser ausgehenden horizontalen Feder, welche bei Absenkung der Bestätigungsstange in die Rastnut der Raststange für die Pumpenauslösung einrastet. Die Vertikalsicht im Detail rechts unter der Betätigungsstange zeigt, daß für diese Auslösebewegung freies Spiel für die Absenkung der Betätigungsstangen geschaffen wurde. Unten ist der dem Längsschnitt zugehö-Querschnitt am oberen Grundplattenende dargestellt. Die Fig.29 zeigt in natürlicher Größe einen Vertikalschnitt mit der Bohrung für die Demonstration des Rastermechanismus zwischen den Betätigungsstangen der Fig.28. Rechts ist eine Variante der Betätigungsstange mit speziellem Rastmechanismus für eine ruckartige Schnittbewegung herausgezeichnet. Links in einem Längsschnittdetail und darunter in der Draufsicht eine spezielle Deckelvariante für den Abschluß der Saugglocke. Die Fig.30 demonstriert die sektorale Schnittbewegung bewirkt durch Keilführungen an der Betätigungsstange einer Vertikalsicht mit Aufrollung des Sektorschlitzes. Darunter ein Querschnittsdetail und zuletzt das kleine Querschnittsdetail direkt auf der Hautoberfläche mit eine Schneidspitzen-Variante.
Die Fig.31 zeigt links einen Querschnitt durch das Detail einer Saugglocke mit Stangensteuerung in mehr konzentrischer Lage und einen speziellen Mechanismus für die Schneidspitzen-Rückfederung; rechts in Vergrößerung unter Winkeldrehung dieser Mechanismus für eine Punktionseinrichtung etwa nach Fig.28, aber mit Schwinglanzette. Die Betätigungsstangen sind um 90 von der Vertikalen in die Hcrizontale umgeschlagen.
Die Fig.32 zeigt in natürlicher Größe die Vorbereitung eines handelsüblichen Meßstreifend durch Kuppierung des Testfeldrandes, dann Schnittmuster einer blechernen Hülle für das Testfeld; als Drittes nach untengezält einen Längsschnitt mit aufsteckbarer Zusatzhülse für die Regulierung der Einstechtiefe. Ganz unten ein Querschnitt in Höhe des Meßfeldes.
Die Fig.33 zeigt einen Teststreifen mit rundlichem Gußstück und deutet in strichpunktierter Linie wieder das Kuppieren des Testfeldrandes an. Darunter im Maßstab 2 : 1 ein Formeinschub für das Gußstück für die Entfernung nach oben und darunter zwei Querschnittsvarianten.
Die Fig.34 zeigt links eine Diagonalansicht bei um ein Scharniergelenk umgelegter Betätigungsstange vor dem Gebrauch der Punktionseinrichtung. Rechts die zugehörige Aufsicht(gestrichelt), eigentlich aber ein Querschnitt-Detail mit Saugglocke und umgebenden Stangen.
Die Fig.35 zeigt oben in Aufsicht einen Schiebermechanimsu für den Saugglocken-Deckelabschluß, links geöffnet, rechts geschlossen.
Darunter im Längsschnitt die Variante eines solchen Deckelabschluß-Mechanismus, links geöffnet, rechts geschlossen. Darunter das Detail einer Vertikalansicht auf einen Deckelschieber für den Andruck des Gummidichtringes von oben, links unten geöffnet, rechts oben geschlossen.
Die Fig.36 in zwei Funktionsphasen einen Stößelmechanismit langsamer Nachschwingung der Schneidespitze und Wiederbelüftungsmechanismus. Unten das Längsschnitt-Detail durch Griffelende und Hautwunde.
Fig.37 zeigt drei Varianten für die Höheneinstellung des Griffels in der Saugglocke im Längsschnitt. Links darunter in Längs- und seitlichem Vertikalschnitt eine Variante der Griffelhöhenregulierung mittels Schiebers. Daneben rechts eine weitere Längsschnitt-Variante.
Die Fig.38 zeigt einen solchen Deckelschieber in Kombination mit einem abhebbaren Deckelschlußmechanismus.
Die Fig.39 zeigt einen Mechanismus für zwei verschiedene Schnittlängen bei einer Schwinglanzette, links oben in der Phase vor dem ersten kürzeren, und rechts unten in der Phase nach dem längeren Reserveschnitt.
Oben jeweils ein schematisierter Längsschnitt und unten ein Querschnitt in Höhe des sperrenden Riegels.
Links unten die Fig.40 zeigt oben im Längs- und unten in einer Draufsicht von unten einen speziellen flachen Punktionsgriffel mit einer Art Kanülenspitze (vgl.Fig.9) wobei ein sich neben dem Testfeld nach oben öffnender Spalt bei geschlossenem Gußstückrand um die Kanülenspitze unten befindet. Es baut sich so ein Druckgefälle zwischen Verletzungsstelle und Testfeld auf, welches den Blutstrom dorthin begünstigt und kanalisiert. Die Fig.41 zeigt im Maßstab 2 : 1 oben in einem Querschnitt nahe der Oberkante der Einrichtung und unten in einem Längsschnitt eine Punktionseinrichtung für zwei verschiedene Schnittlängen vorstellt. Wie in den letzten Beispielen überhaupt wurden die Vakuumpumpe und die Meßvorrichtung weggelassen.
Links in der Mitte noch das Längsschnittdetail in natürlicher Größe für eine Punktiongriffelabdichtung direkt in einer halbkugeligen Dacheinsenkung unter leichter Federdruckeinwirkung abwärts auf den Punktionsgriffel Es handelt sich hier um einen Rotationsgriffel (kleines Querschnittesdetail unten). Aber auch eine Schwingbewegung wäre durchführbar.

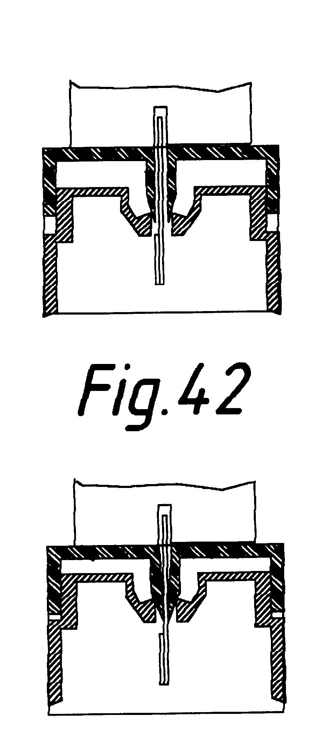

Fig.42 zeigt Längsschnitte durch eine Saugglocke mit vereinfachter Konstruktion für die Abdichtung beim Deckelschluß in zwei Funktionsstadien und natürlicher Größe.

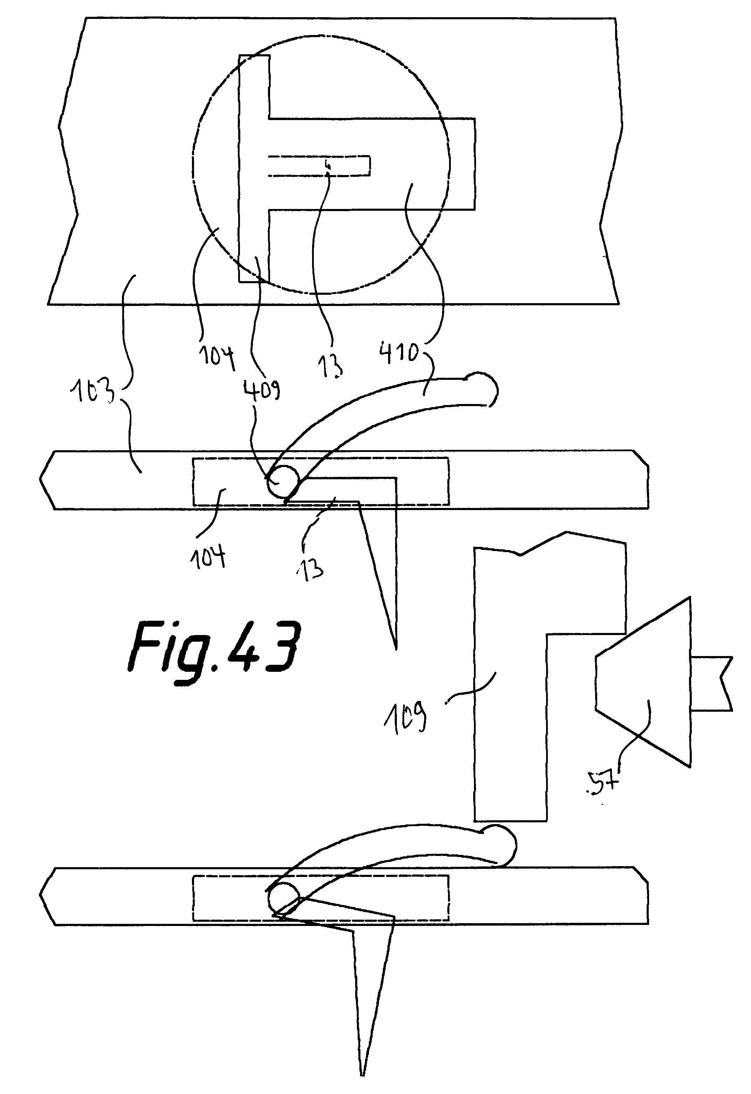

Die Fig.43 zeigt, im Maßstab 6 : 1, oben in einer Draufsicht, in der Mitte und unten im Längsschnitt, das Detail eines Teststreifens, welcher selbst in sich einen Schwingmechanismus für die Punktionsspitze enthält.

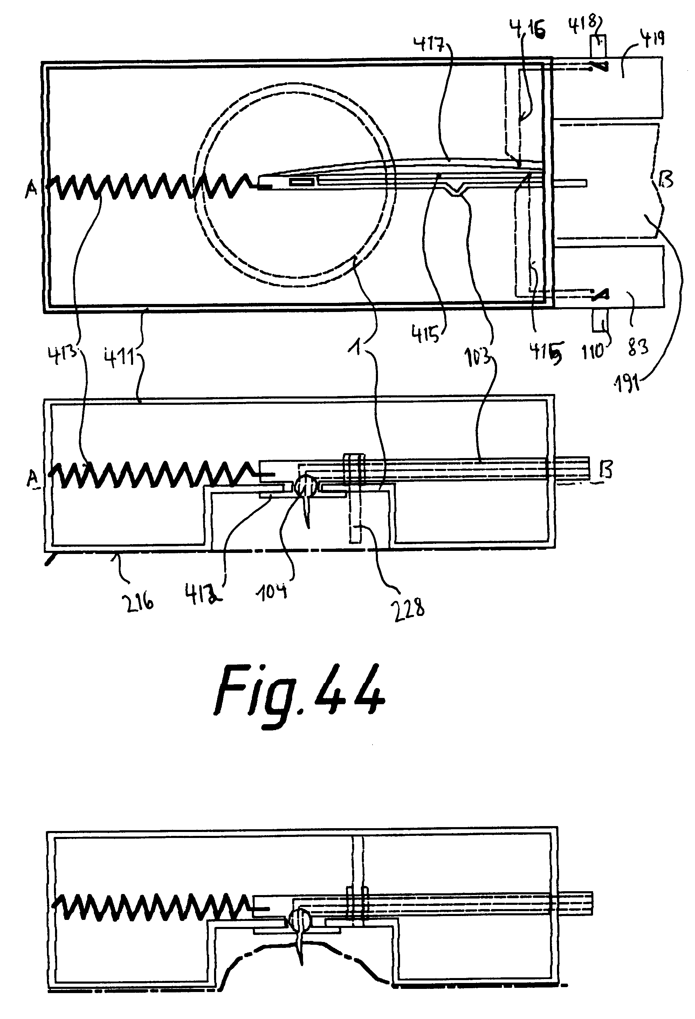

Die Fig.44 zeigt, oben im Querschnitt und in der Mitte und unten im Längsschnitt eine Einrichtung für den Einmalgebrauch mit elektrischer Auslösung des Schneidemechanismus.

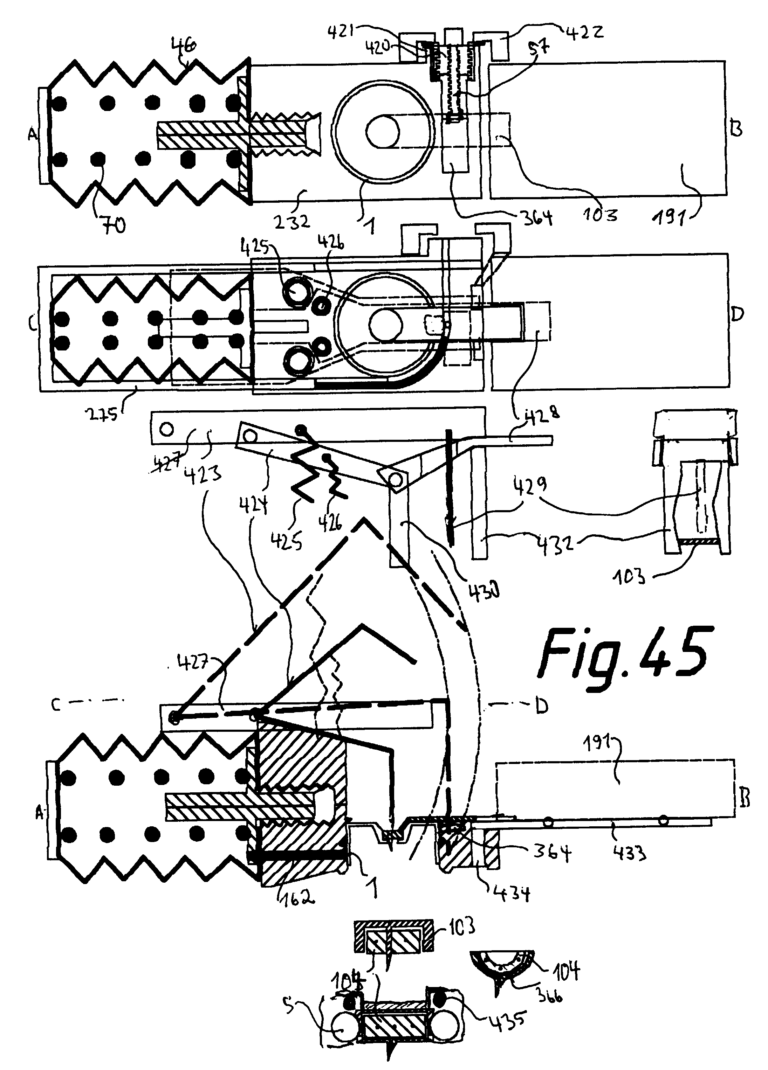

Die Fig.45 bringt in natürlicher Größe oben in zwei Querschnitten in verschiedener Höhe und unten im Längsschnitt eine Punktionseinrichtung für einen rotierenden Punktionsgriffel mit speziellem Deckelmechanismus. In der Mitte in einer Seitenansicht der Deckelschieber, rechts davon eine Vertikalansicht. Ganz unten sind kleine Längsschnitte zu erkennen, welche durch spezielle Teststreifenenden mit Testfeld und Schneidespitze gehen.
Die Fir.46 zeigt im Maßstab 2 : 1 stark schematisiert in einem Längsschnitt eine Punktionsvorrichtung ohne Sogquelle für einen um eine Dachachse schwingenden Punktionsgriffel bei elektromagnetischem Antrieb des Schneidemechanismus. Unten ein Vertikalschnitt durch die Umgebung der Stellschraube für die Regulierung der Schnittlänge.

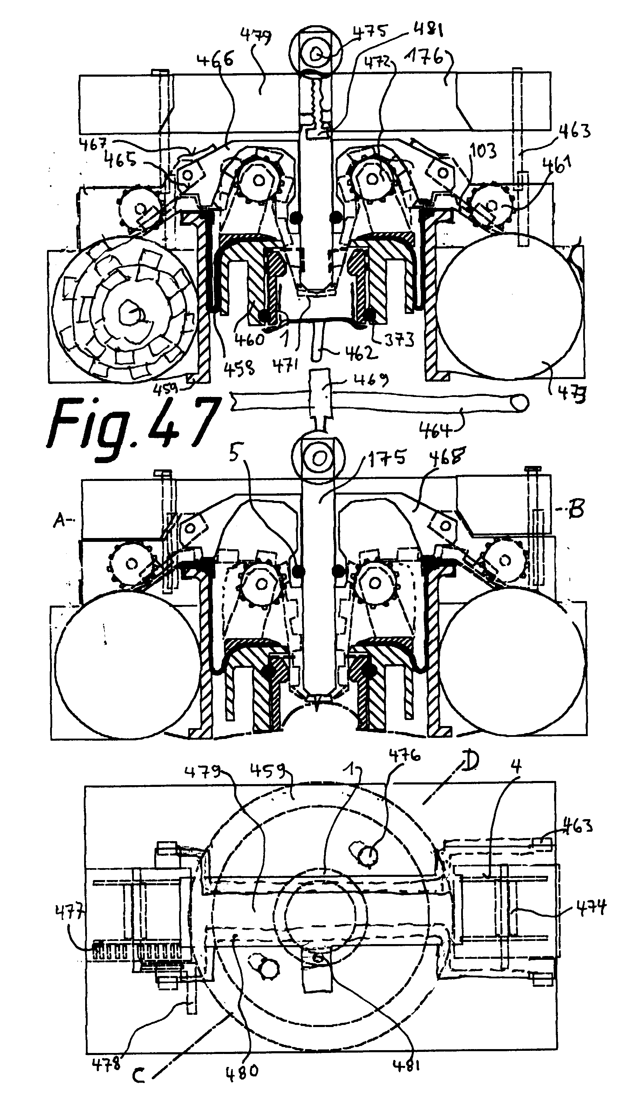

Die Fig.47 bringt in natürlicher Größe oben oben und in der Mitte Längsschnitte durch eine Punktionseinrichtung mit automatischem Testfeld- und Schneidespitzenwechsel unter Banddurchzug. Oben ist das Stadium vor der Hautpunktion dargestellt, unten dasjenige während der Punktion. Unten ein Querschnitt längs der Schnittlinie A - B des mittleren Längsschnittes.

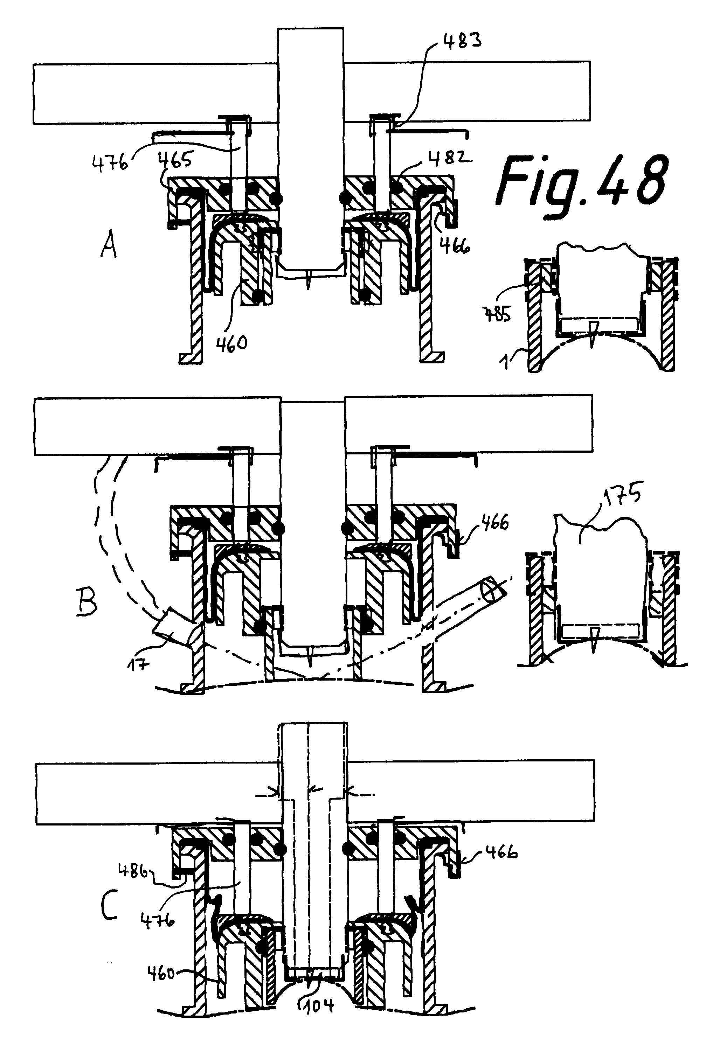

Die Fig.48 zeigt in drei Längsschnitten drei Funktionstadien einer Einrichtung nach Fig.47 bei Schnittführung längs der Linie A - B des Querschnittes auf Fig.47. Rechts zwei Details im Längsschnitt durch die Saugglocke, um die verschiedene Absenkbarkeit der Saugglocke infolge ihrer Elastizität zu demonstrieren.

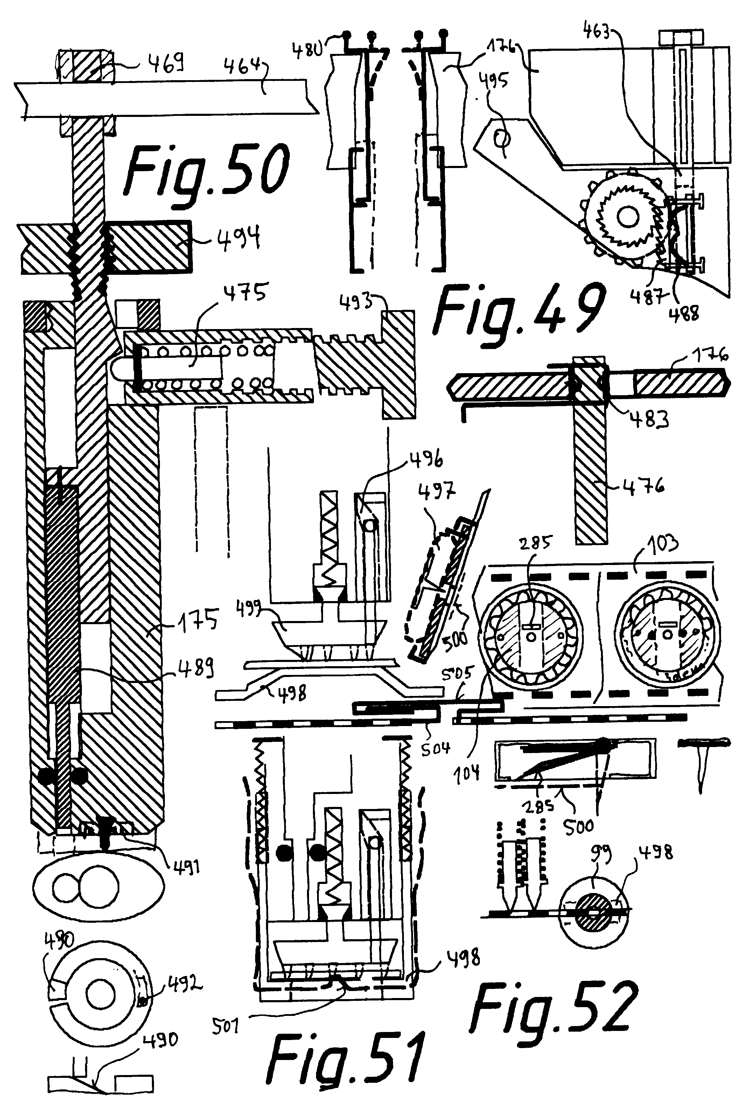

Die Fig.49 zeigt im Maßstab 2 : 1 rechts im Längsund links in einer Funktionsskizze in Vertikalansicht den Mechanismus für den Testbandtransport.
Die Fig.50 zeigt im Maßstab 2 : 1 einen Punktionsgriffel mit spezieller Ausstattung für die Rotationsbewegung einer Schneidspitze im Längsschnitt. Darunter ein Querschnitt etwa in Griffelmitte. Unter diesem Querschnitt eine Draufsicht auf eine Scheibe am Griffel zur Vermittlung der Sektordrehung (ohne die Darstellung von Stiften oder Dornen zur Mitnahme des Testfeldes, wie sie zweckdienlich sind und auf Fig.51 gezeigt werden). Ganz unten eine Scheibe im Längsschnitt nach Sektordrehung um etwa 30 Grad, um die Keilschräge zu demonstrieren, an welcher der Griffelstößel die Schneidebewegung ausführt.
Die Fig.51 gibt im Maßstab 2 : 1 Details über eine Ankoppelung eines Testfeldes an das Punktionsgriffelende oben in einem Vertikal- und unten in einem Längsschnitt (bezogen auf den Längsschnitt der Fig.50).
Die Fig.52 zeigt im Maßstab 2 : 1 oben im Längsschnitt nochmals eine Verriegelung eines Stiftes (476,vgl.Fig. 48) mit der Bodenwand des Glukometers.
Darunter eine Draufsicht auf ein Stück Teststreifen im Maßstab 2 : 1 .
Im Längsschnitt darunter eine Schneidspitze unter Federspannung, welche sich nach Membranzerstörung in die Punktionsstellung aufrichtet.
Ganz unten im Längsschnitt ein Stück Teststreifen im Maßstab 2 : 1 . Hierzu links im Längsschnitt zwei Keilspitzen für den gefederten Antrieb durch den Griffelstößel unter Längsverschiebung des Teststreifens. Rechts im Querschnitt ein Elektromagnet, welcher eine ruckartige Schnittbewegung quer zur Transportrichtung des Teststreifens ausführen kann.

### Ausführliche Beschreibung der Ausführungsbeispiele

Die Figur 1 gibt oben in einen Längs- und unten in einem Querschnitt in natürlicher Größe eine Vorrichtung zur visuellen Kontrolle der Punktionsstelle wieder.
Die weitere Aussstattung der Saugglocke(1) wurde weggelassen. Lediglich eine Träger-Leiste(63) zur Kennzeichnung der Lage einer Lichtquelle(17) ist eingezeichnet. Der Lichstrahl ist deutlich auf den zentralen Hautfleck unter der Saugglocke ausgerichtet (hier strichpunktierte Mittellinie). Die Saugglocke wird größerenteils von der Mantelhülse(137) mit breit offenem Spalt umgeben. Letztere ist im dargestellten Funktionsstadium bis zu einem Randstopp herabgezogen, so daß der beleuchtete zentrale Hautfleck durch den breiten Spalt hindurch eingesehen werden kann. Zur Benutzung wird bei Absenkung der Saugglocke die Mantelhülse auf letztere zurückgeschoben (gestrichelte Darstellung). Unten ist ein Querschnitt in der Überlappungsebene von Saugglocke und Mantelhülse zu erkennen.

Die Figur 2 zeigt im Längsschnitt die linke Hälfte einer Saugglocke mit dem Detail eines Elektromagneten mit dem Magnetkern(98), der aber auch einer mit Anker oder Stößel sein kann. Der Magnet wirkt auf den Schieber(109), der die Kuppe des Stiftes mit nachfolgender Randkerbe gegen die keilförmige Ausladung des Lanzettenschaftes(13) absenkt und dabei die kurze Kippbewegung der Lanzette bewirkt. Die Lanzette ist um die Achse(25) innerhalb der Saugglocke kippbar, deren größerer Durchmesser das Eindringen der Haut unter Sogeinfluß (von Seiten einer nicht dargestellten Sogquelle) erlaubt (strichpunktiert). Das Vordringen der Lanzettenspitze bewirkt, daß die Hautkuppe zentral napfartig eingesenkt ist. Blut kann sich in der entstandenen Senke sammeln. Die Stellschraube(57) erlaubt es, durch Änderung des Abstandes der auf die Keilausladung des Lanzettenschaftes wirkenden Stiftkuppe zum Keil die Schnittlänge zu verändern.

Die Figur 3 zeigt in Aufsicht einen Teststreifen(103), wie er insbesondere zur elektrischen Glukosemessung handelsüblich ist. Im Testfeld(104) sind die beiden Meßflächen hier durch einen Spalt getrennt durch die von oben die Spitze eines Lanzettenschaftes(13) auf die Haut hindurchreicht, wie aus dem Vertikalschnitt längs der Schnittlinie A - B zu ersehen ist. Zur Anwendung kann bei fixiertem Teststreifen die Lanzette innerhalb des Schlitzes nach dem Hauteinstich längsbewegt werden; es können aber auch Teststreifen und Lanzettenschaft durch wenigstens ein Loch(138) im Teststreifen, in welches je ein Querstift aus der Lanzette eingreifen oder anders etwa klemmend miteinander verbunden und gemeinsam horizontal zur Schnitterweiterung bewegt werden. Das über die Leitelemente hinter der Lanzettenspitze(hier weggelassen) hochsteigende Blut tritt direkt auf die Testfelder, wobei natürlich auch farbveränderte Chemikalien zu Meßzwecken einsetzbar sind.
In der Mitte unten wird eine Variante gezeigt, bei welcherdie Lanzettenspitze den Teststreifen seitlich überragt (unten in einer Aufsicht auf den Teststreifen).
Der Längsschnitt darüber zeigt, daß die beiden Meßoder Testfelder zu beiden Seiten dem spitzennahen ganz wesentlich verkürzten Lanzettenschaft mit seiner feinen mäanderartigen Faltung zur Blutableitung anliegen. Rechts ist die bevorzugte Variante in etwa natürlicher Größe links in einem Längschnitt durch die Lanzette dargestellt und zwar innerhalb einer gestrichelt dargestellten (futuralartigen) Verpackungshülle. Der Teststreifen endet hier an seiner Schmalseite in die Lanzettenspitze, was die Verpackung in der für Lanzetten üblichen Weise erlaubt und die Entnahme ohne Verletzung bzw.Abstumpfung der Lanzettenspitze erleichert. Bei einer elektrischen Meßmethode - etwa mittels Potentiometers haben die beiden Testfelder bei gemeinsamem Nulleiter (wir haben getrennte eingezeichnet) und damit je zwei Meßdrähte zum Meßgerät hin (die des unteren Feldes sind gestrichelt dargestellt). Im Querschnitt links wurde der Teststreifen in die Klemmfeder(28) am Schieber eines Punktionsgerätes eingeschoben und findet in einer Saugglocke so leichter Platz als bei Querlage.

Die Figur 4 zeigt ein Wegwerftöpfchen als Saugglocke, das oben und unten je von einer (gestrichelt gezeichneten) Schutzfolie verschlossen ist.
Ebenfalls gestrichelt wird die Möglichkeit demonstriert, nach Sogeinwirkung über die Öffnung(97) einen Faltenbalg mit Hakenstange(64) zur Erzeugung einer Lanzettenkippbewegung abzusenken. Eine leichte Wiederbelüftung über das Ventil(96) läßt den Faltenbalg mit der Hakenstange leicht ansteigen, wobei der Endhaken der Hakenstange in einen Profilabfall am Ende des Keilanstieges an der seitlichen Lanzettenschaftausladung gerät. Die Lanzette wird so aus dem Schnittkanal gezogen. Je nach Einstellung der Stellschraube(57) kann der Endhaken noch während seiner Passage längs der Keilausladung des Lanzettenschaftes von diesem weggelenkt werden. Es kann so die Schnittlänge beeinflußt werden. Der in die Saugglocke eingeschobene Bodenzylinder lenkt durch seitliche Spiegelkanten einen Lichtstrahl (strichpunktiert) für die Hautkontrolle über die kleine Hautblase im zentralen Loch des Bodenzylinders.
(Die schwache Verrasterung des Bodenzylinders etwa mittels Noppen an der Innenfläche der Saugglocke wurde nicht dargestellt). Stärkere Sogwirkung von Seiten der (nicht dargestellten) Sogpumpe läßt nach posivivem Ergebnis der Hautkontrolle (deren Meßanordnung hier ebenfalls weggelassen wurde) die Hautblase in die Lanzettenspitze mit dem Bodenzylinder aufsteigen.

Mit Figur 5 wird im Längsschnitt im Detail die Möglichkeit einer Lanzettenkippung durch einen Elektromagneten aufgezeigt. Über die Magnetspulen(99) kann der Magnetkern(98) angeregt werden, der das Magneteisen(100) in der Aufnahme(10) für die Lanzette anzieht und die Kippbewegung um die Achse(25) in der Trägerleiste zur Folge hat.

Die Figur 6 zeigt zwei Lanzetten in Aufsicht im Maßstab 4 : 1 .
Bei der oberen wurde das Material aus einem Fensterausbruch(37) für eine innere Randaufbiegung(36) auf dieselbe Seite benutzt, an der eine äußere Randaufbiegung (35) vorgenommen wurde. Wie der Querschnitt entlang der Schnittlinie A - B zeigt entsteht bei entsprechender Randumbiegung fast eine Röhre zur Blutableitung aus dem Schnittbereich unter der Haut.
Im Spitzenbereich der unteren Kanüle sind eingefaltete oder geformte Lamellen(101) zur Blutableitung zu erkennen.

Die Figur 7 ist ein Längsschnitt in natürlicher Größe (bei deutlicher Übertreibung des Saugglockendurchmessers) durch eine Punktionseinrichtung, welche das durch Punktion abgeleitete Blut direkt dem Testfeld eines Meßgerätes zuführt, das sogar während der Punktion schon angeschlossen sein könnte. Der sogerzeugende Faltenbalg (69) mit der Druckfeder(70) im Zylinderbecher(111) ist seitlich zur Saugglocke(1) angeordnet. Er wird in komprimiertem Zustand dargestellt. Der Zylinderbecher(111) ist mit dem Dach des Faltenbalges verbunden und zwischen den Backensegmenten(102) verschieblich, die an der Saugglokke befestigt sind. Als Dach der Saugglocke ist der Kolben(2) dahingehend umgestaltet, daß von ihm eine Vielzahl von Zungen(114) nach untenhin ausgehen. Diese sind in Bohrungen eines inneren Kantenvorsprunges des Saugglockenzylinders verschieblich, um ein Verkanten zu verhindern. Auch von unten von der Lochblende(80) aus verlaufen Stifte in Bohrungen jenes Kantenvorsprunges der Saugglocke, sektoriell zu den Kolbenzungen versetzt(Detail Mitte unten im Querschnitt). Mittels ringförmiger Dichtungen(112) ist der Saugglockenzylinder in einen oberen und einen unteren Teil geteilt. Unter dem Dach des Kolbens(2) ist ein Elektromagnet mit den Magnetspulen(99) montiert, dessen Anker eine Endplatte trägt, welche in die Gabel(107) hineinragt. Die Gabel(107) wiederum ist ein Teil des u-förmigen Schiebers(109), dessen unterer Schenkel plattenförmig verbreitert ist mit einer Schienenführung für das Einschieben des Endes eines Teststreifens(103). Deutlich wird dies am Detail als Querschnitt(unten) entlang der Schnittlinie A - B.
Bei Auflage des Kolbendaches, das mit dem Zentrum der elastischen Membran(4) verbunden ist, auf den Tisch kann entlang einer (nicht näher dargestellten) Schiene auf dem Gehäuse von Batterie(83) und Steuerzentrale(84) der Testreifen(103) aufgelegt und unter dabei das Testfeld(104) unter das Fenster der Schneide der Lanzette geschoben werden. Das untere Teil der Saugglocke wird dann mit dem Dichtring aufgesetzt und beide Teile werden mit zwei gegenüberliegenden Klammern(105) über die Randleisten(106) verbunden. Letztere liegen allerdings von der Dargestellten um 90 Grad winkelversetzt.
Mit dem oberen Schenkel des Schiebers ist ein Ende des Bowdenzuges(108) befestigt, während das andere Ende als Auslösestift(115), der über eine Lücke für den verbreiterten Stift(113) von der Lochblende und tritt dann in in eine Lücke einer Kolbenzunge(114, kleines Längsschnitt-Detail ganz unten Mitte).
Der Querschnitt durch die Saugpumpe unten rechts der Mitte zeigt den Zylinderbecher(111) zwischen den Backensegmenten(102), wobei die Bewegung unter Wirkung der Feder durch den Haken an der Handtaste(110, links unten Detail im Maßstab 6 : 1 im Horizontalschnitt verhindert wird. Sobald der Haken gegen die Druckfeder der Taste aus einer Lücke des Zylinderbechers gehoben wird dehnt sich der Faltenbalg aus. Die Luft in der Saugglocke wird über den Kanal(116) abgeleitet. Die Lochblende wird soweit durch Haut und Sog gehoben, bis der verbreiterte Stift der Lochblende gegen den Auslösestift (115,unterstes Längsschnitt-Detail Mitte) stößt. Nach erfolgter optischer Hautkontrolle (gemäß Fig.4) betätigt bei günstigem Ergebnis die Steuerzentrale(84) den Elektromagneten mit der Spule(99) und damit den Bowdenzug (108). Letzterer zieht den Auslösestift(115). (Lichtquelle und Sensor wurden hier nicht eingezeichnet).
Der Auslösestift verläßt seine Lücke in der Kolbenzunge (114) und über dem verbreiterten Stift von der Lochblende, so daß eine Anhebung der Lochblende und eine Absenkung des Kolbens(2) erfolgen kann. Die Klammer(117), die in ein Loch im Teststreifen eingreift, zieht dabei den Teststreifen ein Stück weit ins Innere der Saugglocke. Kurz nach Meldung des Kontaktschlusses zwischen Lanzette und Haut in der Steuerzentrale wird ein zweiter Magnethub ausgelöst, welcher durch plötzliche Seitverschiebung der schneidenden Teile der Spitze eine Gefäßverletzung zur Folge hat. (Die oberen Hautanteile werden von den stumpfen Teilen der Lanzette -wie den seitlichen Randaufbiegungen- (vgl.Fig.6) lediglich kurz verschoben. Der Magnet kehrt durch seine Rückfederung in die Ausgangsposition zurück und damit auch der Schieber. Die Wiederbelüftung der Saugglocke kann durch ein gesondertes (nicht dargestelltes) Ventil erfolgen oder durch Druck auf den Zylinderbecher(111) der Pumpe.

Die Figur 8 zeigt oben einen Längsschnitt durch das obere Teil einer Punktionseinrichtung ähnlich wie die zu Fig.7 beschriebene. Während dort der Schieber(119) unter dem am Rande unter der Dichtung festgeklemmten Teststreifen mit der Lanzette vorbeibewegt wurde, werden hier sowohl die Lanzette mit der abgebogenen Punktionsspitze als auch der Teststreifen(103) zusammen bewegt, was durch einen vorgefertigten Knick unter Verlängerung des Teststreifens ermöglicht wird. Für die Schubbewegung senkt sich die Keilführung der Hakenstange(64) mit ihrer Keilschräge durch ein Fenster im Schieber. Antrieb hierfür ist die elastische Deckelmembran(4) welche unter Sogeinfluß(dessen Quelle weggelassen wurde) mit dem Kolben(2) auf der Schiebhülse(8) abgesenkt wird. Durch Drehung in einem Gewinde im Kolbendach, kann die wirksame Länge der Keilfläche und damit die Schubstrecke vor Benutzung von unten her reguliert werden. Die Einrichtung kann auch ohne Saugglocke betrieben werden. Der Teststreifen liegt dann unmittelbar der Haut auf. Es können auch verkürzte Teststreifen in der Einrichtung mit Blut beschickt und -wie bei gewissen Glukometertypen vorgesehen- mit dem Testfeld voran in das Glukometer eingeschoben werden.

In Fig.9 ist im Maßstab von etwa 8 : 1 links das Detail einer Schneidspitze zu sehen, welche das Ende einer Kanüle darstellt, das sich auf die Schaft(13)-hinterseite, d.h. in das kurze Schaftende, öffnet. Strichpunktiert ist dort ein Blutstropfen eingezeichnet. Rechts ein Querschnitt an der Kanülenbasis, wobei die Längsseite der Bewegungsrichtung bei der Schnittbewegung entspricht.
Rechts daneben im Längsschnitt erweitert sich das Kanülenende(119) zu einem Trichter, welcher auf das Testfeld eines Teststreifens montiert ist, welches von gasdurchlässigen Pcren durchzogen ist. Rechts im Querschnitt soll die siebartige Durchlöcherung des Testfeldes (durch winzige Kreise) symbolisiert werden. Das Blut wird so direkt in das Testfeld hineingesogen.

Die Figur 10 gibt im Maßstab von 2 : 1 einen Längsschnitt durch eine Saugglocke als Punktionseinrichtung. Der Lanzettenschaft ist hier in einem senkrechten Schlitz auf dem Schieber(109) mit seiner Achse in eine Klemme eingeschoben, wobei die Achsnoppe hier nur der Höhenfixation dient. Das Magneteisen(100) ist am obern U-Schenkel des Schiebers(109) oberhalb eines Winkelstückes als Bewegungsbegrenzung der Lanzettenbewegung nach oben befestig. Das Winkelstück ist Bestandteil der Trägerleiste (63), die quer mit der Saugglockenwandung verbunden ist. Das Magneteisen kann vom Magnetkern(98) des Elektromagneten angezogen werden, um einen Schnitt durch die Schieberbewegung zu bewirken. Die zum Dach der Saugglocke gedichtete Stellschraube(57) wird über eine Exzenterscheibe gegen die Zugfeder(133) gegenüber dem Elektromagneten (auf nicht dargestelltem Schlitten) schiebend wirksam. Die Schubstrecke kann so reguliert werden.

Die Figur 11 gibt oben einen Längsschnitt und unten einen Querschnitt durch eine Punktionseinrichtung in natürlicher Größe bei konzentrischer Anordnung. Der Dichtungsring(5) ist als Lippendichtung ausgebildet, der Punktionsgriffel darin wurde weggelassen. Der Saugglockenrand ist topfartig verbreitert und von einem Ring mit Rohrsegment(156) innen umgeben. Eine ringförmige Aushöhlung nimmt die kräftige Druckfeder(70) auf, welche sich gegen das Dach des Zylinderbechers(111) abstützt. Letzterer ist sowohl unten gegen die breiteste Ausladung des Topfes als auch oben gegen das schmalere Rohrsegment(156) mittels der Ringdichtungen(157,158) gedichtet. Vom Zylinderbecher entspringen zwei symmetrisch angeordnete Segmentbrükken(159). Diese liegen bei durch manuellen Druck auf die Ausladung des Zylinderbechers gespannter Feder (70) nach Absenkung des Zylinderbechers entlang dem Rohrsegment letzterem oben an.
Auf dem Querschnitt unten, welcher der Schnittlinie A - B des Längsschnittes folgt, sind die höher überdachenden Segmentbrücken gestrichelt eingezeichnet.
Im Zentrum ist gestrichelt der hinten in der Wandung der Dachhülse verlaufende Segmentschieber(167) erkennbar. In seinen senkrechten Schlitz taucht der Sperrstift(168) ein, gehalten von dem Verbindungsring(169, s.auch im Querschnitt unten). Letzterer wird von den beiden Holmen der Segmentbrücken gehalten. Die in Spannung befindliche Torsionsfeder(11) liegt in einer den Boden fast erreichenden Rinne und umgibt die Saugglocke. Die Torsionsfeder ist einerseits mit dem Sockelteil des feststehenden Rohrsegments verbunden, andererseits mit der Dachhülse.
Oben befindet sich in Paßsitz mit dem Rohrsegment (bei gezeigtem seitlichem Durchlaß für die Segmentbrücken) der Trichterring(170), dessen Verschraubung zum Rohrsegment nicht dargestellt wurde. Vom Trichterring oben geht die Halteklammer(171) für das Glukometer aus.
Es wurde noch die Handtaste(110) eingezeichnet, die als Wippe ausgebildet ist. Das Achslager der Wippe ist auf dem Zylinderbecher befestigt. Der untere federnde Klammerarm umfaßt den äußersten Saugglockenrand. Am anderen Ende der Wippe zeigt ein zugespitzter Stift gegen eine Gummidichtung mit zentraler Bohrung im Zylinderbecher(160). Bei Druck auf den Stift der Handtaste wird gegen die Druckfeder(161) die Stiftspitze in die Gummidichtung gepreßt und dichtet deren Bohrung ab; kurz darnach verläßt das Klammerende den Saugglockenrand. Es kann jetzt die stärkere Druckfeder(70) wirksam werden und damit die Pumpenwirkung über den Sogkanal(162) innerhalb der Saugglocke. (Der Sogkanal und deshalb auch die Saugglokkenwandung sind etwas größer zu dimensionieren).
Die Hand geht nach dem Startdruck auf die Handtasten beidseits mit der Pumpenbewegung leicht nach oben, was das Einschlüpfen der Haut in die Saugglocke begünstigt (durch manuellen Drucknachlaß auf den Saugglokkenrand). Das Achslager kann auch auf dem äußeren Saugglockenrand befestigt und die Abdichtung des Sogkanals dort stattfinde, bei Festklammern des Zylinderbechers oben.
Die Dachhülse(143) ist um das Saugglockendach mit dem Dichtungsring(5) dort drehbar und paßt mit seinem inneren Sechskantprofil um die sechskantige Randausladung des (hier nicht dargestellten) Punktionsgriffels. Der engere untere Querschnitt des Punktionsgriffels ist kreisförmig und wird von der Lippe des Dichtungsringes(5) umschlossen.
Auf dem Querschnitt unten werden zwei vom äußersten Saugglockenrand ausgehende Laschen(163) gezeigt, die der visuellen Hautkontrolle dienen.
Im Längsschnitt rechts ist im Detail einer der beiden zugehörigen Markierungsstifte(164) herausgezeichnet, die gegen die Haut gedrückt werden können.
Nach Abheben der Punktionsreinrichtung von der Haut sind die Eindellungen der Haut durch die Markierungsstifte noch eine Weile sichtbar. Es kann die Beschaffenheit der Haut in der Mitte einer gedachten Verbindungslinie zwischen den Markierungspunkten geprüft werden, weil dort die Punktion stattfinden soll.
Zur Blutentnahme wird die Punktionseinrichtung nach Spannung der Druckfeder(70) erneut so auf die Haut aufgesetzt, daß die beiden Markierungsstifte genau über die Markierungsdellen zu liegen kommen.
Es kann auch Farbpigment (etwa Kreide) auf die Spitzen der Markierungsstifte aufgetragen werden, das sich dann auf der Haut abzeichnet.
Unten im Querschnitt sind innerhalb der Dachhülse(143) die oben offenen, symetrischen Keilsegmentschlitze (165) für die Anspannung der Torsionsfeder(11, oben im Längsschnitt) zu erkennen.
Vom Verbindungsring(170) führen von den Brückensegmentholmen um einen rechten Winkel gedreht die Sperrstifte(168) abwärts in die Schlitze der Segmentschieber(167). Letztere werden in ihrer Schiebebewegung durch die beiden Führungsstifte(172) in der hier verdünnten Dachhülse beschränkt bei entsprechenden Führungsschlitzen in den Segmentschiebern.

Die Figur 12 verdeutlicht im Längsschnitt durch jeweils die linke Hälfte der Punktionsreinrichtung nach Fig.ll mit schematischen Details rechts den Mechanismus der Punktionsgriffeldrehung in zwei Funktionsstadien.
Die rechtsseitigen Funktionsdetails stellen Aufrollungen einer Seitenansicht von innen (also von links) dar. In der oberen Abbildung, welche einem Stadium nach der Hautansaugung entspricht, sind die beiden Segmentbrückenholme(159), um die sich der Verbindungsring(169) schließt, am oberen Ende der Schrägen ihrer Keilsegmentschlitze(165) - hinter denselben- dargestellt. In die Keilsegmentschlitze ragen von den Enden der Holme der Segmentbrücken die Querstifte (166). Wie unten im Detail gezeigt, kann deren Abrollung an der Schrägen der Keilsegmentschlitze durch Rollen(173) begünstigt werden. Die beiden Sperrstifte(168) sind durch die Kraft der Torsionsfeder nach linksverschoben und deren Klemmfedern(174) haben die Schlitze der von ihnen nach oben gezogenen Segmentschieber(167) verlassen. Die Führungsstifte (172) im der Dachhülse(143) hindern eine weitere Anhebung der Segmentschieber. Im Längsschnitt ist unten eine Variante dieses Mechanismus durch Leistenüberlappung angegeben; darunter in einer Draufsich die Montage der Segmentschieber durch eine Schwalbenschwanzpassung deutlicher herausgezeichnet.
Links im Längsschnitt ist zu erkennen, daß die Handtaste(110) hochgezogen wurde; durch Nachlassen des Fingerdruckes ist die Bohrung in der Gummidichtung zur Wiederbelüftung der Saugglocke frei. Die Rotation der Dachhülse war im Uhrzeigersinne erfolgt.
Das Funktionsstadium unten entspricht der Absenkung des Zylinderbechers(111) mit den Segmentbrücken unter Anspannung der kräftigen Feder(70) für die Saugpumpe. Mit den Holmen der Segmentbrücken sind deren Querstifte(166) in den Keilsegmentschlitzen bis fast zum Ende von deren verengtem Vertikalschlitz abgesenkt. Bei der die Torsionsfeder spannenden Drehung im Gegen-Uhrzeigersinne dringen die Sperrstifte (168) in die Schlitze der Segmentschieber ein.
Sie haben diese gesenkt - soweit nicht durch Schwerkraft schon erfolgt - und sind fast bis an das Ende der Vertikalschlitze eingedrungen. Die Absenkung der Segmentbrücken erfolgt drehgesichert. (Die entsprechende Vorkehrung ist im Durchlaß für die Segmentbrücken im Trichterring(170) und durch den Druck der Feder(70) gegeben.

Die Figur 13 zeigt die Punktionseinrichtung der Fig.ll oben wieder in einem Längsschnitt im Funktionsstadium der entspannden Feder(70) nach erfolgter Rotation der Dachhülse(143) in natürlicher Größe. Der Punktionsgriffel(175) weist eine leicht exzentrische Spitze unten auf. Innerhalb des Dichtungssringes(5) um das Testfeld(104) hat der Punktionsgriffe kreisförmigen Durchschnitt und verbreitert sich nach oben unter Kalibersprung in einen Sechskant, um den sich die Dachhülse schließt.
Nach oben hin kann sich der Punktionsgriffel in den schmäleren Teststreifen(103) fortsetzen, dessen Ende in das Glukometer(176) eingeführt ist. Letzteres wird durch die vom Trichterring ausgehende Haltklammer (171) lagefixiert. Die Stellschraube(57) verläuft schräg oberhalb des Zylindersbecher(111) durch das Rohrsegment(156). In der Darstellung wurde die Stellschraube um 90 Winkelgrade gedreht, sie liegt hinten und führt mit ihrem konischen Ende in den Segmentschlitz(177) der Dachhülse, wie unten auf dem Querschnitt in Schnitthöhe A - B des Längsschnittes erkennbar ist. Die Stellschraube hat einen von unten innen in eine Schräggewindeführung im Rohrsegment eingeschraubten Teil, der in einen schmalen Vierkant endet. Auf letzteren ist von außen wie ein Schlüssel die Rändelschraube aufgesetzt, die eine Radiärskalierung zu einer Prcjektionslinie auf dem oberen Rand des Zylinderbechers aufweisen kann.
Das konische Ende erlaubt eine Regelung des Rotationswinkels für die Dachhülse auf Bruchteile eines Millimeters genau, wie er durch den Anstoß des Randes des Segmentschlitzes(177) gegeben ist.
Die Querschnitte durch das untere Ende des Punktionsgriffels zwischen Längs- und Querschnitt zeigen die verschiedene Exzentrität von Lanzettenspitzen.
Die Anordnung eines gegen die Schrägkante eines Keilsegmentschlitzes abgesenkten drehgesicherten Querstiftes dient zur Spannung der Feder für die Rotation der den Punktionsgriffel teilweise umgebenden Dachhülhülse. Dabei führt die Bewegungsumkehr bei Wiederanhebung der Dachhülse nicht sofort zur Rotation der Dachhülse unter Federwirkung, da eine Sperrstiftwirkung bei zugeordneten Schiebesegmenten dies verhindern.
Die Segmentschieber(167) können in ihrem Zusammenwirken mit den Sperrstiften(168) auch durch Hülsen ersetzt werden, welche diese Sperrstifte umgeben und um ein vorbestimmtes Maß von diesen Sperrstiften aus Bohrungen der Dachhülse herausgezogen werden (178,Fig.13 Detail Mitte rechts außen).

Die Figur 14 zeigt schematisch das Beispiel einer Punktionseinrichtung mit Saugglocke(1) für ein auf optischer Messung des Schwärzungsgrades eines Teststreifens beruhenden Glukometers(176). Zwischen je zwei Platten(208) die gegen die Torsionsfeder(219) um ein Scharnier schwenken, sind zwei Faltbeutel ausgebreitet und großflächig mit den Platten verklebt. Aus den Faltbeuteln führen die Sogschläuche (90) in die Saugglocke. Vor dem Aufsetzen der letzteren auf die Haut werden die oberen Platten gegen die Torsionfeder zusammengedrückt und zur Hautansaugung ihre Spreizung freigegeben unter Auflage der Hand auf das Glukometer. Von einer Lichtquelle werden die Strahlen schräg auf die Haut der künftigen Punktionsstelle gerichtet. Es ist ein zweites waagerechtes Strahlenbündel eingezeichnet, das bei Eignung der Haut und nach deren Ansaugung auf ein Signal hin, so wie nach kurzer Drehbewegung die Punktionsgriffels in der Dichtung des eingesenkten Saugglockendaches gegen das Testfeld(104) gerichtet wird. Deutlicher ist dies im Detail in der Mitte im Maßstab 2 : 1 im Längsschnitt zu erkennen. auf die Lanzettenspitze ist das elastische Schlauchende(220) zur besseren Blutsammlung aufgeschoben.
Die Saugglocke weist seitlich zwei gegenüberliegende kegelige Einstülpungen(221) der Wand zur Befestigung des Glukometers mit Hilfsgerät und zur Strahlenzentrierung auf das kleine Meßfeld auf. Das Glukometer befindet sich hierzu in einem Gehäuse mit ringförmig bis elliptischem Durchlaß für die Saugglocke, in deren Wandeinstülpung rechts ein Zapfen des Glukometergehäuses eingeschoben ist.
Die Verriegelung erfolgt von links durch den konischen gefederten Haltebolzen(222).
Der leicht vergrößerte schematische Querschnitt unten zeigt den Umschaltmechanismus von der optischen Hautkontrolle zur Stoffwechselmessung.
Ein durch gestrichelte Linie symbolisierter Lichtstrahl fällt von der Lichtquelle(17) auf das Prisma (224), das auf einem drehbaren Rundstift montiert ist. Je nach Winkelstellung des Prismas wird das Lichtbündel durch eine Linse zentriert auf die Haut oder das Testfeld geworfen. (Der Strahlengang nach der Linse ist stark verkürzt). Das reflektierte Licht wird von einer zweiten Linse gesammelt über das Prisma dem Photosensor(223) zur Messung zugeführt. Die Drehung des gegen Seitverschiebung gelagerten Prismas erfolgt mit der stoßenden Rechtsverschiebung des Magnetkerns(98) in der elektrisch erregten Magnetspule(99) über einen Haltestift(226) in der Spiralnut(225). Diese Magnetaktion erfolgt erst wenn die Hautkontrolle mit positivem Ergebnis unter Signalabgabe für die weitere Bedienung abgeschlossen ist. Durch die Verschiebung des Ventilstößels des Sperrventiles(197) mit dem Magnetkern. gegen seine Druckfeder kommt die Ringnut des Ventilstößels mit den Sogschlaucheinmündungen in Deckung, so daß die Haut in der Saugglocke angehoben wird. (Das Sperrventil wurde im Längsschnitt oben zur Vereinfachung weggelassen. Unten im Querschnitt wurden aus gleichen Gründen Batterie, elektronische Steuereinheit und Leitungsdrähte nicht eingezeichnet).

Die Figur 15 zeigt oben im Längsschnitt im Maßstab 2 : 1 eine Punktionsvorrichtung, welche ein Vakuumspeichertöpfchen nutzt. Aus dem industriell vorgefertigten Unterdruckspeicherraum(227) ragt der Auslösestift(228) durch den Saugglockenraum bis zu dessen Randbezirk. Im Zentrum befindet sich der Durchlaß für den runden zum Durchlaß gedichteten Punktionsgriffel mit leicht exzentrischer Schneidspitze. Die Schutzfolie(216) wird vor Anwendung auf der Haut abgezogen. Die Schutzkappe um das Ende des Teststreifens(103) wurde bereits entfernt und der Teststreifen in den Schacht des Glukometers(176) eingeführt. Letzterer wird dabei von den Halteklammern(171) auf der Drehscheibe(219) gehalten.
Zur Anwendung wird nach Abziehen der Schutzfolie die Saugglocke(1) auf die Haut gesetzt, welche sich durch den Andruck aufstaut und den Auslösestift (228) anhebt. Dessen gewachste Dichtung zum Saugglockendach in kleinem Ansatzstutzen wird aufgebrochen. Infolge der Stiftkonizität mit Verbreiterung nach oben kann die Luft aus dem Saugglockenraum in den Unterdruckspeicherraum entweichen, so daß die Haut in die Schneidspitze hochgehoben wird.
Es wird jetzt das Glukometer mit seiner Drehplatte gedreht, wobei ein von dort entsprinqender Randvorsprung(230) über eine Noppe(231) ruckartig bis zur nächsten Noppe hinwegsetzt. Durch Steigerung der Noppenhöhe kann bei einiger Übung im Hinblick auf den Krafteinsatz ein verschiedener Drehradius erreicht werden. Zur Wiederbelüftung wird die Saugglocke abgehebelt, wenn nicht ein Klebstreifen über einer Wandbohrung der Saugglocke gelöst werden kann (nicht dargestellt).
Unten in einem Längs- und darunter in einem Querschnitt im Maßstab 2 : 1 ene Hautkontrollscheibe mit Randring und zentralem Markierungsdorn. Gegen die Haut gedrückt bleibt ein erkennbarer Abdruck, in welchen die Saugglocke zum Gebrauch hineingestellt werden kann. Zweckmäßigerweise ist die Hautkontrollscheibe transparent, im Zentrum vielleicht mit vergrößernder Linsenwirkung, so daß die Punktionsstelle genauer beurteilt werden kann.

Die Figur 16 zeigt besonders in den Details oben stark schematisiert etwa in natürlicher Größe ein Lösungsbeispiel mit Rotation der Saugglocke bei stehendem Punktionsgriffel (in der Mitte im Längsschnitt). In der schematischen Draufsicht oben erkennt man die Grundplatte(232), auf welcher das Glukometer in der Halteklammer(171) abgestellt wird. Von dem Plattenpaar(207) das längs des Glukometers hinaufreicht sind nur Teile angedeutet und die Gegenplatten parallel an den Breitseiten der Basisplatte wurden ebenso weggelassen wie die beiden Faltbeutel - flächig mit den beiden Platten verbunden- zur Unterdruckerzeugung in der Saugglocke(1). Von zwei Ecken der Grundplatte reichen zwei Gelenksäulen(233,234) am Glukometer hinauf, um welche die Platten zur Sogerzeugung über die Zugfeder(235) nach außen abgespreizt werden, nachdem sie zuvor zusammengepreßt und in Wartestellung gegenseitig in Parallelstellung verklammert waren (nicht dargestellt). Unter der Grundplatte schwenkt die Flügelplatte(236) aus und zieht sich unter dem linken ausladenden Rand der Saugglocke(1, siehe Längsschnitt) vorbei.
Nach Ausdehnung der Faltbeutel kommt es durch die Flügelplatte zur Auslösung der Rotationsbewegung für die Schneidspitze.
Beim Längsschnitt (Blattmitte) wurde das Glukometer weggelassen und lediglich dessen Halteklammer(171) eingezeichnet und das hochragende Ende des Teststreifens(103) oberhalb des Punktionsgriffels mit Schneidspitze, der in einer Ringdichtung des Saugglockendaches(237) abgedichtet ist. Während die Grundplatte (232) am Glukometer festgehalten wird, ist das Saugglockendach drehbar. Es ist deshalb eine weitere Ringdichtung(238) in der Nähe des Randes der Saugglocke(1) erforderlich. Die Saugglocke selbst wird durch den Andruck gegen die (nicht dargestellte) Haut gegen Drehung gesichert. Die Rotation erfolgt durch die Torsionsfeder(11), deren eines Ende mit dem Saugglockendach verbunden ist, während das andere an der Dachhülse(143) - eigentlich ein Ring - festmacht. Die Rotation der Dachhülse wird durch den mittels einer Blattfeder in eine Keillücke unten in der DachHülse bepreßten Sperrstift(239) gehindert. Letzterer ist durch eine Bohrung im ausladenden Kragenring der Saugglocke(1) hindurchgeführt. (Die Funktion wird rechts unten in einer etwas höhengestreckten seitlichen Aufrollung erklärt). Der Sperrstift ist über die Keilführung(240) beidseits des Schlitzsegmentes(241, oben im Längsschnitt) erfolgt.
Linksseitig ist der Zustand der Entriegelung der Kraftübertragung von der Dachhü1se(143) auf das Saugglockendach(237) dargestellt. Dies geschieht unter Absenkung des Sperrstiftes(243) vom Keilvorsprung in einer Ringnut des Saugglockendaches. Der Rückzug des Sperrstiftes(243) erfolgt unter Wirkung der Blattfeder(244), sobald die Rotation der Dachhülse den in einer Bohrung dort befindlichen Sperrstift über die Keilmulde des am Querstift(245) verschieblichen und durch die Stellschraube(57) am Saugglockendach feststellbaren Sektorschiebers zu liegen kommt, der auch die weitere Rotation der Dachhülse verhindert. (Der Operationsraum der Sektorbewegung ist mit dem gestrichelten Ringsegment übertrieben weit dargestellt. Unten wird der Funktionsmechanismus wieder in seitlicher Aufrollung demonstriert mit der Keilführung(240) unter dem Sperrstift(239).) Zur Spannung der Rotationsfeder wird das Saugglockendach an seinem vorzugsweise geriffeltem Rand im Gegenuhrzeigersinne gedreht, wodurch auch der Sperrstift(243) und die Lücke für den Sperrstift(239) in Rastposition gebracht werden.
Ein ähnlicher Überholklinkenmechanismus kann auch für die Punktionseinrichtung nach Fig.11-13 eingebaut werden, wenn die Rückkehr der Schneidspitze des Punktionsgriffels in die Ausganglage durch die Wirkung der Hautelastizität erwünscht ist.
Hinsichtlich der Pumpenanordnung zur Unterdruckerzeugung wird auch auf die Fig.14(oben) verwiesen. Für die Drehbewegung der die feststehende Schneidspitze umgebenden Haut sind die radiär angeordneten Lamellen (auch Fig.28 unten im Querschnitt) von wesentlicher Bedeutung, zwischen denen die Haut segmentiert hochgesaugt wird. Hinsichtlich der Rotationsauslösung sei nachgetragen, daß erst nach Ausklappen der Flügelplatte unter Ausdehnung des Faltbeutes die Keilführung(240) unter den Sperrstift(239) gerät.

Die Figur 17 zeigt eine Punktionseinrichtung mit einem seitlichen Faltenbalg(46) als Pumpe. Es handelt sich oben um einen Längsschnitt in natürlicher Größe und unten um einen Querschnitt in Höhe der Rasterstangen(247).
Der Sogkanal(162) kann auch bei Verkürzung des Nippels zur Montage, Minderung des Totraumes im Faltenbalg und zur Sogleitung Richtung Saugglocke als Bohrung durch die breite Grundplatte(232) geführt werden. Der Topf(248) mit der Ringdichtung für den Punktionsgriffel vermindert den Totraum innerhalb der Saugglocke. Die Dachhülse(143) mit fester Überdachung ihres inneren und äußeren die Torsionsfeder(11) einschließenden Anteils, kann auf Kugeln gelagert sein, von denen nur eine dargestellt ist.
Es sind hier vier Halteklammern vorhanden, deren gebogenes Federende hinter einen Sperrrand am Glukometer(176) einrastet. Rechts vom Glukometer ist die Betätigungsstange(249) im Längsschnitt dargestellt mit einem oberen Querholm zur (nicht eingezeichneten) zweiten analogen Betätigungsstange. Die Betätigungsstange gleitet in einem Rohr, das unten zwei Schlitze (oder eine breite Schlitzung) für die Bewegung der in verschiedenen Höhen und Winkeln von der Betätigungsstange abstehenden Querstifte(251,252) aufweist.

Wie auf dem Detail der Seitenansicht zwischen Hauptzeichnung und Darstellung der Betätigungsstange zu erkennen, steht der Querstift(251) oberhalb der Randkerbe mit Keilführung(250) in der Dachhülse. Bei seiner Absenkung beschreibt letztere eine kleine Drehung im Uhrzeigersinn. Der Drehwinkel kann durch in Millimeter markierten Mulden verschiedener Länge auf der (nicht gezeigten) Hinterseite der Dachhülse festgelegt werden. Dabei ist für jede der Mulden eine Bohrung in einer auf der Grundplatte feststehenden Segmenthülse(253) vorgesehen. (Jede der Bohrungen ist durch einen Stift markiert, wobei aber nur einer eingesetzt wird).
Zwischen Längsschnitt und Querschnitt sind zwei Auslöserwippen eingezeichnet mit dem Ende der Betätigungsstange über dem abgerundeten und durch eine Druckfeder rückgefedertem Wippendende(254).
Die Wippe links entspricht in der Längendimensionierung den Verhältnissen unten im Querschnitt.
Der Wippenhaken(255) ist in eine Mulde der Raststange eingerastet, von denen zwei mit dem Deckel(256) des Faltenbalges verbunden sind, welcher von der Druckfeder(70) ausgedehnt ist. Dem rechts vom Längsschnitt demonstrierten Funktionsstadium würde die gehobene Wippe rechts entsprechen.
Die Torsionsfeder(11) ist schwach zu wählen, weil sie lediglich der Rückstellbewegung im Gegenuhrzeigersinn dient. Der Punktionsgriffel weist den Querstift (257) in einer Schlitzmulde der Dachhülse auf.
Seine Bewegung nach unten ist so gehemmt und seine Rotation gewährleistet. Um die Sektordrehung ruckartiger zu gestalten, wurde die Variante der Fig.22 für eine Auslöserwippe angegeben.
Der Querschnitt unten läßt erkennen, daß entsprechend der Anordnung der Bohrungen für die Einführung der Betätigungsstangen in die Grundplatte das Glukometer (276) schräggestellt ist. Die Schnittlinie A - B gibt die Schnittrichtung des obigen Details durch die Randkerbe mit Keilführung wieder. Auch die funktionelle Zuordnung der Querstifte(252,253) auf den Betätigungsstangen wird deutlicher.
Rechts noch eine Vertikalansicht auf ein Futural für das Punktionsgerät mit beigesteckten Funktionsstangen und (gestricheltem) Querholm hinter der Grundplatte(232). Vor Gebrauch muß die Feder(70) durch Druckanwendung bis zum Einrasten der Rastker- oder Wippenhaken(255) in den Kerben der Raststangen (256).

Die Figur 18 zeigt das Detail eines besonderen Schiebedeckelabschlusses mit besserer Einsicht von oben auf die Haut zur Kontrolle auf Eignung. Sie ergänzt die Thematik der Fig.35.
Oben ist die Aufsicht auf einen Teil des äußeren, oberen Deckelschiebers(257) und des inneren seitlichen Deckelschiebers(258, im Aufbruch) wiedergegeben. Die in späterer Position gezeichneten beiden Gummiplatten(259,260) sind gestrichelt wiedergegeben, der Gummidichtring(267) über dem Saugglockendach feinschraffiert. Im Zentrum der Testreifen(103).
Der Längsschnitt in der Mitte zeigt die dem Teststreifen anliegenden, d.h. zusammengeschobenen Gummiplatten(259,260), welche mit der Randeinfassung(261) aus Kunststoff oder Blech versehen sind. Solche Randeinfassung begünstigt das seitliche Vorbeibewegen des inneren Schiebers(258). Dieser steht entsprechend weiter als hier gezeigt zunächst unter dem äußeren Schieber (oben) weiter heraus und wird zuerst mit der Randleiste des letzteren(257) zur Deckung gebracht. Dabei drücken die seitlichen Keile(259), d.h. ihr sich keilförmig bis zu gleichbleibender Stärke verbreiterndes Profil die Gummiplatten um den Teststreifen zusammen. Der äußere Schieber(257) stützt sich mit oberen Keilen(263) paarweise gegen Randleisten(264) ab und preßt dabei die Gummiplatten aber auch den Gummidichtring(267) unter diesen in ihre Haltefals in der Grundplatte(232). Die Vorrichtung für die seitliche Kippbewegung der Lanzettenspitze wurde hier weggelassen.
Es wird noch eine Vorrichtung zur Höhenregulierung des Saugglockenrandes angegeben, welche die Einstichtiefe beeinflußt. In der mittleren Ebene im Längsschnitt liegt der Dichtungsring(265) zwischen dem eingeschraubten Zylinder der Saugglocke(1) und der Grundplatte ziemlich tief. Nach Freiraum für die Verschiebung folgt ein Kalibersprung nach innen und dann das Schraubgewinde.
Im Vertikalschnitt des Deckelbereichs wird eine der Randleisten(264) geschnitten und darunter ein Teil des äußeren Schiebers mit den hintereinander angeordneten beiden Keilen(263).
Die verstellbare Saugglockenhülse ist eine doppelwandig zum unteren Rand geschlossene(266) und schiebt sich mit dem inneren Zylinder am Dichtungsring(265) in der Saugglocke vorbei. Der äußere Wandzylinder, der mit der inneren um den inneren Zylinder der Saugglocke(1) im Schnittbild eine uförmige Brücke bildet, ist mit der Außenseite des Saugglockenzylinders verschraubt (rechte Bildhälfte). Auf der linken Bildhälfte ist eine Variante sichtbar, bei welcher der Dichtungsring(265) nicht innen in der Saugglocke(1) montiert ist, sondern außen oberhalb des Gewindes zur Verschraubung mit der Saugglockenhülse(266), die auch einwandig sein kann, und deren Rand vorzugweise nach innen einen vorspringenden Kragen aufweist, der etwa mit dem inneren Rand der Saugglocke(1) in Projektion und durch die Verschraubung regelbarem Abstand, diese schildartig überdachend, abschließt. Es kann zusätzlich auch eine Rastfeder mit oder ohne Randleistenriffelung oder Noppenbildung vorhanden sein, um die Höhenverstellung zu fixieren und eventuell in Stufen - etwa mittels gefedertem Raster - fühlbar und mittels Graduierung sichtbar zu machen. Da der Teststreifen bei der eben vorgestellten Lösung erst nachträglich von oben, zweckmäßigerweise schon in Verbindung mit dem Glukometer, in den Spalt zwischen den Gummiplatten eingeführt werden kann, wäre auch hier die lichtmessende Hautkontrolle vorzuziehen.
Von der Lichtquelle(104) wird hierzu der Lichtstrahl auf die Hautstelle im Zentrum der Saugglocke gerichtet und das zurückgeworfene Licht im Photosensor (223) empfangen und dem Photometer und der Steuerzentrale für die Aussendung eines Warnsignals zugeleitet. Letztere ist nur durch ein (zu kleines) Rechteck symbolisiert mit gestrichelten Linien für die Leitungsverbindungen.

Die Figur 19 gibt in natürlicher Größe ein weiteres Beispiel einer Punktionseinrichtung wieder, unten im Querschnitt durch die Mitte des Pumpenfaltenbalges, oben im Längsschnitt mit Vertikalschnitt-Details oben links und rechts. Statt durch eine Torsionsfeder wird die Punktionsgriffeldrehung durch die Druckfeder(268) bewirkt, welche mit der Druckfeder (70) zur Ausdehnung des Faltenbalges(46) in einer Achse auf der Gegenseite der Saugglocke der Grundplatte anliegt. Bei nach rechts gedrückten Raststangen(256) unter Verkleinerung des Faltenbalges tritt in je eine Rastkerbe dieser Stangen eine Rasttaste(271), die quer und senkrecht zur Raststange in einer Bohrung der Grundplatte(232) oder des Basisblockes verläuft(siehe Querschnitt-Detail durch den Rasterbereich). Die Rasttaste wird dabei durch die Druckfeder(272) zwischen Endplatte(277) und Grundplatte(232) angehoben. Die Schiebestangen(275), deren Enden über eine Querplatte verbunden sind und die Druckfeder(268) umfassen, werden nach Einwärtsverschiebung sowohl der Raststangen als auch der Schiebestangen mittels des Rastbolzens(276) verriegelt. Letzterer wird dabei nämlich aus seiner Rastmulde in der Raststange verdrängt, nachdem ihm die Rastmulde der Schiebestange genähert wurde. Dies ist Im Längssschnitt-Detail längs der Schnittlinie A - B unten im Querschnitt über diesem liegend deutlich. (Es ist das Stadium vor der Verrasterung dargestellt, das Verrasterungsstadium wird unter C in Fig.20 dargestellt). Bei Fingerdruck auf eine der beiden Raststasten(271) oder deren in Höhe der Dachhülse bogig verlaufenden Endplatte(277) lassen die Lücken (274) in den Rasttasten die Raststangen vorbei, so daß sich der Faltenbalg(46) zur Unterdruckerzeugung unter Wirkung der Feder(70) ausdehnen kann. Noch vor dem Endstadium dieser Pumpbewegung gibt die Rastmulde in den Raststangen(256) die Verdrängung des Rastbolzens(276) aus den Rastmulden der Schiebestangen frei (Stadium C der Fig.38). Unter Wirkung der Druckfeder(268) gleitet dabei der Blattfederbogen(278) nach rechts mitgenommen, wie in den Segmentdetails unter dem Gerätelängsschnitt im Querschnitt in Höhe des unteren Teiles der Dachhülse gezeigt wird und in der seitlichen Aufrollung rechts daneben. Nach Passage der Steilflanke der Keilnase an der Schiebestange über den Blattfederbogen wird durch letztere Druck auf die Keilschräge der Keilnase ausgeübt und diese nach links zurückbewegt. Voraussetzung dafür ist die Feststellung der Schiebestangenbewegung (es wird nur die eine für den Rotationsmechanismus in Anspruch genommen) durch den Anschlag mit der Stellschraube(57) für die Regulierung des Rotationswinkels der Dachhülse. (Der Blattfederbogen ist in seiner Funktion zur Rückführung der Schneidspitze im Detail rechts gestrichelt eingezeichnet). Der Anschlag mit Stellschraube(57) im Vertikalschnitt rechts oben wird gezeigt; wie im Detail in der Mitte (sektorverschoben) sichtbar ist, richtet sich die an der Stangenführung der Grundplatte befestigte Stellschraube gegen einen Anschlag an der Dachhülse. Die Schiebestangen sind breiter als die Raststangen, so daß der Blattfederbogen von unten in den Sektorschlitz der Dachhülse hineinragt, um dort auf deren Keilnase zu treffen (rechter Vertikalschnitt).
Während auf dem rechten Vertikalschnitt die Halteklammer(171) direkt der Dachhülse aufsitzt und das Glukometer bei der Sektorbewegung mitdreht, ist auf dem linken Vertikalschnitt eine die Klammer tragende Platte drehbar mit der Dachhülse verbunden, so daß die Drehbewegung vom Teststreifen bei festgehaltenem Glukometer mitvollzogen wird. Eine in diesem Falle zweckmäßige Brücke zwischen Grundplatte und der Platte mit der Halteklammer wurde nicht eingezeichnet.
Rechts vom Längsschnitt wird das Detail eines Klebestreifens(280) gezeigt, welcher rings um das Glukometer aufgeklebt werden kann, damit die Haltklammern hinter seiner Keilstufe einrasten können.

Das Detail einer ovalären Hülse unter dem Faltenbalg entspricht einem Querschnitt durch das Längsrohr des Nippels mit Teller um den Sogkanal(162) innerhalb des Faltenbalges. Die ovaläre Form erleichtert bei der Montage das Eindrehen des Nippelgewindes in die Grundplatte unter Befestigung des Faltenbalges.
Das Ende des Teststreifens(103) ist in den Schacht des Glukometers(176) eingeschoben. Die beiden Blattfedern(281) fixieren ihn dort in seiner Taille(131) gegen Herausziehen der Kontakte aus dem Schacht.
Die Ausladung des Gußteiles des Punktionsgriffels unten liegt dem mit der Saugglocke(1) festverbundenen Dacheinsatz(282) auf, welcher den Dichtungsring(265) trägt für die Aufnahme des schmaleren runden Anteiles des Gußteiles oder Gußstückes(283). Dessen Ausladung greift mit seinem Sechskant in den Sechskant der Dachhülse.

Die Figur 20 bezeichnet im Längsschnitt den Rasterfunktionsmechanismus zwischen der hinteren Raststange (256) und der zugeordneten Schiebestange(275) und dem Rastbolzen(276) in den Funktionsstadien A - C. Die Funktionsstadien wurden zu Fig.19 beschrieben. Die strichpunktierten Senkrechten bezeichnen die Endpunkte der Stangenbewegungen, die Pfeile die Verchiebungswege. Die Rastmuldenlänge sind der Funktion anzupassen.

Die Figur 21 bringt verschiedene Punktionsgriffelvarianten und eine Verpackungsart oben im Längs- und unten im Querschnitt in leichter Vergrößerung.
Diese Größenangaben beziehen sich auf die in den Punktionseinrichtungen dargestellten Verhältnisse: in Wirklichkeit wird man aus Kostengründen zu einer Verkleinerung auf Testfeldbreite fortschreiten, ja vielleicht auch die Testfelder noch verkleinern.

Auch die Wandstärke der Plastikwanne(284) für die Einlage der Punktionsgriffel in Mulden und der klebende und abziehbaren Schutzfolie(216) für deren Abdeckung ist übertrieben gezeichnet.
Der Querschnitt durch einen Punktionsgriffel dicht oberhalb der Schneidspitze(285) läßt von oben den Trog mit Testfeld des Teststreifens(103) offen bei nach unten geöffnetem Schacht(286) für den Bluteintritt. (Unter dem Längsschnitt noch ein Querschnitt durch den Sechskant des Gußstücks(283) längs der Schnittlinie A - B. Der Längsschnitt rechts zeigt die Breitseite des Teststreifens(103) und dessen oberes Ende mit den drei Kontaktdrähten für die Einführung in das Glukometer.

Die Figur 22 zeigt eine Variante des Wippenschalters der Fig.17 in der Seitenansicht. Der Querstift (252) der Betätigungsstange liegt dabei auf der Rolle(287) auf, deren Achse durch das Ende der Stütze(288) geht. Die Stütze wird durch eine Winkelverstärkung, an die sie anlehnt, daran gehindert, nach rechts auszuweichen. Das labile Gleichgewicht gegenüber dem Druck von oben wird schließlich durch Linksausweichung der Rolle überwunden. Dabei betätigt der Querstift das Wippenende(254) gegen die Druckfeder der Wippe. Die anschließende Betätigung des Torsionsmechanismus für die Schneidspitze wird dadurch ebenfalls plötzlicher gestaltet.

Die Figur 23 zeigt in der Mitte oben einen Querscnitt durch die wannenartige Umrandung(179) für die Aufnahme des Glukometers(176), wie darunter im Längsschnitt dargestellt. Der Querschnitt folgt der Schnittlinie C - D des Längsschnittes. Das Glukometer wird durch das Zugfederband(191) nach unten gespreßt. Der mit seinem Ende in den Glukometerschacht eingeführte Punktionsgriffel wird dabei mit seinem runden Ende, das durch eine Bodenöffnung in der Umrandungswanne ragt, in die entsprechende Rundung der Einwölbung des Saugglockendaches gedrückt. Die Schneidspitze ragt dort durch einen Schlitz in der im Schlitzbereich schienenartigen Bereich des Bogens der Dacheinsenkung, wo sich die Abdichtung der Saugglocke vollzieht.
Die schienenartige Gestaltung in der gebogenen Dacheinsenkung mit Schlitz für die Schneidspitze ist unten auf dem Querschnitt sichtbar.
Es werden noch radiäre Lamellen(185) gezeigt, die vom Saugglockendach zur Fixierung der die Schnittstelle umgebenden Haut herabragen. Die strichpunktiert dargestellte Haut ist in die Saugglocke hochgezogen, die Druckfeder(70) des Faltenbalges der Saugpumpe entsprechend in einer Entspannungsstellung. Die Kippbewegung für die Schnittführung der Schneidspitze liegt im Mittelpunkt der Rundung der Dacheinsenkung der Saugglocke und wird von der Umrandung des Glukometers mitvollzogen. Zur Führungsunterstützung weist die Umrandung Achsnoppen(182) auf, welche sich innerhalb der gebogenen Schlitze(188) bewegen können.
Die gebogenen Schlitze befinden sich in Wandplatten(290), die auf der Grundplatte(232) feststehen. Die Kippbewegung wird über eine Art Exzenterstange(291) durchgeführt, welche in einer Schrägbohrung der Umrandung des Glukometers sich in einer Zugfeder fortsetzt, welche in dem Stift(292) endet. Dieser Stift wird je nach gewünschtem Kippwinkel in eines der Löcher im Zahnrad(293) eingesteckt, welche verschiedenen Abstand von der Zahnradachse haben. Der Antrieb des Zahnrades erfolgt über die betreffende Schiebestange(275), die nach oben als Zahnstange ausgebildet ist. Die Verbindung mit der Schiebestange über die (hier nicht dargestellte) Druckfeder entspricht der Anordnung in Fig.19 (dort rechtsseitig gelegen). Links in der Mitte ist in einem Vertikalschnitt-Detail dargestellt, wie die Achse(294) der in dieser Variante beidseits von den Schiebestangen angetriebenen Zahnräder(293) bei festem Achsensitz ein Rad mit den Löchern für den Stift(292) antreibt. Die Achse weicht in u-förmigem Bogen der Stiftbewegung aus. Bei der Drehung des Zahnrades im Uhrzeigersinn wird zunächst die Zugfeder angespannt. Gegenüber dem Exzenterstift, diesen herausziehend, wird die Federspannung erst wirksam, wenn der Stift annähernd in die Verlängerunglinie zum Exzenterstift zu liegen kommt. Dies begünstigt eine ruckartige Kippbewegung. Das Ausmaß der Kippbewegung kann an der Stellschraube(57) feinreguliert werden. Die Rückführung der Umrandung mit dem Glukometer erfolgt über die Zugfeder(296). Oben wird beidseits im Maßstab 1,5 : 1 ein Punktionsgriffel gezeigt, der mit für eine visuelle Überwachung der Punktionsstelle ausgerüstet ist. Die Lichtquelle(17) befindet sich im Schacht des Glukometers. Der Lichtstrahl fällt im Schacht(297, rechtsseitiger Vertikalschnitt) am Testfeld(104) vorbei auf die Haut nahe der Schneidspitze

Durch den Schacht auf der anderen Seite des Testfeldes wird das von der Haut reflektierte Licht vom Spiegel oder Prisma(224) rechtwinkelig zum Auge des Beobachters abgebogen. Dies geschieht durch einen (nicht eingezeichneten Schlitz) in der Wandplatte(290) im Zwischenraum zwischen Glukometerschacht und Grundplatte(232) des Punktionsgerätes. Wie im Lösungsbeispiel der Fig.28 ist die Saugglocke mittels zweier oder dreier Rohrstutzen (186), von denen nur einer eingezeichnet ist, mit der Grundplatte abnehmbar verbunden.

Die Figur 24 schildert oben in einem Längs- und unten in einem Querschnitt in natürlicher Größe ein Lösungsbeispiel für eine Schwinglanzette. Als Variante werden Antriebsfedern sowohl zur Unterdruckerzeugung als zur Erzeugung der Schwingbewegung vermieden.
Die Saugglocke(1) steht mit ihrem Rand etwas oberhalb der linealartigen Unterkante des Topfes(298) für den Faltenbalg(46) und der Grundplatte(232).
Da die menschliche Körperoberfläche überall Wölbungen aufweist und zudem über dem Punktionsgebiet elastisch zurückfedert, kann durch entsprechende Anlage des "Lineals" ein unbeabsichtigtes Wegkippen der Saugglocke mit vorzeitiger Wiederbelüftung so besser verhindert werden.
Die Umrandung(179) für die klemmende Einführung des Glukometers(176) weist zu einer Haltegabel der Grundplatte beiderseits die Achsnoppen(182) auf.
Im Längschnitt ist die Umrandung um die Achsnoppen gegen die Spannung der Blattfeder(300) leicht hochgeklappt. An der Schraube(301) durch eine verlängerte Basisplatte der Umrandung kann das Ausmaß möglichen Hochklappens begrenzt werden. Um den Teststreifen(103), der in den Schacht des Glukometers eingeführt ist, erkennt man den Topf(248) zur Totraumverringerung innerhalb der Saugglocke.
Der Gummidichtring(267) wird nach Herunterklappen der Umrandung mit dem Glukometer auf die Grundplatte wirksam. Der Unterdruck wird dadurch erzeugt, daß der Faltenbalg(46) mittels der (unterbrochen und verkürzt dargestellten) Betätigungsstange in den Topf(298) hinein entfaltet wird. Der dabei im Faltenbalg entstehende Unterdruck wird über den Schlauch(90) in der Saugglocke wirksam. Der Faltenbalg wird durch den Dichtungsring um die Betätigungsstange abgedichtet. Für letztere ist innerhalb einer Gabel oder hier des Zylinders(303) ein verkürzt dargestellts Spiel vorgesehen, wobei in unserer Darstellung die Betätigungsstange in der Form eines Tellers innerhalb des Zylinders endet. Die Lage im Zylinderende behält der Teller auch bei Druckausübung auf den Zylinder in Richtung Faltenbalg bei. Aber nur solange, bis die sperrende Wirkung der Sperrbolzen(195) im Zylinder in einer Ringnut der Betätigungsstange überwunden wird. (Eigentlich ist das sperrende Glied hier eine Kugel, hinter welcher eine Druckfeder liegt, deren Druck gegen die Ringnut an der Stellschraube verändert werden kann). Die längs des Topfes für den Faltenbalg in einer Rinnenverschalung geführte kantige Schiebestange(275) ist unten im Längsschnitt dargestellt. Ihr Ende ist durch einen Stift mit dem Zylinderrand verbunden. Die Bewegung der Schiebestange erfährt nach Lösung des Sperrasters eine ruckartige Beschleunigung, wobei die schräggestellt Blattfeder(304) den Querstift(305) auf dem Stößel (306) verdrängt. Der Stößel ist durch eine seitliche Bohrung des Saugglockenzylinders in letztere eingeführt und zur Außenluft in der Saugglockenwand gedichtet. Sein freies Ende trift auf den Teststreifen(103) nahe an dessen Ende und erteilt im einen seitlichen Bewegungsimpuls. Die Länge der Verschiebewegung des Teststreifens wird durch die Strecke festgelegt, welche der zur Saugglockenwand ebenfalls mit einer Dichtung versehene Gegenstößel im Saugglockeninneren aufweist. Diese Länge ist mittels der Stellschraube(57) in der Gewindebohrung einer der Saugglocke aufgesetzen Tülle regelbar. Ist die Innenbewegung des Stößels gesperrt, so weicht die Blattfeder(304) dem Ende des Querstiftes aus und passiert diesen. Die in Gegenschräge ausgerichtete zweite Blattfeder holt vor ihrer Passage am Querstift den Stößel wieder nach außen zurück.
Damit nach Überwindung des Sperrrasters im Zylinder (303) des Handtellers für die Pumpenbetätigung der Faltenbalg sich nicht zurückziehen kann, wird die Rückwärtsbewegung der Betätigungsstange(249) durch die Sperrnut(307) mittels eines gefederten Bolzens(308) verhindert. Die Positionierung dieses Sperrasters am Ende des Topfes für den Faltenbalg ist durch den strichpunktierten Pfeil bezeichnet. Entsprechend befindet sich das Trapezlager (309) für die Achse des Wippenbalkens(310) auf dem Topf(298) über dem Faltenbalg. Zur Wiederbelüftung der Saugglocke nach Signalton vom Glukometer her, der die Blutsättigung des Meßfeldes bezeichnet, hebt Druck auf das freie Ende des Wippenbalkens den Bolzen(308) und löst die Sperre an der Betätigungsstange. Vor erneuter Benutzung muß nach Auswechseln des Teststreifens unter vorübergehender Aufklappung über der Saugglocke der Handteller am Ende der Betätigungsstange unter Verkleinerung des Faltenbalges zurückgezogen werden, um die dargestellte Ausgangsphase wieder herzustellen.
Die breite Einkerbung zwischen rechtem Saugglockenrand und der Grundplatte(262) erlaubt zum Umfassen der Einrichtung dort den Daumen einzuschieben. Für kleinere Hände dient eine Daumenschlaufe vorn und weiter oben demselben Zweck.

Die Figur 25 zeigt drei Varianten des Verschlusses des Saugglockendaches insbesondere bei Verwendung von Kipp- oder Schwinglanzetten oder Schneidspitzen. Die linke Variante zeigt oben im Längsschnitt in natürlicher Größe, unten im Querschnitt in Höhe des Gummisichtringes(267) einen Abdichtungsmechanismus ähnlich dem in Fig.18 beschriebenen.

Die Gummiplatten(260) sind hier dünner. Gegen ihren inneren Randwinkel lehnt je eine Blattfeder(311). Deren Keilausladung lehnt an eine Abschrägung des oberen inneren Zylinders der Saugglocke(1).
Vor Gebrauch wird der Topf vor oder nach Einführung des Teststreifenendes in den Schacht des Glukometers(176) mit seiner den Gummidichtring(267) tragenden Oberfläche mit dem Glukometer in Haftung gebracht. (Beispielsweise durch nicht dargestellte Permanentmagnete). Bei der Absenkung des Topfes in das Innere der Saugglocke bis hin zu zwei oder drei von der Saugglocke ins Innere ragenden Anschlagstiften werden die Keilausladungen der Blattfedern nach innen gedrängt. Sie teilen diese Bewegung den Gummiplatten mit, deren Ränder über dem Teststreifen hermetisch geschlossen werden. Die Dichtung nach außen zwischen Glukometer und obererm Saugglockenzylinder besorgt zuletzt der zusammengepreßt Gummidichtring, welcher ringum in dünner elastischer Membranverbindung mit den Gummiplatten steht.
Die Variante der Lösung in der Mitte - oben wieder im Längsschnitt und unten im Querschnitt in Höhe des Deckelrings(312) - besteht eben aus letzterem, welcher rings um den Schacht des Glukometers dicht aufgeklebt wird. Mittels seitlicher Klammerfedern (3139 von der Außenwand des Saugglockenzylinders aus wird nach Teststreifenbestückung der Deckel ring gegen den Gummidichtring(267) auf dem Sauglockenzylinder gepreßt.
In der Variante rechts (im Längsschnitt) weist der Teststreifen für die Anlage auf dem Gummidichtring des Saugglockenzylinders eine rundliche Profilverbreiterung(315) für den Andruck auf diesen mittels Federn auf.
Für die visuelle Hautkontrolle der Punktionsstelle ist im mittleren oberen Beispiel eine Lichtquelle (17) so in die Saugglockenwandung eingebaut (die Lampenfassung und ihre Dichtung in der Saugglockenwandung wurden weggelassen). Es führen Leitungsdrähte über die Batterie(83) zum Stromkreisschluß über den Schalter mit der Handtaste(110), die jedoch neben dem Glukomter ähnlich wie ein Fotoauslöser auf der Grundplatte montiert ist. Vor Deckelschluß kann die Handtaste gedrückt und die Hautkontrolle visuell vorgenommen werden.

Die Figur 26 zeigt ein Lösungsbeispiel für den Rückzug der Schneidspitze nach dem Schnitt aus dem Wundbereich, um das Blut besser austreten zu lassen. Die Lösung bezieht sich auf das Verfahren der Rotationslanzette. Oben ist eine Aufsicht wiedergegeben, darunter ein Längsschnitt und ganz unten ein Punktionsgriffe-Detail im Maßstab 2 : 1.
Der Hebel(316) ist fest mit der Dachhülse(143) verbunden, welche mit einem Polygonal (etwa Sechskant) oberhalb des Dichtungsringes(265) mitnehmend den Punktionsgriffel(175) umgibt. Der Punktionsgriffel (Detail unten) ist hier beispielsweise in seinem unteren runden Abschnitt verschmälert und von der Randhülse(317) im Schiebesitz umgeben.
Die Oberkante der Randhülse verläuft in einer spiralförmigen Schräge, in deren niedrigsten Bereich die Blattfeder(318) vom Saugglockendach aus eingreift. Lediglich mit einem Segment(320) greift der Randring(320) der Dachhülse durch und wird in einem dem Segment in Drehrichtung sich anschliessenden Schlitz durch Stellschraube(57) in seiner Drehung blockiert. Das Gewinde der Stellschraube liegt in einer Bohrung des auf dem Saugglockendach drehbaren Ringes(321). Die Schraubenfeststellung erfolgt durch die Klemmwirkung vom Schraubenende gegenüber der ringförmigen Dachbegrenzung der Saugglocke. Der federnde Hebel(316) liegt über einer segmentalen Kulissenführung (Detail unter dem Hebel in Seitenansicht), deren steiler Kurvenanstieg die Rotationsdrehung durch die Hand ruckartig gestaltet. Die Spiralführung der Randhülse ist so gewählt, daß in der Endphase der Drehung die Randhülse nach unten geschoben wird, so daß sie die Haut von der Schneidspitze abdrängt. Innerhalb der Randhülse kann das Blut hochsteigen und wird vom (nicht dargestellten) Testfeld aufgesaugt. Die Einführung des Punktionsgriffels muß gemäß einer Markierung am oberen inneren Rand der Dachhülse winkelgerecht erfolgen.

Die Figur 27 gibt in einem Querschnitt-Detail einen analogen Mechanismus zur Randhülsenabsenkung bei einer Schwinglanzette. Zugleich wird demonstriert wie die Schwingbewegung und deren Rückstellung in Gegenschwingung von beiden Seiten der Saugglocke durch zwei Schiebestangen erfolgen kann.
Das Ausmaß der Einwärtsbewegung des Stößels(306) über die Schiebebewegung der Blattfeder(304) auf der Schiebestange unter dem Stößel wird hier durch die Bewegungsfreiheit der drehgesicherten Schraubhülse für die Stellschraube(57) in ihrer Tülle bestimmt. Der Gußteil mit dem Testfeld wurde hier beispielsweise rund gewählt. Unten zeigt der Längsschnitt durch die Schiebestange die Lücke in derselben, welche das elastische Ausweichen der Blattfeder(304) vor dem Querstift(305) des Stößels erlaubt. Nach der Blattfeder(304) trifft die Blattfeder auf der Schieberstange der Gegenseite der Saugglocke auf den Querstift des Gegenstößels. Dessen Ende drängt das Gußteil des Teststreifens wieder zurück. Zuvor aber drängt seine Federgabel (322) die Randhülse mit ihren Keilschultern über die Schneidspitze nach unten. (Links und rechts der Saugglocke im Vertikalschnittdetail vor und nach der Abdrängung der Randhülse.)

Die Figur 28 zeigt oben im Längsschnitt und unten im Querschnitt in Höhe des Oberrandes des Gehäuseblockes oder der Grundplatte(232) eine Punktionseinrichtung, welche für einen Rotationsgriffel ausgelegt ist in natürlicher Größe.
Die Saugglocke(1) kann auch Teil der Grundplatte (232) sein, deren Unterrand den Saugglockenrand leicht zur Stabilisierung der Anlage auf der gewölbten Körperoberfläche des Anwenders nach unten überragt. Es sind zwei Faltenbälge(46) mit ihren Druckfedern(70) als Pumpenvorgesehen. Vom Faltenbalgdach führt der dort kreisförmige Deckel(147) mit einer Querleiste zur Raststange(256). Die Betätigungsstange(249) weist den Querstift(251) auf, welcher in eine Keilschulter in einer Kerbe (250, rechtes mittleres Detail in Fig.17) eingreift oder wie hier dargestellt in eine Keilführung auf einer segmentalen Kulissenführung wie seitlichen Detail der Fig.26 ohne die Steilflanke, beide Vorkehrungen innerhalb der drehbaren Dachhülse(143). Eine weitere Variante ist in Fig.30 beschrieben. Zwischen dem an der Saugglocke befestigten Topf(248) mit dem Dichtungsring(265) für den Punktionsgriffel(175) ist das Kugellager(323) vorgesehen. Der manuelle Druck vom Glukometer(176) her wird vom Randring(319) abgefangen, der mit der Grundplatte verbunden ist und den Gummidichtring (267) zum um den Glukometerschacht verklebten Dekkelring(312) hin trägt. Der runde Gußteil des Punktionsgriffel setzt sich bis in den Glukometerschacht in den flachen Teststreifen(103) fort. Das Glukometer befindet sich zwischen der Haltklammer(171). Der Deckel ring des Glukometers ist mittels des Deckelschiebers(258,vgl.Fig.18) mit der Grundplatte verriegelt unter Andruck gegen den Gummidichtring (267). Mit der rechten Betätigungsstange ist über das Scharniergelenk(324) der Querholm(325) verbunden. Nach dessen Abwinkelung ist sein freies Ende mittels einer Blattfeder(326) mit Löchern in einer Gabel der linken Betätigungsstange(249) verrastert (links im Vertikalschnittdetail).
Die rechte Betätigungsstange ist im Vertikalschnitt herausgezeichnet. Beide Betätigungsstangen weisen, wenn auch leicht höhenversetzt, das Scharniergelenk (327) auf, um die Stangen nach Betrieb der Einrichtung im rechten Winkel umlegen zu können(vgl.die rechte Aufsicht der Fig.34).
Das Vertikalschnittdetail rechts unten zeigt eine bevorzugte Achsenlagerung des Scharniergelenkes(324) mit rechteckiger Gelenknoppe in einem Schlitz(328) der Betätigungsstange, wie dies für die Funktion einer Stangenvariation nach Fig.29 (rechter Längsschnitt) zweckmäßig ist.
Ganz oben links zwei Querschnitt-Details für Varianten der Gestaltung der Kanal einmündung für die Wiederbelüftung dicht oberhalb des Saugglockenrandes. (Um die Schrägführung des Kanals im Längsschnitt (Mitte) zu vermeiden.) In der oberen Variante wird die Kanalöffnung durch einen Winkel gegen Verschluß duch hochsteigende Haut abgedeckt. In der unteren geschieht dies durch weitere Tiefverlagerung.
In der Variante oben rechts wird die aufsteigende Haut durch eine vorspringende Stufe mit Schlitzung abgedrängt. Am einfachsten aber erscheint die im Längsschnitt strichpunktierte waagerechte Bohrung in der Höhe des oberen toten Winkels des Saugglockenraumes, welche zur Außenluft verplombt wird. Im Querschnitt unten wird die Stellschraube (57) in einer Leiste, die auf der Grundplatte feststeht, gezeigt, welche gegen den mit der Dachhülse drehenden Segmentschieber(167) stößt. In einer die Bohrungen für die Auslösetaste und Stangen kreuzenden Bohrung lagern die schraffiert wiedergegebenen beiden Sperrkugeln für deren Rastermechanismus(vgl.Fig.29 im Vertikalschnitt). Die beiden Rasttasten(271) sind durch die Leiste(330) starr verbunden. Von letzterer führt ein Schrägarm mit der Rastfeder am Ende(331) in die Rastkerbe(232) in der Betätigungstange. Der Mechanismus ist doppelseitigsymmetrisch angelegt.
Die Symmetrie der starken Druckfedern(70) und deren Wirkrichtung wirkt sich günstig gegen eine vorzeitige Abhebung der Saugglocke infolge eines Rückschlageffektes aus. Jedoch kann auch eine Punktionseinrichtung gebaut werden, bei der einer der Faltenbälge und dessen Bedienungselemente eingespart werden.
Am Randring(319) wurde im Gerätelängsschnitt noch die Lichtquelle(17) eingezeichnet, welche durch den transparenten Punktionsgriffel hindurch ihr Licht auf die Haut wirft. Das reflektierte Licht wird vom Photosensor(223) empfangen. Die Strom- und Signalverbindungen sowie das Photometer wurden nicht wiedergegeben. Lichtquelle und Photosensor können auch unter dem Topf(248) montiert sein.

Zur Inbetriebnahme der Punktionseinrichtung werden die Betätigungsstangen aufgerichtet. Der Querholm der rechten Betätigungsstange wird zum Ende der linken Betätigungsstange(249) umgelegt und mittels der Blattfeder(326) mit dieser verrastert. Der Querholm wird nun hochgezogen bis zum Anschlag eines (nicht eingezeichneten) Stiftriegels am Ende einer Längsnut der Bohrung in der Grundplatte (vgl.Fig. 39). Durch Druck auf die Deckel der Faltenbälge werden diese bis zur Verrasterung durch die Sperrkugel (vgl.Fig.29) zusammengepreßt. Es wird durch Herausziehen des Deckelschiebers (soweit nicht schon früher die Trennung vom Glukometer vorgenommen wurde) das Glukometer mit seinem Deckelring abgenommen und der Punktionsgriffel gewechselt, d.h. ein neuer Teststreifen mit seinem Ende in den Schacht des Glukometers eingeführt. Der Deckelring wird dann wieder auf den Gummidichtring(267) über der Saugglocke aufgesetzt und mittels des Deckelschiebers verriegelt. Nachdem die optische Kontrolle über einen (nicht dargestellten) Schalter ausgelöst wurde und die Eignung der Haut bestätigte, auf welche die Saugglocke aufgesetzt wurde, wird der über dem Glukometer aufgeklappte und geschlosne Bogen durch Druck auf den Querholm(325) gegen die Haut abgesenkt. Dabei wird von der Rastkerbe (332) der Schrägarm der Leiste(330) mitgenommen und und nach kurzem Schaltweg die Rasttaste(271) ausgelöst. Die Faltenbälge dehnen sich unter Einfluß ihrer Druckfedern(70) aus und über den Sogkanal(162) wird die Luft aus der Saugglocke abgesaugt.
Die Haut steigt dabei in und neben der Schneidspitze des Punktionsgriffels auf. Nach fast maximaler Entfaltung der Faltenbälge wird das Tiefertreten der Betätigungsstangen im Kugelraster(334,Fig. 29) freigegeben. Die Absenkbewegung zur Sogerzeugung erfolgte durch Absenkung des Querholm in den Schlitzen(328,Fig.28 Vertikaldetail rechts unten).
Die Sperrung der Abwärtsbewegung der Betätigungsstangen in den Kugelrastern begünstigt die Ruckhaftigkeit der weiteren Stangensenkung. Dabei gerät der Querstift(251) über die Schräge der Kulissenführung und bewirkt die Sektordrehung der Dachhülse, welche durch den Ansstoß der Verstellschraube am Segmentschieber(167). Die Lücke im Deckelschieber für den Durchlaß des Querstiftes(251) wurde nicht dargestellt. Die Wiederbelüftung nach Meldung vom Glukometer kann durch seitliche Geräteabkippung erfolgen. In Fig.34 wird ein besonderes Wiederbelüftungsventil vorgestellt.

Die Figur 29 gibt linksseitig einen Vertikalschnitt durch die funktionssteuernden Sperraster längs Der Querbohrung(335) durch die Grundplatte einer Punktionseinrichtung nach Fig.28 wieder.
Die Rasttaste(271) ist durch die Druckfeder(336) angehoben. Ihre Rastkerbe steht dadurch oberhalb der (schraffierten) Rasterkugel, welche die Raststange(256), die mit dem Deckel(147) eines Faltenbalges (Fig.28) verbunden ist, an ihrer Bewegung hindert. Eine solche Bewegungshinderung besteht auch für die Betätigungsstange(249) in deren Rastkerbe durch die Raster-oder Sperrkugel, welche der Raststange anliegt.
Rechtsseitig wird das Funktionsstadium nach Herabdrücken der Rasttaste gezeigt. Die Sperrkugel wurde durch die Anhebung der Raststange von der Schräge der Rastkerbe in die Rastkerbe der Rasttaste abgedrängt. Nach Anhebung der Raststange stieg deren Rastkerbe in die Höhe der rechten Sperrkugel zur Betätigungsstange hin auf. Durch den manuellen Druck von oben auf die Betätigungsstange verdrängt der Rastkerbenrand die Sperrkugel in die Rasterkerbe der Raststange. Letztere kann wegen der längsovalen Gestalt der Rasterkerbe sich den Faltenbalg dehnend noch etwas weiter aufsteigen. Die Absenkung der Betätigungsstange erfolgt nach Kraftaufstau über dem Raster plötzlich.
Rechts von dieser Darstellung wird im Maßstab 2 : 1 die Variante einer Betätigungsstange im Längsschnitt gezeigt. Die Betätigungsstange(249) setzt sich in einer Hülse mit Gabelung(338) fort, deren Gabelstücke das Scharniergelenk(327) aufweisen. Bei der Absenkung der Betätigungsstange innerhalb der Hülse wird der Druck zunächst von der Druckfeder(337) zwischen einem Querstift in der Betätigungsstange und der Oberkante der Hülse aufgenommen. Wird die Verrasterung der Hülsenbewegung in den seitlichen Sperrastern(339) überwunden, so erfolgt die Hülsenabsenkung ruckartig.
Der Vereinfachung halber werden die Sperrkugeln des Sperrasters(339) durch Blattfedern in die Rastkerben in der Hülse gedrückt; sie können auch in Analogie zu den Verhältnissen bei den Sperrbolzen(195,Fig.24) einstellbar gestaltet sein.
Das Detail unten zeigt eine Variante der Einführung des Topfes(248) mit dem Gummidichtring(267) auf dem Deckel mit Klappscharnier(340). Der Topf kann vom Eingang der (nicht dargestellten) Saugglocke abgeschwenkt werden. Die schräg neben dem Klappscharnier in einer Mulde unter dem letzteren sich ausspannende Zugfeder(341) stabilisiert die Endstellungen des Topfes. Oben liegt ein Längsschnitt, unten eine Draufsicht vor, beides in natürlicher Größe.

Die Figur 30 zeigt eine Betätigungsstange(249), welche durch einen Sektorschlitz(unten im in der Draufsicht) einer Dachhülse(143) hindurchtritt. Dabei trifft der linke untere Keil(342) auf den linken Blattfederbogen(343) an der Dachhülse und bewirkt deren Sektordrehung. Bei weiterer Absenkung der Betätigungsstange stößt der obere rechte Keil an der letzteren gegen den rechten Blattfederbogen(344), was die Gegenbewegung der Dachhülse zur Folge hat. Bei (nicht dargestellter) vorzeitiger Bewegungssperre der Sektordrehung (vgl.Fig.28 im Querschnitt), weichen die Blattfederbogen den Keilen an der Betätigungsstange aus.
Die Variante des erweiterten Keiles(345) blockiert die Abwärtsbewegung der Betätigungsstange bei einer Gegensektorbewegung, welche die eigentliche Schnittbewegung an Länge überschreitet. Dies ist besonders zweckmäßig, wenn nach der Schneidspitze des Punktionsgriffel eine kurze einseitige Schneidkante folgt, wie unten im Querschnitt innerhalb eines strichpunktiert markierten Hautfeldes gezeigt wird.
Bei der Gegenbewegung mit dem stumpfen Lanzettenanteil, öffnet letzterer einen Spalt für den Blutaustritt.

Die Figur 31 zeigt in einem Querschnittdetail durch ein Punktionsgerät nach Fig.28 in Höhe des Gußgußstückes(283) eines Punktionsgriffels. Letzterer wird nach Art einer Schwinglanzette seitlich abkippend im Spalt zwischen Stößel(306) und Gegenstößel(321) bewegt (Rechtsseitig im Maßstab 2 : 1 nach Winkeldrehung der Saugglocke um etwa 22 Grad). Die Betätigungsstangen mit den Keilen(342,346) sind zur Demonstration in die Waagerechte gedreht. Da die (in Wirklichkeit senkrecht verlaufenden) Betätigungssstangen durch den Querholm(325,Fig.28) verbunden sind, kommen die auf die beiden Stangen verteilten Keile nacheinander in Kontakt mit dem Stößel bzw. mit dem Gegenstößel zur Ausführung der Schwingung des Punktionsgriffels. Das Ausmaß der Stößelbewegungen kann an der Randtülle(347) getrennt für Stößel und Gegenstößel an Stellschrau-a ben eingestellt werden.
Es wird noch ein Federmechanismus skizziert, der dazu dient, die Schneidspitze gefedert in einer Ausschlagstellung zu fixieren. Es wird dann der Schneideschlitz zum Blutaustritt offen gehalten(vgl. Längsschnitt-Detail unten zu Fig.34). Wird aber die Saugglocke unbeabsichtigt wiederbelüftet, so kann die sich aus der Schneidspitze zurückziehende Haut letzterer keine Schnittwunde zufügen, da sie die Schneidspitze gegen die schwache Federwirkung aufrichtet. Die symmetrisch im Doppel angelegte Drahtfeder(348) ist am Innenvorsprung(349) an einem Mittelbogen befestigt und liegt mit einem Ende auf dem Keilvorsprung(350) auf, mit dem anderen auf der Schiebehülse(351) vor dessen Querstift. Während der Betätigung des Stößels(306) kommt das Moment, in dem das Drahtfederende den Keilvorsprung verläßt. Dabei schiebt das andere Drahtfederende die Außenhülse(353) in Richtung Gußstück(283) vor. Sie gibt der Stößelbewegung auf das Gußstück für dessen Kippung (d.h. dem Druck des Gegenlagers des Gegenstössels) nach und hält nach Rückzug des Stößels unter Wirkung der Druckfeder(354) das Gußstück in der Kippstellung nach rechts.
Der Querschnitt links zeigt die der Verschmälerung des Punktionsgerätes dienende Stangen- und Rasteranordnung radiär zum Punktionsgriffel, was eine Schräglage des Glukometers(176) zwischen den Funktionsstangen (Rasttasten, Rast- und Betätigungsstangen) bedingt.

Die Figur 32 demonstriert im Maßstab 2 : 1 eine besonderer Punktionsgriffelausstattung für die Regung der Einstichtiefe der Schneidspitze(285). Oben wird in einer Draufsicht gezeigt, wie ein handelsüblicher Teststreifen für den Glukometer längs der strichpunktieren Linie abgeschnitten werden kann, um den Bluteintritt innerhalb eines (hier nicht dargestellten) Gußstücks auf das Testfeld(104), in welchem die chemische Reaktion sich ereignet, zu ermöglichen.
Darunter erkennt man den Schnittbogen einer dünnen Blechhülse, welche anstelle eines Gußstückes das Testfeld(104, im Längsschnitt darunter) diesem eng anliegend, umgibt(unten im Querschnitt).
Bei der rechts (nach unten versetzten) Variante des Schnittbogens wird die Schneidspitze(285) durch tütenartiges Zusammenrollen kanülenähnlich gebildet; nach Verlötung oder anderem Dichtschluß entspricht die Lösung derjenigen, welche mit Fig.40 für das Gußverfahren beschrieben ist.
Bei der linksseitigen Variante des Schnittbogens bleibt die Umgebung der Schneidespitze zur Saugglocke hin offen. Die Zahnwellenleisten werden einander genähert. Im Längsschnitt unten ist erkennbar, wie ein ovalärer nach innen federnder Blechring (356) mit einem Randfalz in die Zähne der Blechhülse eingreift. Je nach gewünschter Länge des Überstandes der Schneidspitze kann die Blechhülse unter seitlichem Druck an seiner Öffnung aufgeklappt und längs der Zahnleiste verschoben wieder eingerastet werden.

Die Figur 33 zeigt das Ende eines Punktionsgriffels im Längsschnitt in natürlicher Größe. Dem Teststreifenanschnitt in der Fig. oben entsprechend ist das Testfeld(104) nach unten zu eröffnet und in das Gußstück(283) eingebettet. Darunter wird im Maßstab 2 : 1 - oben im Längs- und unten im Querschnitt - gezeigt, wie der Einschub(357) von oben sowohl den nur nach oben geöffneten(358) Schlitz als auch den durchgehenden Schacht(359) entstehen läßt. Letzterer kann bis in die Basis der Schneidspitze hinabreichen und so eine Blutableitungsrinne bilden. Ganz rechts ist noch der Querschnitt bei einem durchgehenden Formeinschub abgebildet.

Die Figur 34 zeigt für eine Punktionseinrichtung nach den Fig.28,29 die Möglichkeit des Umschlagens der Betätigungsstangen um ihre Scharniergelenke und ihre Anordnung oberhalb der Grundplatte.
Links ein Längsschnitt, rechts ein Querschnitt mit der Variation einer spiegelbildlichen Anordnung der Stangenbohrungen bei Ausstattung mit Stößel und Gegenstößel für eine Variante nach Art der Schwinglanzette.

Die Figur 35 zeigt links übereinander den Mechanismus für eine Nachführung der Schneidspitze nach der ruckartigen Schnittbewegung zur Erweiterung des Wundspaltes. Im Längsschnittdetail ganz unten wird diese Punktionsgriffelnachführung gezeigt und der Wundspalt sichtbar. Die Schneidspitze bewegt sich in Höhe des Geflechtes mit stärkeren Blutkapillaren.
Die Betätigungsstange mit dem Keil(342) für die Bewegung des Stößels(306) tritt mit dem Blattfederbogen(360) bei seiner Absenkung über den Querstift (361) der Ventilstange(362) des Belüfungsventils für die Saugglocke. Das Bewegungsspiel der Ventilstange wird durch dessen Stift im Führungsschlitz (363) begrenzt. Das Ausmaß der Absenkung der Betätigungsstange ist durch dessen Aufschlagen auf die Schräge des Keilschiebers(364) festgelegt.
Bei weiterer noch stärkerer Druckausübung auf die Betätigungsstange wird der Keilschieber gegen eine stärkere Druckfeder nach rechts verdrängt und mit seiner Frontabstumpfung gegen die Betätigungsstange blockiert. Dieses Stadium der Keilschieberbewegung nach rechts bewirkt die langsame Nachschwingung und anschließende Blockierung der Schneidspitze, wie dies in der unteren Abbildung erkennbar ist. Der Stößel liegt linksverschoben fest. Wird die Betätigungsstange nach Signal vom Glukometer her wieder hochgezogen, so verläßt ihr Ende die Frontabplattung des Keilschiebers. Dieser wird durch seine starke Druckfeder wieder nach links verschoben. Der Anschlag(365) des Keilschiebers kann durch eine Stellschraube ergänzt werden, an welcher das Ausmaß der Verschiebung des Keilschiebers eingestellt wird(vgl.Fig.39).
Bei der weiteren Anhebung der Betätigungsstange nimmt deren Blattfederbogen die Querstange der Ventilstange mit nach oben, wobei deren Langkerbe (366) den Dichtring zwischen Ventilstange und Grundplatte überbrückt, so daß über den Belüftungskanal(367) Luft in die Saugglocke eindringt.

Die Figur 35 beschreibt rechtsseitig einen Mechanismus zum Deckelabschluß der Saugglocke. Dies geschieht oben in der Aufsicht und unten im Längsschnitt, links in der Öffnungs-und rechts in der Verschlußphase. Im Unterschied zur Vorgehensweise in Fig.18 bleiben die beiden Gummiplatten (259,260) auf dem Saugglockendach liegen. Der Funktionsgriffel muß also durch den Schlitz zwischen ihnen in den Saugglockenraum durchgesteckt werden. Die Gummiplatten liegen dem Topf(248) auf. Der Gummidichtring(267) wird von einer Halterung (388) von der Dachklammer(389) her zentrisch über den Gummiplatten in Position gehalten. Bei der Bewegung des Deckelschiebers(257) in Richtung Saugglockendach werden die in Bohrungen der Innenleisten der Gummiplatten verlaufenden Schenkel der haarnadelähnlichen Federklammer mit ihren Keilbögen an den auf der Grundplatte feststehenden Pfosten (269,270,271) nach innen unter Annäherung der Gummiplattenränder an den Teststreifen(103) abgedrängt.
Der in der Mitte für die Passage am Teststreifen vorbei geschlitzte Deckelschieber überholt mit seinen Noppen (372) an seiner Unterseite die quere Hülsenführung des rechten Schenkels der Federklammer und den in diese eintauchenden linken Schenkel der geteilten Federklammer. Die Vertikaldetails ganz unten zeigen, wie der Deckelschieber mit seiner gestuften Ncppenführung dabei in der Endstellung seiner Schiebebewegung gegen die Dachklammer auf den Gummidichtring(siehe Längsschnitt) Druck ausübt und damit die Abdichtung bewerkstelligt.
Das Glukometer(176) befindet sich in der Halteklammer (171). Der Dachkomplex mit der Saugglocke(1) wird mittels der (Rohr-)Stutzen(186) mit Endoliven und durch Federraster in Nischen auf der Oberfläche der Grundplatte(232) befestigt.

Die Figur 37 dient der Erläuterung von Gestaltungsarten für die Höhenverstellung des Punktionsgriffels in Punktionseinrichtungen nach dem Rotationsprinzip. Die Darstellung erfolgt in Längsschnitten in natürlicher Größe. Im Beispiel der linken oberen Abbildung erfolgt die Höhenverstellung in einem Gewinde zwischen Wandring(319) und Dachhülse(143). Außer dem Dichtungsring(265) zwischen Dachhülse und Punktionsgriffel, ist ein weiterer Dichtring(373) zwischen der Saugglocke(1) und einer nach unten gezogenen Randtülle der Dachhülse vorgesehen. Der Randring ist mittels der abschwenkbaren Klemmfeder(374) gegen die Saugglocke fixiert.
Im mittleren Beispiel ist keine Tülle von der Dachhülse in den Saugglockenraum herabgezogen, weshalb der Dichtring(373) in der Saugglocke -er könnte auch an der Dachhülse montiert sein - höher liegt.
Im Beispiel der rechtseitigen Abbildung ist das Gußstück (283) des Punktionsgriffels zur besseren Einführung und Abdichtung nach unten konisch verjüngt. (Der untere Durchmesser wird durch die Breite des Testfeldes bestimmt).
Die Dichtung erfolgt am unteren Rand des Faltenbalges (375), dessen oberer Rand mit dem Dach der Saugglocke(1).
Die Höhe der Eintauchtiefe der Schneidspitze kann von unten in der Saugglocke her durch Verschraubung des den unteren Faltenbalgrand stützenden Randringes bestimmt werden.
Die untere Abbildungsreihe beginnt links mit einem Längsschnitt durch ein Beispiel der Höhenregulierung mittels Hebung und Senkung eines verlängerten Rundteiles des Punktionsgriffels im Dichtungsring(265) bei Anwendung des Keilschiebers(257). Der Keilschieber ist rechts in einer Seitenansicht sichtbar, stützt sich unten gegen die Saugglocke und hebt in schräger Parallelführung und Schlitzführung im Randring(319) denselben. Das Ausmaß der Verschiebung wird an der Stellschraube(57), die an einem Haltarm fest auf der Saugglocke steht, durch Festklemmung in einer Schlitzführung des Keilschiebers geregelt(Seitenansicht). Die kleine Dachhülse(143) greift in den Sechskantring des Punktionsgriffels ein und kann durch (nicht dargestellte) Segmentschlitze des Randringes (auf dem Längsschnitt vorn und hinten) in früher beschriebener Weise erfolgen.
Im Längsschnitt der Variation rechts in der zweiten Bildreihe ist der Randring(319) außen oben mit der Saugglocke(1) verschraubt. Der Randring greift in eine Ringnut der in der jeweils eingestellten Höhe frei drehbaren Dachhülse(143) ein. Die Dichtung erfolgt zwischen Punktionsgriffel und Saugglockendach.
Die Drehung der Punktionsgriffel der oberen Bildreihe kann beispielsweise über die Schlüsselhülse(376,in der rechten oberen Abbildung mit dem Faltenbalg) mittels Stifte vom unteren Hülsenrand in Löcher (oder Kerben) des Randvorsprunges des Punktionsgriffels erfolgen. Hierzu rechts ein vergrößertes Detail der linken Hälfte eines Gußstückes(283) mit Einführungstrichter und Loch für einen der Stifte.

Die Figur 38 zeigt ein Beispiel für eine Höhenregulierung für einen Punktionsgriffel mit Schwinglanzette in zwei zusammengefügten Schnitten, die rechte Hälfte im Längs-, die linke im Vertikalschnitt in natürlicher Größe.
Der Deckel(377) mit der Halteklammer (nicht dargestellt) für den Glukometer über ihm ist mittels dreier Zugfedern (378) in Haken der Grundplatte(232) befestigt. (Besser noch würde man einen einrastenden Haltering wählen). Randklammern(379) verbinden den Deckel(377) mit dem Deckelring(380). Die schwachen Zugfedern(381) halten eine weitere Deckelplatte mit Winkelstangen (382) in der Schwebe, welche in Bohrungen der Grundplatte leicht rückgefedert werden. Vom Deckel(377) erstrecken sich ebenfalls Fcrtsätze(383) in Vertiefungen der Grundplatte. Vor der Befestigung der Zugfedern(378) wird der Teststreifen zwischen den auf der Deckelplatte mit den Winkelstangen ruhenden Gummiplatten mit harter Randeinfassung (261,Fig.18) und durch entsprechende Schlitze der Deckel hindurchgesteckt und in den Schacht des Glukometers eingeführt. Fortsätze und Winkelstangen werden dann in die entsprechenden Vertiefungen der Grundplatte eingeschoben. Der Keilschieber(257) zwischen dem Deckel (377) und dem Deckelring(380) wird bei analoger Ausstattung wie zu Fig.37 entsprechend der gewünschten Punktionsgriffeltiefe in der Saugglocke gegen die Keilschräge(rechts im Vertikalschnitt) verschoben und festgeschraubt. Nach Einhaken der starken Zugfeder(378) senkt diese den Deckelring. Dessen Innenschräge (links im Längsschnitt) drückt die beiden Gummiplatten dichtend gegen den Teststreifen zusammen. Der Deckel mit den Winkelstangen(382) wird dann gegen den Gummidichtring(267) gepreßt. Zur Höhenverschiebung von Punktionsgriffeln nach Art der Schwinglanzette kann natürlich auch eine Randringverschraubung dienen, wie etwa die in der rechten Abbildung der zweiten Reihe von oben. Die Verengung des Saugglockendaches für den Dichtungsring entfällt dabei.

Die Figur 39 zeigt ein Beispiel für eine Einstellung zweier Schnittlängen für den Fall der Erfolglosigkeit der ersten Auslösung einer Kippbewegung, ohne daß die Lanzettenspitze vorher aus der Einstichstelle entfernt werden muß. Diese Vorgehensweise ist schonender insbesondere im Hinblick auf den unterschiedlichen Gefäßreichtum verschiedener Hautpartien. Die Vorrichtung lehnt sich an diejenige der Fig.34 an. Diesmal aber wird der Keilschieber(364) dazu benutzt, die Tiefe der Absenkung der Betätigungsstange festzulegen und damit auch das Ausmaß der Verdrängung des Stößels(306) durch den Keil (342). Die wirksame Länge des Keilschiebers(364) wird an der Einstellschraube(57) begrenzt, deren Gewinde in einer Bohrung des Endwinkels des Schlittens gedreht wird.
Dessen(384) Bewegung nach links ist durch den Anschlag(385) begrenzt. Der Schlitten weist den Galgen (386) mit einer Rastkerbe für das Ende der nach unten gefederten Sperrstange(387) auf. Ein weiterer Keilschieber(390) läuft parallel. Die in der Bohrung an seinem Ende drehend und verrastert eingelassene Stellschraube läuft durch eine Bohrung in einer auf der Grundplatte feststehenden Leiste.
In der Abbildung links oben, welche der Phase vor der Schnittauslösung entspricht, ist die Stellschraube (57) maximal einwärts geschraubt, so daß dem Ende der Betätigungsstange nur eine minimale Keilabsenkung möglich ist. Sollte das Glukometer keine Blutsättigung des Testfeldes melden, so braucht der Benutzer nur die Betätigungsstange zu ziehen. Die vorzeitige Anhebung der Ventilstange(362) wird durch den gefederten Riegel (391) gegen den Querstift der Ventilstange über der Grundplatte verhindert oder durch einen anderen Raster.
Bei der Anhebung der Betätigungsstange hebt dessen Blattfederbogen(360) einen Querstift an der Sperrstange(387).
Der Galgen(386 und damit der Keilschieber(364) ist dann entriegelt(rechte untere Abbildung) und bietet damit dem Ende der Betätigungsstange kaum mehr Widerstand. Er wird nach rechts verdrängt. Die zweite Absenkung der Betätigungsstange findet ihre Grenze jetzt an der Einstellung des zweiten Keilschiebers, indem das Stangenende auf dessen Keilfläche trifft. Der Ventilstift muß bei Rückzug der Betätigungsstange entriegelt bzw. der zusätzliche Raster zur Wiederbelüftung über den Belüftungskanal(367, Fig.34) überwunden werden. Die Anhebung des Ventilstößels erfolgt über Mitnahme von dessen oberem Querstift durch den Blattfederbogen der Sperrstange.
Der Rücktransport des Keilschiebers(364) mit seinem Schlitten nach links erfolgt erst bei maximaler Hebung der Betätigungsstange. Die an letzterer befestigte Querplatte(392) setzt sich in eine Winkelplatte mit Schlitz fort. Die Länge des Schlitzes wird dem Weg der Betätigungsstange angepaßt. Der Schlitzführung(393) für den Führungsstift der Betätigungsstange kann deshalb in diesem Beispiel auch entfallen. In der Endphase der Hebung des Betätigungsstange wird die Platte mit Schlitz (394) vom Querstift in der Querplatte(392) an gehoben.
Der Querstift(395) in der Verlängerung der oben geschlitzten Platte greift von unten in den Schrägrand des durch den Schlitten(384) verlängerten Rahmens ein und bewirkt dessen Rückführung nach links (also von der Lage in der linken unteren in diejenige der rechten oberen Abbildung.
Bei Wegfall des Führungsschlitzes(393) können der Dichtungsring und die Langkerbe(366) zur Eröffnung des Belüftungskanals(vgl.Fig.34) vorteilhafterweise auch direkt an der Betätigungsstange liegen unter Fortfall der Ventilstange.

Die Figur 40 zeigt oben im Längs- und unten im Querschnitt in natürlicher Größe das Ende eines Punktionsgriffels. Analog zur Darstellung in Fig.32 stellt die Schneidspitze eine Art Kanülenende dar, dessen Hohlraum sich bei eventuell sonst nach unten geschlossenem Rahmen in den Raum mit dem Testfeld(104) öffnet. Wenn die Schneidspitze innerhalb der Haut liegt, so stellt sich bei dieser Anordnung zum Testfeld hin ein Luftdruckgefälle ein, wobei der Schlitz(358) nach oben in den Sograum offen sein muß. Das Material für den Gußteil mit Schneidspitze kann Glas, Kunststoff, Metall oder anderes sein.

Die Figur 41 zeigt oben im Quer- und unten im Längsschnitt im Maßstab 2 : 1 eine Punktionseinrichtung für zwei verschiedene Schnittlängen unter Anwendung eines Punktionsgriffels nach dem Rotationsprinzip.
Die Schnittlinie A - B des Längsschnittes liegt linksseitig etwas tiefer als rechtsseitig(strichpunktierte Linie).
Das Drehmoment wird durch die starke Zugfeder(396) erzeugt, welche von einem Stift in einem Segmentschlitz der Dachhülse(143) zu einem Vorsprung am Dach der Saugglocke(1) ausgespannt ist. Die Rotation wird durch den Konus der Stellschraube(57) begrenzt, welcher in den Segmentschlitz(397) der Dachhülse hineinragt. Die Stellschraube(57) führt durch den Schwenkhebel(398) mit dem Scharniergelenk(400) an der Saugglocke und wird durch die Zugfeder(399) an diese herangezogen.

Es ist eine zweite Einstellschraube(401) vorhanden, welche in einem Gewinde im Brückenarm(402) verstellbar ist und einen etwas breiteren Konus aufweist als die Stellschraube(57) und in Höche und radiärer Anordnung gegen letztere etwas versetzt ist. Der Brückenarm steht fest auf der Grundplatte der Saugglocke(1). Der Schwenkhebel (398) hat eine Ausnehmung für die Stellschraube (401) und reicht mit seinem nach unten federnden Dachbogen(403) bis auf die Rundnoppe(404), für die er ein Loch aufweist, wie im Längsschnitt zu erkennen ist. Die Rasttaste(271) hindert in der Halbrinne der Dachhülse(143,Querschnitt oben) die Dachhülse an der Drehung bis gegen die Druckfeder (336) durch Absenkung derselben eine Lücke in der Rasttaste (wie abgebildet) die Dachhülsenbewegung freigibt. Dabei stößt das Ende des Segmentschlitzes(177) gegen die Stellschraube (57). Erfolgt nach dieser Schnittbewegung keine Bestätigung vom Glukometer, daß Blut vom Testfeld aufgenommen wurde, so kann der Schwenkhebel gegen die Zugfeder(399) abgekippt werden. Dabei kann die Kante des Segmentschlitzes den Konus der Einstellschraube(57) passieren und knallt gegen die Einstellschraube(401). Während der Ausschwenkbewegung des Schwenkhebels wirkte die Schrägflanke des nach unten federnden Dachbogens(403) gegen die Rundnoppe(404) auf dem Dach der Dachhülse und drängte diese im Uhrzeigersinne zurück bis zur Einrastung in die Raststange, die ebenfalls abgesenkt werden muß. Dies kann über eine Keilführung zwischen Rasttaste und Schwenkhebel erfolgen, die nicht dargestellt ist.
Die Saugpumpe wurde weggelassen; lediglich die Öffnung(97) für die Sogeinleitung in der Saugglocke ist eingezeichnet.
Links in der Mitte ist in natürlicher Größe eine Punktionseinrichtung mit besonders geringer Höhe der Dachgestaltung angeführt. Die Dichtung des Punktionsgriffels erfolgt in Analogie zu den Verhältnissen in den Fig.28 und 41 über dessen Rundung zu einer entsprechend glatten Trogmulde im Saugglockendach. Das untere Griffelende ist dabei aber vorzugsweise halbkugelförmig und die Dachmulde für deren Aufnahme entsprechend. Als Schneidspitze kann eine Kanüle oder eine nur ganz gering exzentrisch montierte Lanzettenspitze gewählt und dadurch der Lochdurchlaß in die Saugglocke klein gehalten werden.
Die Rotation des Punktionsgriffels erfolgt vom Sechskant seiner Randausladung über dem Saugglockendach über die Dachhülse in einer der bereits geschilderten Weisen.
Die Dachhülse weist Querstifte oder Noppen oder einen Randring auf, der von oben auf die Randausladung des Griffels drückt. Sie selbst wird durch Federn in Verbindung mit der Saugglocke nach unten gepreßt, um die Abdichtung des Saugglockendaches zu gewährleisten.
Ein Griffel mit halbkugeligem Ende kann auch in einer anderen Gerätestruktur als Schwinglanzette eingesetzt werden.

Die Figur 42 zeigt in Längsschnitten eine vereinfachte Deckelkonstruktion zur Abdichtung um den Teststreifen, oben in Annäherung des Glukometers an die Saugglocke, unten nach Koppelung.
Die Saugglockewandung (1) ist starr. In ihren rechtwinkeligen Einführungsschlitz paßt das zugespitzte um den Teststreifen nach unten verlängerte Schachtende (408) eines Gummideckels, der um den Glucometerschacht mit diesem verklebt ist. Der Gummischacht wird dichtend um den Teststreifen zusammengepreßt von den Kanten um den Einführungsschlitz der Saugglocke.

Die Figur 43 bringt oben in einem Quer- und unten in zwei Funktionsstadien im Längsschnitt im Maßstab von 8 : 1 einen Teststreifen mit integrierter kippender Lanzettenspitze.
Der Lanzettenschaft (13) verläuft hier waagerecht in einem Schlitz des Testfeldes (104) und weist am Ende die Querachse (409) auf. Am anderen Ende des Lanzettenschafts biegt deren Spitze rechtwinkelig noch unten (bei Verwendung eines Punktionsgerätes nach Fig.7,8 oder 45 in entsprechender Modifikation gegen und schließlich in die Haut).
Nach der Achse setzt sich das Schaftende unter Knickung in eine Winkelplatte (410) nach oben fort. Zwischen den Längs schnitten durch den Teststreifen (103) ist das Ende des Schiebers (109) eingezeichnet, dessen Ende bei seiner Absenkung auf die gerundete Kante der Winkelplatte trifft und diese absenkt. Die Schneidspitze beschreibt dabei eine Ahlenkbewegung um die Querachse des Lanzettenschaftes. Die Länge der Schnittbewegung wird an der Einstellschraube (57) geregelt, deren Konus eingezeichnet ist. (Der Gesamtmechnismus kann analog früherer Darstellungen ergänzt werden).

Die Figur 44 gibt in etwa 1,5 facher Vergrößerung ein Punktionsgerät für den Einmalgebrauch wieder und zwar oben in einem Querschnitt längs der Schnittlinie A - B des Längsschnittes darunter.
Der obere Längsschnitt zeigt das Gerät vor, der untere während des Gebrauchs und zwar nach der Verschiebung der Lanzettenspitze zur Schnittausführung.

Das gestreckte Gehäuse (411) weist in seinem Inneren einen fabrikmäßig erzeugten Unterdruck auf.
Die Saugglocke (1) besteht in einer Wandeinstülpung mit zentralem Schlitz im Dachbereich. Dieser Schlitz ist mittels der Deckelplatte (412) verschlossen, welche rechtwinkelig vom Teststreifen (103) absteht und beidseits des Testfeldes (104) Einschnitte für das Saugglockendach aufweist. Zwischen der Gehäusewand und dem Testreifenende befindet sich die Zugfeder (413) in Spannung. Die Linksverschiebung des Teststreifens aber wird durch die Plastiksaite (414) verhindert, die ebenfalls vom Teststreifenende ausgehend sich, in Gegenrichtung zur Zugfeder, an die Gegenseite der Gehäuswand erstreckt. Eine zweite Plastiksaite (417) übernimmt die gleiche Brückenfunktion, ist aber nicht gespannt, sondern hat ein streckenmäßig festgelegtes Bewegungsspiel. Durch jeden der Plastiksaiten ist ein Glühdraht (415,416) hindurchgezogen, der über je eine Handtaste (110,418) einen Stromkreis zur den Batterien (83,419) schließen läßt.
(Die Leitungsverbindungen werden durch gestrichelte Linien symbolisiert). Das freie und zur Gehäusewand gedichtete Ende des Teststreifens befindet sich im Schacht des Glukometers (191).
Im oberen Längsschnitt mit Blick auf die Breitseite des Teststreifens sind die Leitungen für die Stoffwechselmessung gestrichelt eingezeichnet. (Das Glukometer wurde hier weggelassen).
Der Auslösestift (228), welcher in einer Dachbohrung der Saugglocke lösbar gedichtet ist, wurde durch die angestaute Haut und den Sog angehoben (unterer Längsschnitt, vgl.Fig.33). Die Haut wurde in die Lanzettenspitze gehoben, welche von der Mitte des Testfeldes (104) nach unten sich erstreckt.

Durch Betätigung der Handtaste (110) wurde die Plastiksaite (415) erhitzt und und zum schmelzen gebracht. Die Zugfeder (413) hat den Teststreifen ruckartig nach links verschoben. Dies war möglich, weil der Verschluß durch die Deckelplatte nach Luftdruckausgleich über die Bohrung des Auslösestiftes aufgehoben ist. Der Knick im Teststreifen (103, siehe Querschnitt) ist im Stadium des unteren Längsschnittes durch Streckung ausgeglichen. Die Schutzfolie (216) wird vor Gebrauch von der Geräteunterseite abgezogen.

Die Figur 45 zeigt oben in zwei Querschnitten längs der Schnittlinien A - B und C - D des unteren Längsschnittes ein Punktionsgerät nach dem Rotationsprinzip. Der im Längsschnitt nur symbolisierte Deckelarm wird in der Mitte noch näher skizziert und rechts durch eine Vertikalansicht ergänzt.
Unten in der Mitte die Details dreier Griffel-Testfeldvarianten im Längsschnitt und im Maßstab 2 : 1.
Der eingeschraute Nippel dient (wie in Fig.37) der Befestigung des Faltenbalges (46) mit seiner Druckfeder (70) an der Grundplatte (232) mit der Saugglocke (1). Die Einstellschraube (57) dient der Regulierung des Keilschiebers (364). Für die Festlegung eines zweiten Schnittes ist die Einstellschraube in die außen drehbare Gewindehülse (420) eingeschraubt, die im Einschub (421) verstellt werden kann. Die Verschiebung des Einschubes wird durch den vertical verschieblichen Keilschieber (422) blockiert. Der Teststreifen verläuft quer in den Schacht des Glukometers (191) und zwar oberhalb des Keilschiebers. Mit getrennten Querachsen in der Dachleiste (427) werden die große Deckelklappe (423) und die kleine Deckelklappe (424) von einer starken (425) und einer schwachen (426) Zugfeder in Richtung Saugglockendach gehalten. Die Lasche (428) der kleinen Deckelklappe untergreift mit seitlichen Gabelholmen die große Deckelklappe und hat eine Öffnung für den Durchlaß des Sperrstiftes (430) von der großen Deckelklappe her.
Bei geschlossenen Deckelklappen liegt dieser Sperrstift dem biegsamen Ende (schwarz) der Schiebestange (275) auf, solange der Faltenbalg (46) zusammengepreßt ist. Wird er losgelassen, nachdem die Saugglocke saugend auf die Haut gesetzt wurde, so kann der Sperrstift in seinem Schlitz nach unten treten. Unter Einfluß der starken Zugfeder (425) tritt jetzt aber auch die große Deckelklappe ruckartig tiefer. Ihre Schiebegabel (432) dreht dabei von dessen Rändern her den Teststreifen (103, unterer Querschnitt und Detail in Vertikalansicht rechts darunter bei überzeichneter Höhe). Beim Teststreifen handelt es sich um eine Art gebogenen Plastikspatel, dessen rundes Ende mit dem Testfeld und der Schneidspitze in einer Einsenkung der Saugglocke lagert. Zur Abdichtung liegt der leicht gefederte Winkel der kleinen Deckelklappe dem Testfeld auf (Längsschnitt). Das Glucometer (191), in dessen Schacht das Teststreifenende eingeführt ist ruht auf der Platte (433) auf Kugeln gelagert. Die Platte ist um die Achse (434) schwenkbar. Zweckmäßig kann die Platte (433) auch durch zwei Holme mit getrennten Achsen ersetzt werden, welche außerhalb der Benutzung in Richtung Faltenbalg umgeschlagen werden. Die Deckelklappen werden in Schlußstellung durch dicke Striche symbolisiert, in geöffnetem Zustand gestrichelt.
Unten in der Mitte sind drei Varianten eines Griffelendes im Maßstab 2 : 1 im Längsschnitt gezeichnet. Der linke obere Griffel liegt mit kreisförmigem nach unten vorspringedem Rand einer (nicht dargestellten) kreisförmigen Rinne im Saugglockendach an. Die Schneidspitze verläuft durch das Testfeld (104) nach unten. Die Leitungen in letzterem sind als drei Punkte zu erkennen.
Die untere Variation zeigt das Testfeld (104) in eine Art Plastikbüchse eingebettet, aus der die Schneidspitze wie ein Kanülenende sich nach unten öffnet. Gehalten und nach oben gedichtet wird der Griffel im Saugglockendach im Dichtungsring (5).
Mit einem hutartigen Rand ruht der Griffel über dem Dichtungsbereich auf dem Kugellager (435) auf dem Saugglockendach. Die Erleichterung der Drehung durch ein solches Kugellager ist bei entsprechend hoher Druckkomponente von oben auch bei anderen bisher beschriebenen Punktionsgeräten sinnvoll.
(Feinste Druckausgleichsöffnungen seitlich vom Testfeld und unterhalb des Dichtungsring wurden nicht dargestellt).
Die Variante rechts zeigt ein Griffelende zum Einsatz in einer Dachkonstruktion analog zu Fig.41 Mitte rechts. Das Testfeld ist der halbkugeligen Abschlußkontur angepaßt. Dicht neben der Schneidspitze als Kanülenspitze die Druckausgleichsöffung (436).

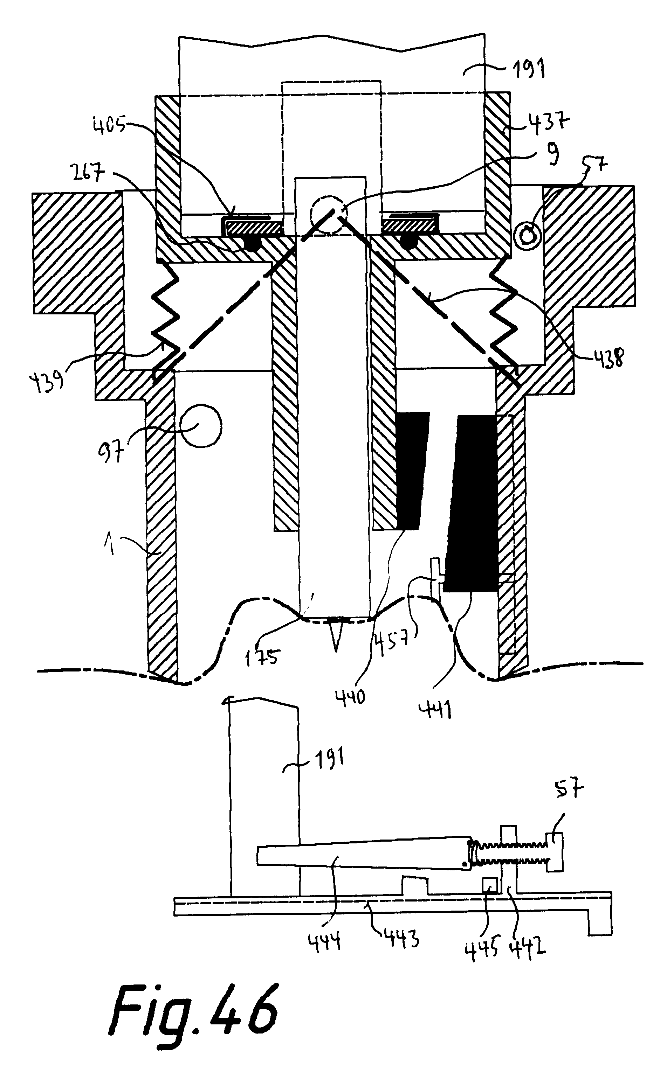

Die Figur 46 zeigt im Längsschnitt im Maßstab 3 : 1 eine Punktionseinrichtung bei hochgelagerter Schwingachse und beim Einsatz von Permanentmagneten. Zwischen der nach oben ausladenden Saugglocke (1) und dem Aufnahmezylinder (437) dient der Faltenbalg (439) zur Abdichtung. Der Aufnahmezylinder schwenkt um die Achse (9), welche von vorn und hinten von je einer Giebelstütze (438) getragen wird. Der Punktionsgriffel (175) sei beispielsweise flach gewählt und im Aufnahmezylinder etwa auch durch eine Schienenführung (nicht dargestellt) verstärkt und stabilisiert. Das Griffelende ist in den Schacht des Glukometers (191) eingeführt.
Dessen zum Schacht gedichteter Deckelring ist mittels des von hinten eingeschobenen Deckelschiebers (405) mit dem Aufnahmezylinder verriegelt und durch den Gummidichtring (267) auch zu ersterem gedichtet. Der Permanentmagnet (440) unten am Aufnahmezylinder wird durch die Konusstange(444) mit der Einstellschraube (57) gehindert sich zum Permanentmagneten (441) hinzubewegen. Letzterer kann mit seiner Schräge parallel zum Permanentmagnet (440) längs einer Schiene innen in der Saugglockenwand zur Abstandsverringerung zwischen den Magneten verschoben und mittels der Stellschraube (457) festgestellt werden. Einer der Permanentmagneten kann auch durch einen Elekromagneten ersetzt werden. Die Haut (strichpunktiert) ist in die Saugglocke und in die Schneidspitze hochgesaugt. Die Pumpe ist weggelassen und nur die Öffnung zur Luftableitung (97) eingezeichnet.
Unten das Längsschnittdetail der Konusstange (440) mit der Einstellschraube (57), verstellbar in einer Lasche des Schiebers (442) dessen Schulter bei Zug nach rechts längs der Schiene (443) gegen den Anschlag (445) stößt. Der Rückzug der Konusstange vom Glukometer (191) löst die Kipp- und Schneidebewegung aus, deren Länge an der Einstellschraube (57) reguliert werden kann. Analog kann die Kippbewegung auch durch das rasche Einwärtsschieben einer Konusstange (bei einstellbarer Schublänge) bewirkt werden. Schwächere Permanentmagnete können auch zur leicht lösbaren Fixierung der Ausschwenkbewegung der Schneidspitze genutzt werden.

Die Figur 47 zeigt oben und in der Mitte im Längsschnitt und unten im Querschnitt in Höhe der Schnittlinie A - B des mittleren Längsschnittes das Beispiel einer Punktionsvorrichtung ohne die Notwendigkeit eines manuellen Griffelwechsels.
Die Wiedergabe erfolgt in natürlicher Größe, wobei die obere Abbildung sich auf das Stadium vor und die untere Abbildung auf das Stadium während der Punktion bezieht.
Hinsichtlich der Unterdruckerzeugung für die Saugglocke (1) handelt es sich um eine konzentrische Raumanordnung wie etwa im Beispiel der Fig.11-13.
Wie beim Beispiel der Fig.24 wird die kräftige Druckfeder für die Vakuumerzeugung vermieden; es ensteht hier oberhalb der Saugglocke unter Verwendung der zentral durchbrochenen Topfmanschette oder Rollmembran (458), welche sich zwischen der Oberkante des äußeren Stützzylinders (459) und dem pilzförmigen Kolben (460) ausspannt, mit denen an den nicht abrollenden Teilen der gewebebeschichtete Teil der Rollmembran fest verhaftet ist. Über dem Kolben (460) geschieht dies über eine auf letzterem angeschraubte Schale, am Stützzylinder mittels Klebung. Die dargestellte Umschlagfalte stimmt nicht mit dem Stadium der Hautansauggung überein. Die Falte wölbt sich nämlich unter Sogeinfluß nach innen (mittlere Abbildung).
Erst wenn der Punktionsgriffel (175) zur Wiederbelüftung mit der Haut angehoben wird, entsteht der nötige Überdruck für die Faltenbildung.
Zur sicheren Überdruckerzeugung empfiehlt sich nach jeder Punktion ein letztmaliger Griffelrückzug nach Verschluß der Saugglocke mit dem Deckel (462), der mit elastischem Rand in die Saugglocke eingreift und zweckmäßigerweise dort mittels (nicht dargestelltem weit über dem Saugglockenrand liegenden) Bajonettverschlusses verschraubt wird.
An seinem verkürzt und am Ende zu schmal dargestellten Stempel kann der Deckel bei Saugglockenhochstand entfernt werden.
Die Verwendung einer Rollmembran zur Sogerzeugung wurde erprobt und ist nur deshalb praktikabel, weil der für die Hautansaugung erforderliche Unterdruck verhältnismäßig gering ist. Die Punktionseinrichtung für den automatischen Testfeldwechsel kann auch mittels anderer Sogquellen betrieben werden.
Als Teststreifen (103) dient ein Band, auf dem die Testfelder regelmäßig aufgereiht sind. Die Randperorationen (vgl.Fig.52 Mitte) des Teststreifens dienen den Zahnradpaaren (461) zum Eingriff, welche mit der Anhebung des Glukometers (176) über die Laschen (463, vgl.Fig.49) im Gegenuhrzeigersinne gedreht werden. Eine Bandbeförderung ist jedoch nur möglich, wenn durch Zug am Handgriff (464), der Deckelzylinder (465) gegen die über den Umfang verteilten Blattfedern (466,Fig.48) so weit abgehoben wird, daß der Teststreifen die Randdichtung zwischen Deckelzylinder und Stützzylinder passieren kann. (Die Lösung entspricht dem Beispiel der Fig.7).
Der Handgriff ist zweckmäßig als geschlossener Bügel (nicht dargestellt) innerhalb einer Bohrung der Stange (469) schwenkbar. Die Zahnradpaare (461) werden dabei mit angehoben. Ihre Achsen liegen in je einer Kappe (495), die um eine Achse in der Dachausbuchtung (468) des Deckelzylinders nach oben abschwenkbar und durch einen (vereinfacht symbolisierten) Schieberiegel (467) zum Deckelzylinder feststellbar ist. Innerhalb der Dachausbuchtung liegen zwei weitere Zahnradpaare (470) über welche der Teststreifen mit seinen Randperforationen auf seinem Weg über die Schiene (471) um das Punktionsgriffelende geleitet wird.
Jedes der beiden Zahnradpaare ist mittels der Stütze (472) auf der Kappe des Kolbens (460) befestigt. (Um Totraum zu vermeiden und die Rollmembran abzustützen ist der Raum um die Stützen und Zahnradpaare mit Füllmaterial ausgekleidet, das nicht dargestellt wurde).
Die Abdichtung des Pumpenraumes über der Rollmembran erfolgt um den Punktionsgriffel mittels des Dichtungsringes (5) im Deckelzylinder und mittels des Dichtringes (373) zwischen Saugglocke und Kolben (460). Der Teststreifen liegt mit seinen unbenutzten Testfeldern in der rechten Trommel (473) um die Achse (474) mit (nicht dargestelltem) Klemmschlitz für das Einschieben des Bandanfanges. Das Ende des Teststreifens oder Bandes liegt im Klemmschlitz der Achse der linken Trommel. Auf dem Querschnitt unten ist die Torsionsfeder (477) eingezeichnet sowie die durch Teleskopauszug verlängerbare Kurbel (478), um die Torsionsfeder zwischen Trommel und Kurbel anzuspannen, so daß sie durch ihr Drehmoment im Gegenurzeigersinne den Transport des Teststreifens unterstützt. (Der Kurbelgriff kann zur Bewegungssperrung - wie nicht dargestellt wurde - gegen die Stützzylinderwandung eingeschoben werden).
Das Glukometer stellt - nur gehäusemäßig - eine Sonderkonstruktion dar um Höhe einzusparen.
Der zentrale Schacht (479) läßt die Absenkung des Glukometers um die Dachausbuchtung (468) zu. Weitere Schachtausnehmungen sind für den Rasterbolzen (475) sowie die Rasterwinkel der Laschen (463) und ihrer Verbindungsstangen (480) vorgesehen. Die Lage der beiden Stifte (476) mit ihren Verriegelungslaschen auf dem Gehäuseboden des Glukometers ist ebenfalls eingezeichnet. Ihre Funktion wird mit Fig.48 beschrieben. Die Trommelgehäuse sind fest mit dem Stützzylinder (459) verbunden. Die Einstellschraube (481) erlaubt die REgulierung ein Eintauchtiefe des Punktionsgriffels in die Saugglocke. Die Verbindungsstangen (480) dienen der Abkoppelung des Teststreifentransportes (vgl.Fig.49 links).

Die Figur 48 zeigt auf drei Längsschnitten durch die Punktionseinrichtung längs der Schnittlinie C - D des Querschnittes in Fig.47 unten drei Funktionsstadien A - C . Das Stadieum A entspricht demjenigen im oberen Längsschnitt der Fig.47, das Staium C dem unteren Längsschnitt in Fig.47 unter Hautansaugung. Die Stangen im Punktionsgriffel samt Handgriff wurden weggelassen. Es ging vorallem um die Darstellung der beiden Stifte (476), welche die Absenkbewegung des Glukometers auf den Kolben (460) übertragen. Die Stifte durch die Schalen und die Rollmembran hindurch zu dieser gedichtet mit dem Kolben verschraubt und durch die Dichtungen (482) im Deckelzylinder verschieblich. Mit der Bodenplatte des Glukometers sind die Stifte über die drehbare und in (nicht dargestellter Ringnut) über Verstiftung höhengesicherte Tülle (483) mittels einer Zunge verriegelt (vgl.Fig.52 oben).
Das Stadium B zeigt zunächst die Absenkung der Saugglocke auf die Haut durch die Absenkung des Punktionsgriffels vom Handgriff her.
Nur auf der mittleren Abbildung ist eine Vorrichtung zur visuellen Kontrolle der Punktionsstelle skiziiert, wie sie im Stadium A angewandt werden kann. Es bestehen (gestrichelt dargestellte Leitungsverbindungen zur Glukometerbatterie über einen (nicht dargestellten) Schaltknopf dort für die Stromeinschaltung. Die Lichtquelle (17) wirft Licht über eine Linse auf die zur Punktion vorgesehene Hautstelle. Das von dort reflektierte Bild kann über die Linse im Schrägtubus in der Wandung des Stützzylinders beobachtet werden.
Punktionsgriffel (175) und Saugglocke (1) sind über die Gummimembran (484) gedichtet mit einander verbunden. Der Klemmring (485) besorgt die Membranbefestigung zum Punktionsgriffel hin. Die hier übertrieben demonstrierte Absenkung des Griffels innerhalb der Saugglocke, in welche die Haut hochgesaugt ist, dient der Demonstration, daß der Andruck der Saugglocke federnd durch die Gummimembran bedingt und durch die Eindringtiefe des Griffels variert werden kann. Der Patient kann über Druck auf den Stützring das Einschlüpfen der Haut in der Innenzone um die Saugglocke nicht beeinträchtigen oder beeinflussen.
Im Stadium C ist nach Absenkung des Glukometers der Kolben (460) mittels der Stifte (476) abgesenkt worden. Dabei enstand über und in der Saugglocke ein Luftunterdruck, welcher auch die Rollfalte der Topfmanschette mit einsog und die Haut in die Schneidspitze hob. Gestrichelt werden die elektrischen Leitungen vom Testfeld (104) aus den entsprechenden Bezirken chemisch reagierender Substanzen (vgl.Fig.52, Draufsicht in der Mitte) dargestellt über Kontakte oder Kontaktfedern Signale abgreifen und auf der Punktionsgriffeloberfläche von den als quer kleine Winkel symbolsierten Kontaktfedern des Glukometers blank soweit hinaufreichen, daß Signalwerte im Meßgerät auch nach Anhebung von Griffel und Photometer empfangen werden können. Die mittlere gestrichelte Linie könnte dem Nullleiter entsprechen, der etwa mit der Querplatte der teilversilberten Schneidspitze Kontakt gewinnen könnte.
Die rechts stehenden Längsschnittsdetails des Saugglockenbereiches sind im Maßstab 1,5 : 1 wiedergegeben.
Neben einer der Blattfedern (466) für den Pumpenraumverschluß wird auch einer der Verriegelungsstifte (486) gezeigt, welche das Ausmaß der Trennung zwischen Stütz- und Deckelzylinder begrenzen.

Die Figur 49 zeigt im Maßstab 2 : 1 rechts einen Längschnitt durch die Kappe (495) mit einem der Zahnradpaare für den Teststreifentransport.
Man erkennt ein Sperrzahnrad, das fest mit dem Zahnrad verbunden ist und der Zahnstange (487) bei deren Anhebung mitgenommen wird. Die Zahnstange liegt in einer Rahmenführung und wird mittels der Blattfeder (488) dem Sperrzahnrad genähert. Der Rahmen für die Zahnstange wird über einen Winkel Lasche (463) mitgenommen, die sich ihrerseit mit einem Endwinkel der Oberkante des Glukometers (176) an dessen Schachtinnenseite anklammert.
Links ist schematisiert die Mitnahme des unteren Rahmens durch die Laschen eines Zahnradpaares dargestellt, wobei die Streckenlängen auf die funktionellen Bedürfnisse abzustimmen sind. Jede der beiden rechten Laschen ist mit einer solchen linksseitig vom Schacht durch eine Verbindungsstange (480,vgl.
Fig.47 ur.^{>}ten). Auf diese Weise können die Verbindungsstangen mit einem Handgriff einander genähert werden - gestrichelt dargestellt - wobei die für die Mitnahme mit der Glukometerbewegung verantwortlichen Winkel den Gehäuserand des Glukometers verlassen. (Die Lasche und Rahmen zusammenhaltende Schiene ist gestrichelt angedeutet).

Die Figur 50 zeigt im Maßstab 2 : 1 einen Punktionsgriffel (175) mit spezieller Ausstattung für die Rotationsbewegung einer Schneidespitze in einem um einen kleinen Sektorwinkel drehbaren Testfeld innerhalb eines Teststreifenbandes.im Längsschnitt.
Wie der Querschnitt etwa in Griffelmitte unter dem Längsschnitt verdeutlicht, hat der Punktionsgriffel eine ovaläre Gestalt und ist eine Nase der Stange (469) fest mit dem oberen Ende des Stößels (489) verbunden, dessen unterer Abschnitt nach Kalibersprung durch einen Dichtring geführt wird. Das untere dünnere Ende des Stößels ist es nun das auf die sektorale Keilschräge (490) einer am Griffelende gegen die Torsionsfeder (491) drehenden Scheibe trifft. Der Stift (492) vom Griffel in eine Sektornut der Scheibe begrenzt den Drehraum.
Die dem Rasterbolzen (475) anliegende Keilnase der Stange (469) behindert deren Anhebung im Griffel durch Zug am Handgriff (464) wenig. Erheblichen Widerstand aber bildet die Steilflanke der Ahsenkung der Stange. Die Stärke dieses Wiederstandes kann an der Stellschraube (493), die gegen eine Druckfeder wirkt, reguliert werden. An der Schraube (494) wird das Ausmaß der Stößelabsenkung und damit die Schnittlänge der Schneidespitze festgelegt.

Die Figur 51 gibt im Maßstab 2 : 1 Details über eine Ankoppelung eines Testfeldes an das Punktionsgriffelende oben in einem Vertikal- und unten in einem Längsschnitt (bezogen auf den Längsschnitt der Figur 50).
Die Scheibe (499, ohne Darstellung der Keilschräge für den Stößel) weist Dornen auf, deren Winkelgestaltung den Durchlaß des Teststreifens begünstigen. Bei ihrer Abwärtsfederung mit der Scheibe (499) durch eine Druckfeder im Griffelende besteht eine Rctationshemmung in der seitlichen Kulissenführung (495) und damit eine zentrische Ausrichtung der drehbaren Scheibe des Testfeldes (104, Fig.52 Draufsicht in der Mitte), zwischen deren Wellenprofil die Dornen eingreifen. Drehbar wird die Scheibe erst nach ihrer Anhebung und der Freigabe der Sektorbewegung durch den von der Scheibe in die Kulissenführung des Griffels aufsteigenden Stift. Diese Anhebung wiederum wird durch das Eindringen eines aufgeblasenen Gummikissens (497) während des Teststeifendurchzuges bewirkt. Dabei dient die kräftiger gefederte Schienenführung (498) als Widerlager. Das Gummikissen hat über dem Zentrum des anliegenden Tellers der Schneidspitze - bei Verklebung mit diesem - eine zentale Lücke für den elektrischen Kontakt. Eine weiteres Lcch spitzenwärts ist durch eine Weichplastikmembran (500) verschlossen. Diese wird durch die Heizdrahtschlinge (501, unten auf dem Längsschnitt) über einen Stromstoß vom durch elektrische Leitungen verbundenen Glukometer mit entsprechender Steuervorrichtung eröffnet. Die Luft entweicht dann aus dem Gummikissen und die Schneidspitze dringt in die hcchgehobene zwischen die Schiene eindringende Haut. Bei entsprechender mechanischer Anordnung kann auch die Schneidspitze durch Andruck an Weichplastikmembran dieselbe eröffenen.

Die Figur 52 zeigt im Maßstab 2 : 1 oben im Längsschnitt nochmals eine Darstellung der Verriegelung eines Stiftes (476,vgl.Fig.48) mit der Bodenwand des Glukometers (176) Der Handhebel kann am Haken links vom Finger verschoben und die Tülle (483) mit der verriegelnden Nase so gedreht werden, daß letztere durch die Lücke in der Bodenwand geschoben werden kann. Zweckdienlicherweise wird man diese Lücke etwa im Winkel von 45 Grad zur dargestellen Hebellage für eine linksseitige Verriegelung anordnen.
Darunter eine Draufsicht auf eine Stück Teststreifen im Maßstab 2 : 1 . Man erkennt die seitlichen Randperforationen des Teststreifens (103), welche erlauben, zum Transport auch in der Photoindustrie längst geläufige Verfahren einzusetzen.
Zwei Testfelder (104) liegen auf einer in einer Umrandung drehbaren Scheibe. Die Kreis stellen metallene Kontaktpunkte da (falls meßtechnisch erforderlich). Die zentraleren (schwarz ausgefüllten) Kontakte entsprechen der Lücke im (hier entfernten) Gummikissen oder eines sonstigen Füllmaterials, das auch durch Aufspulen eines getrennten Streifens von der Schneidspitze (285) -diese freilegend - entfernt werden könnte. Man erkennt das Wellenprofil der drehbaren Testscheibe, wobei auch der Innenkreis innerhalb des Wellenprofils durch eine zerstörbare Weichplastikmembran geschützt sein könnte. Die Testscheibe kann auch durch einen überstehenden Rand oder Einzelzarken (wie auf der schrägen Darstellung links bei Fig.51) gegen Loslösung gesichert sein und industriell bereits im Sektorwinkel ausgerichtet.
Ein Längsschnitt darunter zweigt eine Schneidspitze, welche unter Spannung gegen eine der Oberwandung eines Testfeldes anliegenden Schenkel nach links abgebogen ist. Die Lage wird durch eine Querachse (rechts in der Vertikalen) gesichert. Wird die Weichplastikmembran (500) zerstört, so schnellt die Schneidspitze (285) in die gestrichelt dargestelle senkrechte Lage für die Punktion.
Erst jetzt erfolgt die Hautansaugung.
Ganz unten im Längsschnitt ein Stück Teststreifen im Maßstab 2 : 1 zur Darstellung einer alternativen Schnitterzeugung. Links werden zwei parallele Keilstößel bei (nicht gezeigtem) Druck auf ihre Druckfedern gegen den Teststreifen gedrückt. Die Distanz ihrer Spitzen ist so gewählt, daß eine von ihnen über eine Perforation in deren Anfang zu liegen kommt und beim Eindringen in dieselbe eine Teststreifenverschiebung in eine der Richtungen - bei entsprechend rückgefederter Toleranz - bewirkt.
Rechts davon eine Magnetspule (99) im Querschnitt welche stoßartig und quer zum Teststreifentransport bei entsprechend rückgefederter Toleranz der Schienenführung(498) die Schneidebewegung ermöglicht.
Zwischen der mittleren Draufsicht der Fig.52 bis zwischen die Abbildungen der Fig.51 sind zwei Teststreifenenden dargestellt, die mittels der (504,505) miteinander vereinigt sind.

Zur Inbetriebnahme eine Einrichtung nach den Fig.47-52 werden die Verriegelungen der beiden Stifte (476,Fig.48, Fig.52 oben) gelöst und das funktionsbereite Glukometer wird abgehoben. Nach Herausdrehen des (nicht dargestellten) verschraubten Stiftes (466) kann dann der Deckelzylinder nach seitlichem Herausschieben auch der Blattfedern (466, Fig.48) vom Stützzylinder abgenommen werden. Eine Trommel (433) mit einem Teststreifen wird gegen eine Blattfeder in ihr Gehäuse eingeschoben und der Teststreifen aus dem Seitschlitz der Trommel herausgezogen, nachdem seine Klammer am Ende entfernt wurde.
Der Teststreifen wird über die Zahnradpaare (472) geführt, wobei zwischen beiden eine Schleift für den Punktionsgriffel hängen bleibt. Die linke Leertrommel wird aus ihrem Gehäuse herausgezogen und das freie Teststreifenende in den Achsenschlitz eingesteckt.
Die Leertrommel wird in ihr Gehäuse geschoben, der Deckel zylinder aufgesetzt, der Griffel einschoben und das Glukometer aufgesetzt und verriegelt. Im Normalfall aber wird am Ende eines verbrauchten Teststreifens nach dessen Lösung aus dem Schlitz der Trommelachse ein Haken aufgeschoben und mit ihm der Haken des verbrauchten Bandes verbunden ohne daß das Gerät geöffnet werden muß.
Die Saugglocke (1) wird am Griffel abgesenkt, die Lage des Testfeldes und seine Nummer auf der Unterseite und diejenige außerhalb der rechten Dichtung zwischen Deckel- und Stützzylinder (jweils zwischen den Testfeldern aufgedruckt) werden verglichen. Der Deckel (462) wird auf die Saugglocke aufgeschraubt. Er hat ein düsenartig feines Entlüftungsloch, um jetzt den Punktionsgriffel am Handgriff hochziehen zu können. Dabei wird die Rollmembran im Innern ausgerichtet und läßt die Kolbenrückführung erst zu. Der Deckel (462) wird abgenommen und der Stützzylinder auf die Haut aufgesetzt. (Er braucht dabei nur an zwei gegenüberliegenden Kanten Platz auf der Körperwölbung zu finden). In fraglichen Fällen erfolgt visuelle Hautkontrolle durch den Tubus. Erst wird jetzt vom Handgriff her Druck auf gegen die Hautausgeführt, bis der Signalton des Glukometers die Blutsättigung des Testfeldes meldet. Das Gerät kann jetzt abgekippt oder der Handgriff gezogen werden.
Der Glukosewert ist nach der vorgeschriebenen Zeit auf dem Glukometer ablesbar. Der Handgriff wird abgesenkt und der Saugglockendeckel aufgeschraubt. Vor neuer Nutzung wird der Handgriff ganz herausgezogen.
Mittels der in Fig.1-52 geschilderten Punktionseinrichtungen können durch Teilung des Testfeldes oder durch Wahl eines Testfeldes mit entsprechendem Chemismus auch andere Substanzen als Glukose - eventuell wie Azeton auch zusätzlich - bestimmt werden. Auch die Milchsäurebestimmung, weil inzwischen üblich, soll hier erwähnt werden sowie die Untersuchung auf Alkohol und Drogen.
Es sollen nicht nur die einzelnen geschilderten Konstruktionsbeispiele geschützt sein, sondern auch die beliebige Kombination der Erfindungsmerkmale wie sie aus der Beschreibung, den Zeichnungen und nachfolgenden Patentansprüchen hervorgehen.

## Patentansprüche

1. Einrichtung für die Stoffwechselkontrolle bei einem Lebewesen unter Blutgewinnung aus der Haut mittels der Schneidspitze eines Punktionsmittels, das in die Haut eingestochen wird, vorzugsweise innerhalb einer Saugglocke in Verbindung mit einer Sogquelle, wobei die Haut unter Unterdruckeinwirkung angehoben wird, und wobei die Schneidspitze dann quer zur Hautoberfläche bewegts wird,
**dadurch gekennzeichnet ,**
daß Mittel vorhanden sind, die Bewegung der Schneidspitze des Punktionsmittels etwa parallel zur Hautoberfläche in der Regel auf eine einzige und in ihrer Länge variierbare Schnittbewegung von einer Anwendungslänge von nur ausnahmsweise bis zu zehn Millimetern zu beschränken und daß wenigstens ein Testfeld auf einem auswechselbaren Träger mit Chemikalien für wenigstens eine Substanzbestimmung der Blutaustrittsstelle in einer Weise genähert wird, daß das aus durch das Punktionsmittel zerstörten Gefäßen auf die Testfläche vordringen kann, wobei das betreffende Testfeld über Mittel zur Signalübermittlung mit einer Meßvorrichtung verbunden werden kann, vorzugsweise direkt mit einem handelsüblichen Meßgerät,
und wobei vorzugsweise Mittel zur optischen Kontrolle der zur Punktion bestimmten Hautstelle vorhanden sind, um die Punktion einer krankhaft veränderten Hautstelle auszuschließen.

2. Einrichtungsbezogenes Verfahren für die Stoffwechselkontrolle bei einem Lebewesen unter Blutgewinnung aus der Haut mittels der Schneidspitze eines Punktionsmittels, das in die Hasut eingestochen wird, vorzugsweise innerhalb einer Saugglocke in Verbindung mit einer Sogquelle, wobei die Haut unter Unterdruckeinwirkung angehoben wird, und wobei die Schneidspitze dann entlang der Hautoberfläche bewegt wird,
**dadurch gekennzeichnet,**
daß die Bewegung der Schneidspitze etwa parallel zur Hautoberfläche auf eine Anwendungslänge von nur ausnahmensweise zehn Millimeter beschränkt und plötzlich gestaltet wird, um ein Ausweichen von Blutgefäßen vor der Schneide des Punktionsmittels zu verhindern, und daß wenigstens ein Testfeld auf einem auswechselbaren Träger mit Chemikalien für wenigstens eine Substanzbestimmung der Blutaustrittsstelle in einer Weise genähert wird, daß das Blut aus wenigstens einem durch das Punktionsmittel zerstörten Blutgefäß auf die Testfläche vordringen kann, wobei das betreffende Testfeld über Mittel zur Signalübermittlung mit einer Meßvorrichtung verbunden werden kann, vorzugsweise direkt mit einem handelsüblichen Meßgerät für die jeweils zu untersuchende Substanz.

3. Einrichtung nach Anspruch 1 ,
dadurch gekennzeichnet,
daß Mittel vorhanden sind, die Bewegung der Schneidspitze in einer Ebene schwingend oder kippend zu gestalten und dabei in ihrem Ausschlag zu begrenzen, vorzugsweise unter Einführung wenigstens eines Stößels durch eine Dichtung in der Saugglockenwandung, der von außen bewegt wird.

4. Einrichtung nach Anspruch 1 ,
dadurch gekennzeichnet,
daß eine Hülse den Träger des Testfeldes teilweise umgibt und dabei schlüsselartig in die Außenkontur des Trägers so eingreift, daß sie bei ihrer Sektordrehung die letztere mitbewegt, vorzugsweise unter Abdichtung des Saugglockendaches mittels eines Dichtungsringes um eine kreisförmige Ausladung des Trägers des Testfeldes und bei größerem bewegungshemmendem Durchmesser dieser Ausladung zusätzlich unter reibungsherabsetzender Lagerung einer Ausladung des Trägers oberhalb der Dichtung des Saugglockendaches auf einem Teil desselben, wobei in jedem Falle der Rotationswinkel durch einen Anschlag zu einem der rotierten Teile festgelegt werden kann.

5. Einrichtung nach Anspruch 1 ,
dadurch gekennzeichnet,
daß die Schnittbewegung längs der Hautoberfläche über eine Art Keilführung zwischen einem wenigstens zeitweise und zur Bewegungsübertragung mit dem Träger des Testfeldes verbundenen Teil und wenigstens einer Stange bewirkt wird, vorzugsweise über wenigstens eine Handstange, die unter Überwindung einer Rasterhemmung vor Wirksamkeit der Keilführung parallell zur Saugglockenachse abgesenkt wird.

6. Einrichtung nach Anspruch 1 ,
dadurch gekennzeichnet,
daß um den Schacht des Meßgerätes und den in diesen ein geführten Träger für das Testfeld zum Meßgerät gedichtet sich ein Ring befindet, der während seiner Befestigung am Saugglockendach zu diesem einen Dichtungsring einschließt, wobei vorzugsweise zusätzliche Gummiteile vorhanden sind, welche eine Abdichtung um den Träger dem Schacht gegenüber bewirken.

7. Einrichtung nach Anspruch 1 ,
dadurch gekennzeichnet,
daß die Abdichtung des Trägers des Testfeldes und Punktionsmittels zum Dach der Saugglocke über die Einpassung der gewölbten Endfläche des Punktionsmittels in eine entsprechend gewölbte und dicht angepaßte Mulde im Saugglockendach oben erfolgt, welche für den Durchtritt der Schneidspitze eine Öffnung aufweist unter federnder leichter Druckausübung zwischen Punktionsmittel und Saugglockendach.

8. Einrichtung nach Anspruch 1 ,
dadurch gekennzeichnet,
daß ein mit einer Zylinderpumpe als Sogquelle verbundener und mit dessen Pumpenstößel zusammen bewegter Teil einen Sperraster gegenüber einem Teil, der unter Federspannung stehend die Bewegung längs der Haut auf die Schneidspitze überträgt, entriegelt, wobei vorzugsweise, wenn der den Träger mit der Schneidspitze drehende Teil die letzteren als Hülse radiär umgibt, der mit dem Pumpenstößel angehobene Teil in Verbindung mit dem die Rotation übertragenden Teil einen Eingriff in wenigstens eine feststehende Führungskante mit dem Erfolg der Entriegelung verläßt.

9. Einrichtung nach Anspruch 1 ,
dadurch gekennzeichnet,
daß wenigstens ein über eine Klappe zum Saugglockendach gefederter und zeitweise gedichteter Deckel vorhanden ist, vorzugsweise auch eine zweite Klappe, die mit der ersten zusammen gegen eine getrennte Federung angehoben werden kann, welche gegen das Punktionsmittel lehnt, wobei eine der Klappen nach Lösung eines Sperrrasters gegenüber der Absenkbewegung einer der Deckelklappen durch Einwirkung auf den Träger des Testfeldes und der Schneidspitze deren Bewegung längs der Haut bewirkend einwirkt.

10. Einrichtung nach Anspruch 1 ,
dadurch gekennzeichnet,
daß die Ablenkung durch wenigstens einen Magneten, über Einwirkung auf einen mit der Sc:hneidspitze verbundenen Teil deren Bewegung längs der Haut bewirkt.

11. Verfahren nach Anspruch 2 ,
dadurch gekennzeichnet,
daß die Eindringtiefe der Schneidspitze in den Saugglockenraum nahezu stufenlos um eine auch zum Gebrauch nutzbare Höhe verstellt werden kann, vorzugsweise unter Höhenverstellung des Punktionsmittels unter Abstandsänderung zwischen Meßgerät und Saugglockendach

12. Verfahren nach Anspruch 2 ,
dadurch gekennzeichnet,
daß eine zweite in ihrer Länge begrenzbare Bewegung der Schneidspitze längs der Hautoberfläche ohne erneuten Einstich bei angehobener Haut statthat, sei es zur Schnitterweiterung ruckartig im Falle fehlender Erfolgsmeldung von Seiten des Meßgerätes oder langsamer zur Erweiterung des Wundspaltes für den Blutaustritt, vorzugsweis unter vorheriger Rückführung der Schneidspitze in ihre senkrechte Ausgangslage.

13. Verfahren nach Anspruch 2 ,
dadurch gekennzeichnet,
daß auf einem bandartigen Teststreifen mehrere Träger mit Chemikalien und jeweils mit einer Schneidspitze vorhanden sind, welche durch Verschiebung des Teststreifens als Träger der in ähnliche Einheiten zusammengefaßten Chemikalien nacheinander zu getrennten Messungen mit der Haut und schließlich mit Blut und Körperflüssigkeit in Kontakt gebracht werden, wobei vorzugsweise jedes Testfeld als Trägereinheit für Chemikalien zur Anwendung gegenüber der Haut an das Ende eines Griffels geschoben wird, welcher gegen die Haut abgesenkt werden kann und Leitungsfunktionen zur Signalvermittlung an das Meßinstrument über besondere Mittel übernimmt.
